(19) Europäisches Patentamt
European Patent Office
Office européen des brevets

(11) EP 4 450 521 A1

(12) **EUROPEAN PATENT APPLICATION**
published in accordance with Art. 153(4) EPC

(43) Date of publication:
**23.10.2024 Bulletin 2024/43**

(21) Application number: **22906698.0**

(22) Date of filing: **16.12.2022**

(51) International Patent Classification (IPC):
***C07K 16/28*** (2006.01) ***A61K 47/68*** (2017.01)
***A61K 31/551*** (2006.01) ***A61K 39/395*** (2006.01)
***A61P 35/00*** (2006.01)

(52) Cooperative Patent Classification (CPC):
**A61K 31/551; A61K 39/395; A61K 47/68; A61P 35/00; C07K 16/28**

(86) International application number:
**PCT/CN2022/139637**

(87) International publication number:
**WO 2023/109953 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.12.2021 CN 202111551337**

(71) Applicant: **Fortvita Biologics (Singapore) Pte. Ltd.**
**Singapore 189767 (SG)**

(72) Inventors:
- **HE, Kaijie**
  **Suzhou, Jiangsu 215123 (CN)**
- **ZHOU, Shuaixiang**
  **Suzhou, Jiangsu 215123 (CN)**
- **LIU, Xiaodan**
  **Suzhou, Jiangsu 215123 (CN)**
- **LU, Jia**
  **Suzhou, Jiangsu 215123 (CN)**

(74) Representative: **Sagittarius IP**
**Marlow International**
**Parkway**
**Marlow SL7 1YL (GB)**

Remarks:
The complete document including Reference Table(s) and the Sequence Listing(s) can be downloaded from the EPO website

(54) **ANTIBODY-DRUG CONJUGATE TARGETING CLAUDIN18.2**

(57) The present invention relates to an antibody-drug conjugate (ADC) targeting Claudin18.2 and a composition containing the molecule. The present invention further relates to therapeutic and diagnostic uses of the antibodies and antibody fragments.

EP 4 450 521 A1

## Description

The present invention relates to an antibody-drug conjugate (ADC) targeting Claudin18.2 (CLDN18.2) and a composition containing the molecule. The present invention further relates to therapeutic and diagnostic uses of the antibodies and antibody fragments.

## BACKGROUND

Claudins are a family of proteins and are important components that constitute tight junctions between cells. They establish an intercellular barrier that controls the intercellular flow of molecules and thus can control the flow of the molecules between cells. Claudins family proteins have a tetraspanin domain, both N-terminus and C-terminus of which are included in the cytoplasm. Different Claudins are expressed on different tissues and their functional changes are associated with the development of cancer in various tissues. For example, Claudin-1 is expressed in colon cancer and has prognostic value, Claudin-18 is highly expressed in gastric cancer and pancreatic cancer, and Claudin-10 is highly expressed in hepatocellular carcinoma. Claudins, as cell membrane surface proteins, are useful targets of various therapeutic strategies.

Isoform 2 of Claudin-18 (Claudin18.2 or CLDN18.2) is a highly selective cell lineage marker. Its expression in normal tissues is strictly confined to differentiated epithelial cells of the gastric mucosa and its expression is absent from the gastric stem cell zone. CLDN18.2 is expressed in a considerable portion of primary gastric cancers, and its expression level is retained in gastric metastatic cancer tissue. Expression of CLDN18.2 has also been found in pancreatic cancer in addition to gastric cancer, and it is an ideal target molecule for the treatment of these cancers (Singh, P., Toom, S. & Huang, Y. Anti-CLDN18.2 antibody as new targeted therapy for advanced gastric cancer. J Hematol Oncol 10, 105 (2017). https://doi.org/10.1186/s13045-017-0473-4). Regarding gastric cancer, in 2014, there were about 410,000 new cases and about 290,000 deaths in China, which account for almost half of the new cases and deaths worldwide, and the new cases and deaths are still showing an increasing trend. However, there is a large unmet clinical need for tumor therapy, and therefore the development of drugs targeting Claudin18.2 is extremely necessary.

Despite the clinical success of therapeutic antibodies, naked Mabs targeting cell surface tumor antigens rarely provide sufficient efficacy alone. To increase the activity of Mabs, new strategies focus on binding them to toxic molecules. Plant and bacterial toxins and small chemotherapeutic molecules may be good candidates because they are very effective and active in very small amounts.

Due to technological advances made during the last several years, the field of antibody-drug conjugates (ADCs) for the treatment of cancer has recently undergone increasing development activities by pharmaceutical companies aimed at solving the problems that the ADCs originally presented with respect to immunogenicity, affinity, specificity, undesirable toxicity, yield, half-life, etc.

Despite some advances, there remains a need for other therapeutic strategies for treating tumors as well as for components used in such therapeutic strategies, in particular for antibodies against Claudin18.2 that feature high affinity, good specificity, and/or low risk of immunogenicity and for ADC molecules that feature high activity, low toxicity, long half-life, good specificity or affinity, and/or good half-life or pharmacophore properties.

## SUMMARY

The present invention provides an antibody-drug conjugate (ADC) targeting Claudin18.2, which has the following advantages:

(1) binding to target cells expressing human CLDN18.2 and having high affinity therefor;
(2) capable of entering cells through endocytosis to kill target cells; in some embodiments, the ADC of the present invention has high endocytosis efficiency;
(3) having remarkable bystander killing effect;
(4) having high anti-tumor effect;
(5) having low toxicity;
(6) having good stability;
(7) having good pharmacophore properties.

In some embodiments, CLDN18.2 is expressed or overexpressed on the surface of a cell; in some embodiments, the target cell is a CHO cell or 293 cell, e.g., a CHO-S cell or an HEK293 cell, expressing CLDN18.2; in some embodiments, the target cell is a cancer cell expressing CLDN18.2, e.g., a cell naturally expressing CLDN18.2 or artificially transfected to express CLDN18.2 or artificially transfected to have an increased expression level of CLDN18.2, e.g., a gastric cancer or pancreatic cancer cell line or colon cancer or colorectal cancer cell line expressing CLDN18.2. In some embodiments,

the target cell is a cell line with a moderate expression level of hCLDN18.2, e.g., NUGC-4 or SNU620. In some embodiments, the target cell is a cell line with a high expression level, e.g., a DAN-G cell that overexpresses hCLDN18.2.

## BRIEF DESCRIPTION OF THE DRAWINGS

FIG. 1 shows that the HB37A6 antibody specifically binds to CLDN18.2 on the cell surface.

FIG. 2 shows that the HB37A6 antibody does not bind to CLDN18.1 on the cell surface.

FIG. 3 shows the binding of the HB37A6 antibody to the gastric cancer cell line NUGC-4, the gastric cancer cell line KATO III-hCLDN18.2, and the pancreatic cancer cell line DAN-G-hCLDN18.2.

FIG. 4 shows the anti-tumor effect of the HB37A6 antibody in the pancreatic cancer mouse model.

FIG. 5 shows the anti-tumor effect of the HB37A6 antibody in the gastric cancer mouse model.

FIG. 6 shows the cell binding effect of IEX019 molecules.

FIG. 7 shows the endocytosis of IEX019 molecules on DANG-hCLDN18.2 cells.

FIG. 8 shows the killing effect of IEX019 molecules in cell line with low expression of hCLDN18.2 (FIG. 8A), cell lines with moderate expression of hCLDNA8.2 (FIG. 8B), and cell line with high expression of hCLDN18.2 (FIG. 8C).

FIG. 9 shows the bystander killing effect of IEX019 molecules.

FIG. 10 shows the tumor inhibition efficacy (FIG. 10A) of IEX019 molecules in mice and the weight change (FIG. 10B).

FIG. 11 shows the tumor inhibition efficacy (FIG. 11A) of IEX019 molecules in mice and the weight change (FIG. 11B).

FIG. 12 shows the tumor inhibition efficacy (FIG. 12A) of IEX019 molecules in mice and the weight change (FIG. 12B).

## DETAILED DESCRIPTION

### I. Definitions

Before the present invention is described in detail below, it should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this specification, the following definitions will be used, and wherever appropriate, terms used in the singular form may also include the plural form, and vice versa. It should be understood that the terms used herein are for the purpose of describing specific embodiments only and are not intended to be limiting.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

As used herein, the term "comprise" or "include" is intended to mean that the described elements, integers, or steps are included, but not to the exclusion of any other elements, integers, or steps. Herein, the term "comprise" or "include", unless otherwise specified, also encompasses the situation where the entirety consists of the described elements, integers, or steps. For example, when referring to an antibody variable region "comprising" a specific sequence, it is also intended to encompass an antibody variable region consisting of the specific sequence.

The term "CLAUDIN" or "CLDN" used herein is the most important skeleton protein that determines the structures of tight junctions between cells, and it is involved in adhesion junctions and plays an important role in the metastasis and invasion of tumor cells. Claudin proteins are widely present in mammalian epithelial and endothelial cells, primarily

on the lateral surface of epithelial cells and on the plasma membrane of basal cells. Different Claudin proteins have their respective specific expression in different tissues, wherein the Claudin18 (CLDN18) gene is located at 3q22.3, has a molecular weight of 24 kDa, comprises 261 amino acid residues, and is a member of the Claudins superfamily, and its protein structure comprises 2 extracellular loops and 4 transmembrane domains. Two subtypes of human CLDN18 or Claudin18 protein are Claudin18.1 or CLDN18.1 (UniProt ID: P56856-1) and Claudin18.2 or CLDN18.2 (UniProt ID: P56856-2). In the primary structural sequences of the two proteins, only the amino acid residues at some positions from the N-terminus signal peptide to the extracellular loop 1 (Loop1) structure are different, and especially at the extracellular loop 1, CLDN18.1 and CLDN18.2 differ by only 8 amino acids. The intergeneric sequence homology of the two subtypes of CLDN18 protein is also very high. The sequences of the extracellular loop 1 of CLDN18.2 are completely identical in different species such as humans, mice, and rhesus monkeys, and the homology of human and mouse CLDN18.2 proteins reaches 84%, indicating that the sequence of CLDN18.2 protein is extremely conservative (O. Tureci. et al., Gene, 481:83-92, 2011). CLDN18.2 or any variants and isoforms thereof may be isolated from cells or tissues in which they are naturally expressed or recombinantly produced using techniques well known in the art and/or those described herein. In one embodiment, the CLDN18.2 described herein is human CLDN18.2.

The term "anti-CLDN18.2 antibody", "anti-CLDN18.2", "CLDN18.2 antibody", "antibody that binds to CLDN18.2", or "antibody that specifically binds to CLDN18.2" used herein refers to an antibody capable of binding to (human) CLDN18.2 with sufficient affinity such that the antibody can be used as a therapeutic agent targeting (human) CLDN18.2. In one embodiment, the (human) CLDN18.2 antibody binds to (human) CLDN18.2 with high affinity *in vitro* or *in vivo.* In one embodiment, the (human) CLDN18.2 antibody does not bind to CLDN18.1. In one embodiment, the (human) CLDN18.2 antibody binds to cells expressing CLDN18.2 but does not bind to cells expressing CLDN18.1. In some embodiments, the binding is determined, e.g., by radioimmunoassay (RIA), bio-layer interferometry (BLI), MSD assay, surface plasmon resonance (SPR), or flow cytometry.

Expression of CLDN18.2 in a cell can be determined by a variety of means, such as an anti-CLDN18.2 antibody. For example, the binding strength (e.g., determined by FACS) of a cell with "high expression of CLDN18.2" for an anti-CLDN18.2 antibody may be 500 times, 600 times, 700 times, 800 times, 900 times, or preferably no less than 1000 times, e.g., 1100 times, 1200 times, 1300 times, or no less than 1400 times or more, of the binding strength of an anti-CLDN18.2 antibody for a cell not expressing CLDN18.2. For example, the binding strength (e.g., determined by FACS) of a cell "moderately expressing CLDN18.2" for an anti-CLDN18.2 antibody may be 5 times to 500 times, e.g., 6 times, 7 times, 8 times, 9 times, 10 times, 15 times, 20 times, 30 times, 40 times, 50 times, 60 times, 70 times, 80 times, 90 times, 100 times or more, but no more than 500 times, of the binding strength of an anti-CLDN18.2 antibody for a cell not expressing CLDN18.2.

The terms "complete antibody", "whole antibody", and "full-length antibody" are used interchangeably herein to refer to an antibody molecule having the structure of a native immunoglobulin molecule. In the case of a conventional four-chain IgG antibody, the full-length antibody comprises two heavy chains (H) and two light chains (L) interconnected by disulfide bonds. In the case of a heavy chain antibody having only heavy chains but lacking light chains, the full-length antibody comprises two heavy chains (H) interconnected by disulfide bonds.

For the conventional four-chain IgG antibody, the full-length antibody heavy chain generally consists of a heavy chain variable region (abbreviated herein as VH) and a heavy chain constant region, wherein the heavy chain constant region comprises at least 3 domains CH1, CH2, and CH3. The full-length antibody light chain consists of a light chain variable region (abbreviated herein as VL) and a light chain constant region, wherein the light chain constant region consists of one domain CL. Each heavy chain variable region VH or each light chain variable region consists of three CDRs and 4 FRs, arranged from amino-terminus to carboxyl-terminus in the following order: FR1, CDR1, FR2, CDR2, FR3, CDR3, and FR4.

The term "antibody fragment" includes a portion of an intact antibody. In a preferred embodiment, the antibody fragment is an antigen-binding fragment.

"Antigen-binding fragment" refers to a molecule different from an intact antibody, which comprises a portion of the intact antibody and binds to an antigen to which the intact antibody binds. Examples of the antibody fragments include, but are not limited to, an Fv, a Fab, a Fab', a Fab'-SH, a $F(ab')_2$, a dAb (domain antibody), a linear antibody, a single-chain antibody (e.g., scFv), a single-domain antibody (e.g., VHH), a bi-valent antibody or a fragment thereof, or a camelid antibody.

The term "antigen" refers to a molecule that induces an immune response. Such an immune response may involve antibody production or activation of specific immune cells, or both. Those skilled will understand that any macromolecules, including essentially all proteins or peptides, can be used as antigens. In addition, an antigen may be derived from recombinant or genomic DNA. As used herein, the term "epitope" refers to a moiety of an antigen (e.g., CLDN18.2) that specifically interacts with an antibody molecule.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to antigen epitopes. CDRs of

heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; Al-Lazikani et al., "Standard conformations for the canonical structures of immunoglobulins", Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures (North et al., "A New Clustering of Antibody CDR Loop Conformations", Journal of Molecular Biology, 406, 228-256, (2011)).

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

CDRs can also be determined based on having the same Kabat numbering positions as a reference CDR sequence (e.g., any one of the exemplary CDRs of the present invention).

Unless otherwise stated, the term "CDR" or "CDR sequence" used herein encompasses CDR sequences determined by any one of the schemes described above.

Unless otherwise stated, residue positions of an antibody variable region (including heavy chain variable region residues and light chain variable region residues) used herein are numbered according to the Kabat numbering system (Kabat et al., Sequences of Proteins of Immunological Interest, 5th Ed., Public Health Service, National Institutes of Health, Bethesda, Md. (1991)).

In one embodiment, the heavy chain variable region CDRs of the antibody of the present invention are determined according to the following rules:

The VH CDR1 is determined according to AbM rules, and VH CDR2 and VH CDR3 are both determined according to Kabat rules.

In one embodiment, the light chain variable region CDRs of the antibody of the present invention are determined according to Kabat rules.

In one embodiment, the heavy chain variable region CDRs of the antibody of the present invention are determined according to the following rules: the VH CDR1 is determined according to AbM rules, and the VH CDR2 and CDR3 are both determined according to Kabat rules; and the light chain variable region CDRs are determined according to Kabat rules.

It should be noted that boundaries of CDRs in variable regions of the same antibody determined by different assignment systems may differ. That is, CDR sequences of variable regions of the same antibody defined by different assignment systems differ. Therefore, when it comes to defining an antibody with specific CDR sequences defined in the present invention, the scope of antibody also encompasses such antibodies whose variable region sequences comprise the specific CDR sequences but having claimed CDR boundaries different from the specific CDR boundaries

defined by the present invention due to a different protocol (e.g., different assignment system rules or their combinations) applied.

Antibodies with different specificities (i.e., different binding sites for different antigens) have different CDRs (under the same assignment system). However, although CDRs vary from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the smallest binding unit may remain unchanged, while the remaining CDR residues defined according to Kabat or Chothia may be replaced by conservative amino acid residues.

The term "Fc region" is used herein to define a C-terminus region of an immunoglobulin heavy chain, which comprises at least a portion of a constant region. The term includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies, or comprises the second, the third, and the fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering system (also known as the EU Index) as described in, for example, Kabat et al., Sequences of Proteins of Immunological Interes, 5th Ed., Public Health Service, National Institutes of Health, Bethesda, MD, 1991. Herein, the term "Fc region" does not comprise a heavy chain variable region VH and a light chain variable region VL as well as a heavy chain constant region CH1 and a light chain constant region CL of an immunoglobulin, but in some cases, it may comprise a hinge region at the N-terminus of the heavy chain constant region.

"Antibody in the form of IgG" refers to the IgG form that the heavy chain constant region of the antibody belongs to. Heavy chain constant regions of all antibodies of the same type are identical, and heavy chain constant regions of antibodies of different types are different. For example, an antibody in the form of IgG4 means that the heavy chain constant region of the antibody is from IgG4, or an antibody in the form of IgG1 means that the heavy chain constant region of the antibody is from IgG1.

As used herein, the term "bind to" or "specifically bind to" means that the binding effect is selective for antigens and may be distinguished from unwanted or non-specific interactions. The ability of an antigen-binding site to bind to a particular antigen may be determined by enzyme-linked immunosorbent assay (ELISA) or conventional binding assays known in the art, such as radioimmunoassay (RIA), bio-layer interferometry, MSD assay, or surface plasmon resonance (SPR).

As used herein, an "antibody-drug conjugate (ADC)" refers to a structure obtained by linking an antibody and a drug.

The general term "saccharide" used herein refers to a monosaccharide, such as glucose (Glc), galactose (Gal), mannose (Man), and fucose (Fuc). The term "saccharide derivative" used herein refers to a derivative of a monosaccharide, i.e., a monosaccharide comprising a substituent and/or a functional group. Examples of saccharide derivatives include amino saccharides and saccharide acids, such as glucosamine (GlcN), galactosamine (GalN), N-acetylglucosamine (GlcNAc), N-acetylgalactosamine (GalNAc), N-acetylneuraminic acid (NeuNAc), N-acetylmuramic acid (MurNAc), glucuronic acid (GlcA), and iduronic acid (IdoA). Examples of saccharide derivatives also include compounds denoted herein as E(A)x, wherein E is a saccharide or a saccharide derivative, and wherein E comprises x functional groups A.

The core-N-acetylglucosamine substituent (core-GlcNAc substituent) is defined herein as GlcNAc being bonded to an antibody by C1, preferably being bonded to an amide nitrogen atom on the side chain of the asparagine amino acid of the antibody via an N-glycosidic bond. The core-GlcNAc substituent may be present at a native glycosylation site of the antibody, but it may also be introduced to a different site on the antibody. Herein, the core-N-acetylglucosamine substituent is a monosaccharide substituent or (if the core-GlcNAc substituent is fucosylated) a disaccharide core-(Fuc$\alpha$1-6)GlcNAc substituent, also referred to as GlcNAc(Fuc).

"Glycosylation engineering" refers to the process of performing glycosylation engineering to change the glycan chain of an antibody. Glycosylation of antibodies may be further engineered for a variety of purposes to obtain antibodies with novel glycosylation, e.g., removing glycosylation to eliminate FcγR affinity and complement fixation/effector function, or reducing fucose and sialic acid groups and increasing bisecting N-acetylglucosamine, galactose, and mannose for enhancing Fc-mediated ADCC and CDC effects. Glycosylation engineering means are known in the art, such as increasing or decreasing the glycan chains on the antibody surface by changing glycosylation sites of the antibody, modifying the glycan chains chemically or enzymatically *in vitro,* catalyzing glycosylation of the antibody by changing glycosylation pathways of the expression system (e.g., consisting of enzymes such as glycosidase and glycosyltransferase), and changing the glycosylation of the antibody by specific cell culture conditions. In some embodiments, the "glycosylation engineering" of the present invention is performed by enzymatic modification of glycan chains *in vitro*. Preferably, the glycosylation engineering of the present invention is performed by modifying the glycan chain with a glycosidase (e.g., endoglycosidase or glycosyltransferase).

The antibody with engineered glycosylation of the present invention refers to an antibody whose glycosylation pattern is engineered compared with antibodies with native glycosylation patterns. Preferably, the antibody with engineered glycosylation refers to an antibody obtained after the antibody expressed in an expression system (e.g., mammalian cells) has been enzymatically modified for glycan chains *in vitro* (e.g., the glycan chains have been modified by glycosidases (e.g., endoglycosidases or glycosyltransferases)). More preferably, the antibody with engineered glycosylation of the present invention refers to an antibody comprising core-GlcNAc and a saccharide derivative E(A)x linked thereto, wherein GlcNAc is bonded to the antibody by C1, preferably being bonded to an amide nitrogen atom on the side chain of the asparagine amino acid of the antibody via an N-glycosidic bond. If the -GlcNAc substituent of the GlcNAc-E(A)x substituent is fucosylated, typically fucose is linked to C6 of the-GlcNAc substituent via $\alpha$-1,6. A fucosylated -GlcNAc substituent refers to core-GlcNAc(Fuc), and a fucosylated GlcNAc-E(A)x substituent refers to GlcNAc(Fuc)-E(A)x.

The term "site-specific conjugation" described herein refers to conjugation characterized by specifically linking a drug/an active substance to a specific site of an antibody via a linker.

The term "alkyl" described herein refers to a fully saturated branched or unbranched hydrocarbon group. Alkyl preferably contains 1-24 carbon atoms, and more preferably 1-16 carbon atoms, 1-12 carbon atoms, 1-6 carbon atoms, or 1-4 carbon atoms. Representative examples of alkyl include, but are not limited to, methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, and the like.

The term "aryl" refers to a monocyclic or bicyclic aromatic hydrocarbon group having 6-20, e.g., 6-12, carbon atoms in the ring moiety. Preferably, aryl is ($C_6$-$C_{10}$)aryl. Non-limiting examples include phenyl, biphenyl, naphthyl, or tetrahydronaphthyl, each of which may be optionally substituted with 1-4 substituents, e.g., alkyl, trifluoromethyl, cycloalkyl, halogen, hydroxy, alkoxy, acyl, alkyl-C(O)-O-, aryl-O-, heteroaryl-O-, amino, sulfhydryl, alkyl-S-, aryl-S-, nitro, cyano, carboxyl, alkyl-O-C(O)-, carbamoyl, alkyl-S(O)-, sulfonyl, sulfonamido, heterocyclyl, and the like, wherein R is independently hydrogen, alkyl, aryl, heteroaryl, aryl-alkyl-, heteroaryl-alkyl-, and the like.

The term "cycloalkyl" is a cyclic alkyl group, that is, a monovalent, saturated, or unsaturated hydrocarbon group having a cyclic structure. Cycloalkyl includes all saturated or partially saturated (containing 1 or 2 double bonds) hydrocarbon groups having a cyclic structure. Cycloalkyl groups may contain 3 or more, e.g., 3-18, 3-10, or 3-8, carbon atoms in the ring, and typically, according to the present invention, contain 3 to 6 atoms. Examples of cycloalkyl groups include, but are not limited to, cyclopropyl, cyclobutyl, cyclopentyl, and cyclohexyl.

As used herein, the term "heteroaryl" refers to a 5-20 membered (e.g., 5-14 membered, 5-8 membered, or 5-6 membered) monocyclic-, bicyclic-, or fused polycyclic ring system containing 1-8 heteroatoms selected from N, O, or S. Preferably, heteroaryl is a 5-10 membered ring system. Representative heteroaryl groups include 2- or 3-thienyl, 2- or 3-furanyl, 2- or 3-pyrrolyl, 2-, 4- or 5-imidazolyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 3- or 5-1,2,4-triazolyl, 4- or 5-1,2,3-triazolyl, tetrazolyl, 2-, 3- or 4-pyridyl, 3- or 4-pyridazinyl, 3-, 4- or 5-pyrazinyl, 2-pyrazinyl, and 2-, 4- or 5-pyrimidinyl.

The term "pharmaceutically acceptable salt" refers to salts that retain the biological effects and properties of the ADCs of the present invention and are not biologically or otherwise undesired. The ADCs of the present invention may exist in their pharmaceutically acceptable salt forms, including acid addition salts and base addition salts. In the present invention, pharmaceutically acceptable non-toxic acid addition salts refer to salts formed by the ADCs of the present invention and organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. Pharmaceutically acceptable non-toxic base addition salts refer to salts formed by the ADCs of the present invention and organic or inorganic bases including, but not limited to, alkali metal salts (e.g., lithium, sodium, or potassium salts), alkaline earth metal salts (e.g., calcium or magnesium salts), and organic base salts (e.g., ammonium salts formed by reaction with N group-containing organic bases).

The term "solvate" refers to an association compound formed by the ADC of the present invention and one or more solvent molecules. Solvents for forming solvates include, but are not limited to, water, methanol, ethanol, isopropanol, ethyl acetate, tetrahydrofuran, *N,N*-dimethylformamide, dimethylsulfoxide, and the like.

"Pharmaceutically acceptable" and "pharmaceutical" are used interchangeably herein, provided that there is no contradiction according to the context.

The term "drug-to-antibody ratio" or "DAR" refers to the ratio of the small molecule drug moiety (D) conjugated to the Ab moiety described herein to the Ab moiety. In some embodiments described herein, DAR may be determined by p and r in formula I; for example, the DAR may be 1-20, e.g., 2-18, 4-16, 5-12, 6-10, 2-8, 3-8, 2-6, 4-6, or 6-10, e.g.,

2, 3, 4, 5, 6, 7, 8, 9, 10, 11, 12, 13, 14, or 15. DAR may also be calculated as the average DAR of the molecular population in the product, i.e., the overall ratio of the small molecule drug moiety (D) conjugated to the Ab moiety described herein to the Ab moiety in the product as measured by detection methods (e.g., by conventional methods such as mass spectrometry, ELISA assay, electrophoresis, and/or HPLC), and such a DAR is referred to herein as average DAR. In some embodiments, the average DAR of the conjugate of the present invention is 1-20, e.g., 2-18, 4-16, 5-12, 6-10, 2-8, 3-8, 2-6, 4-6, or 6-10, e.g., 1.0-8.0 or 2.0-6.0, e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and ranges with any two of these values as endpoints.

The term "therapeutic agent" described herein encompasses any substance that is effective in preventing or treating a tumor, e.g., cancer, including a chemotherapeutic agent, a cytokine, an angiogenesis inhibitor, a cytotoxic agent, other antibodies, a small molecule drug, or an immunomodulatory agent (e.g., an immunosuppressant).

The term "cytotoxic agent" used herein refers to a substance that inhibits or prevents cell functions and/or causes cell death or cell destruction.

"Chemotherapeutic agents" include chemical compounds useful in the treatment of cancer or immune system diseases.

The term "small molecule drug" refers to an organic compound having a low molecular weight and capable of regulating biological processes. "Small molecule" is defined as a molecule with a molecular weight of less than 10 kD, usually less than 2 kD, and preferably less than 1 kD. The small molecule includes, but is not limited to, inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation, and are less likely to induce an immune response compared with large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant. In some embodiments, the immunomodulatory agent of the present invention includes an immune checkpoint inhibitor or an immune checkpoint agonist.

The term "effective amount" refers to an amount or dosage of the antibody, fragment, composition, or combination of the present invention which generates expected effects in a patient in need of treatment or prevention after being administered to the patient in a single dose or multiple doses.

"Therapeutically effective amount" refers to an amount effective to achieve a desired therapeutic result at a necessary dose for a necessary period of time. The therapeutically effective amount is also such an amount that any toxic or undesired effect of the antibody, fragment thereof, composition, or combination is inferior to the therapeutically beneficial effect. The "therapeutically effective amount" preferably inhibits a measurable parameter (e.g., tumor volume) by at least about 30%, and even more preferably by at least about 40%, 45%, 50%, 55%, 60%, 65%, 70%, 75%, 80%, 85%, 90%, or even 100%, relative to untreated subjects.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The terms "host cell", "host cell line", and "host cell culture" are used interchangeably and refer to cells into which exogenous nucleic acids are introduced, including progenies of such cells. Host cells include "transformants" and "transformed cells", which include primary transformed cells and progenies derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein.

The term "label" used herein refers to a compound or composition which is directly or indirectly conjugated or fused to an agent, such as a polynucleotide probe or an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or an antibody and indirect labeling of a probe or antibody by reacting with another reagent which is directly labeled.

"Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human.

An "isolated" antibody or another molecule (e.g., an ADC molecule) is an antibody or molecule that has been separated from its natural environment or components of the environment in which it is expressed. In some embodiments,

the antibody or ADC molecule is purified to a purity greater than 95% or 99% as determined by, e.g., electrophoresis (e.g., SDS-PAGE, isoelectric focusing (IEF), and capillary electrophoresis) or chromatography (e.g., ion exchange or reverse-phase HPLC).

The term "anti-tumor effect" refers to a biological effect that can be demonstrated by a variety of means, including but not limited to, for example, decrease in tumor volume, decrease in number of tumor cells, decrease in tumor cell proliferation, or decrease in tumor cell viability.

The terms "tumor" and "cancer" are used interchangeably herein and encompass solid and hematological tumors.

The terms "cancer" and "cancerous" refer to or describe a physiological disease in mammals that is typically characterized by unregulated cell growth. In certain embodiments, cancers suitable for treatment with the antibody of the present invention include gastric cancer, pancreatic cancer, or gastroesophageal junction cancer, including metastatic forms of such cancers.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein.

The term "pharmaceutical supplementary material" refers to diluents, adjuvants (e.g., Freund's adjuvants (complete and incomplete)), excipients, carriers, stabilizers, or the like, that are administered with the active substance.

The term "pharmaceutical composition" refers to a composition that exists in a form allowing effective biological activity of the active ingredient contained therein and does not contain additional ingredients having unacceptable toxicity to a subject to which the composition is administered.

The term "pharmaceutical combination" refers to a non-fixed combination product or a fixed combination product, including but not limited to a kit and a pharmaceutical composition. The term "non-fixed combination" means that the active ingredients (e.g., (i) the ADC molecule of the present invention, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. In some embodiments, the ADC molecule of the present invention of the present invention and the additional therapeutic agent used in the pharmaceutical combination are administered at levels that do not exceed their levels when used alone. The term "fixed combination" means that two or more active agents are administered to a patient simultaneously in the form of a single entity. The dose and/or time intervals of two or more active agents are preferably selected such that the combined use of the components can result in a therapeutic effect on the disease or condition which is greater than that achieved by the use of either component alone. The ingredients may each take a separate formulation form and such separate formulation forms may be the same or different.

The term "combination therapy" refers to the administration of two or more therapeutic agents or therapeutic modalities (e.g., radiotherapy or surgery) to treat the diseases described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powder and liquid). The powder and/or liquid can be reconstituted or diluted to a desired dose before administration. In addition, such administration also includes using all types of therapeutic agents at approximately the same time or in a sequential manner at different times. In any case, the therapeutic regimen will provide the beneficial effect of the pharmaceutical combination in the treatment of conditions or symptoms described herein.

As used herein, "treatment", "treat", or "treating" refers to slowing, interrupting, arresting, alleviating, stopping, lowering, or reversing the progression or severity of an existing symptom, condition, disorder, or disease.

As used herein, "prevention", "prevent", or "preventing" includes the inhibition of the development or progression of symptoms of a disease or condition, or a specific disease or condition. In some embodiments, subjects with family history of cancer are candidates for preventive regimens. Generally, in the context of cancer, the term "prevention" "prevent", or "preventing" refers to the administration of a drug prior to the onset of signs or symptoms of cancer, particularly in subjects at risk of cancer.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are referred to as "expression vectors" herein.

"Subject/patient/individual sample" refers to a collection of cells or fluids obtained from a patient or a subject. The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds that are naturally not mixed with tissues, such as preservatives, anticoagulants, buffers, fixatives, nutrients, and antibiotics.

II. Antibody-drug conjugate

The present invention provides an antibody-drug conjugate having formula (I):

$$Ab\text{-}(L\text{-}(D)_r)_p \quad (I)$$

or a pharmaceutically acceptable salt or solvate thereof,
wherein:

Ab is an antibody or a fragment thereof that binds to CLDN18.2 (e.g., human CLDN18.2); L is a linker;
D is a drug, including a prodrug, preferably an anti-tumor compound; and
p is 1-10, e.g., 1-9, 2-8, 3-7, 4-6, or 2-6, such as 1, 2, 3, 4, 5, 6, 7, 8, 9, or 10.
r is 1-5, e.g., 1, 2, 3, 4, or 5, preferably 1 or 2.

In some embodiments, Ab in the formula (I) of the present invention is a human or humanized antibody, preferably a human antibody. In some embodiments, Ab in the formula (I) of the present invention is an antibody fragment, preferably an antigen-binding fragment, such as an Fv, a Fab, a Fab', a Fab'-SH, a $F(ab')_2$, a domain antibody (dAb), a linear antibody, a single-chain antibody (e.g., scFv), a single-domain antibody (e.g., VHH), a bi-valent antibody or a fragment thereof, or a camelid antibody.

In some embodiments, Ab in the formula (I) of the present invention is a bispecific or multispecific antibody.

In a preferred embodiment of the present invention, Ab comprises 3 complementarity determining regions from the heavy chain variable region (HCDRs), i.e., HCDR1, HCDR2, and HCDR3.

In a preferred embodiment of the present invention, Ab comprises 3 complementarity determining regions from the light chain variable region (LCDRs), i.e., LCDR1, LCDR2, and LCDR3.

In some embodiments, Ab comprises 3 complementarity determining regions from the heavy chain variable region (HCDRs) and 3 complementarity determining regions from the light chain variable region (LCDRs).

In some aspects, Ab comprises a heavy chain variable region (VH). In some aspects, Ab comprises a light chain variable region (VL). In some aspects, Ab comprises a heavy chain variable region and a light chain variable region. In some embodiments, the heavy chain variable region comprises 3 complementarity determining regions from the heavy chain variable region (HCDRs), i.e., HCDR1, HCDR2, and HCDR3. In some embodiments, the light chain variable region comprises 3 complementarity determining regions from the light chain variable region (LCDRs), i.e., LCDR1, LCDR2, and LCDR3.

In some embodiments, the VH

(i) comprises or consists of an amino acid sequence having at least 90%, 91%, 92%, 93%, 94%, 95%, 96%, 97%, 98%, or 99% identity to the amino acid sequence set forth in SEQ ID NO: 4; or
(ii) comprises or consists of the amino acid sequence set forth in SEQ ID NO: 4; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence set forth in SEQ ID NO: 4, wherein preferably, the amino acid changes do not occur in the CDRs.

In some embodiments, the VL

(i) comprises or consists of an amino acid sequence having at least 90%, 91%, 92%, 93%, 94%, 95%, 96%, 97%, 98%, or 99% identity to the amino acid sequence set forth in SEQ ID NO: 9; or
(ii) comprises or consists of the amino acid sequence set forth in SEQ ID NO: 9; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence set forth in SEQ ID NO: 9, wherein preferably, the amino acid changes do not occur in the CDRs.

In some embodiments, the 3 complementarity determining regions from the heavy chain variable region (HCDRs) of the present invention, i.e., HCDR1, HCDR2, and HCDR3, are

(i) three complementarity determining regions HCDR1, HCDR2, and HCDR3 contained in a VH set forth in SEQ ID NO: 4, or

(ii) relative to the sequence in (i), a sequence comprising a total of at least one and no more than 5, 4, 3, 2, or 1 amino acid change (preferably amino acid replacement, and more preferably conservative replacement) in the three HCDRs.

In some embodiments, the 3 complementarity determining regions from the light chain variable region (LCDRs) of the present invention, i.e., LCDR1, LCDR2, and LCDR3, are

(i) three complementarity determining regions LCDR1, LCDR2 and LCDR3 contained in a VL set forth in SEQ ID NO: 9,
(ii) relative to the sequence in (i), a sequence comprising a total of at least one and no more than 5, 4, 3, 2, or 1 amino acid change (preferably amino acid replacement, and more preferably conservative replacement) in the three LCDRs.

In some embodiments, the HCDR1 comprises or consists of the amino acid sequence set forth in SEQ ID NO: 1, or the HCDR1 comprises an amino acid sequence having one, two, or three changes (preferably amino acid replacements, and more preferably conservative replacements) compared with the amino acid sequence set forth in SEQ ID NO: 1.
In some embodiments, the HCDR2 comprises or consists of the amino acid sequence set forth in SEQ ID NO: 2, or the HCDR2 comprises an amino acid sequence having one, two, or three changes (preferably amino acid replacements, and more preferably conservative replacements) compared with the amino acid sequence set forth in SEQ ID NO: 2.
In some embodiments, the HCDR3 comprises or consists of the amino acid sequence set forth in SEQ ID NO: 3, or the HCDR3 comprises an amino acid sequence having one, two, or three changes (preferably amino acid replacements, and more preferably conservative replacements) compared with the amino acid sequence set forth in SEQ ID NO: 3.
In some embodiments, the LCDR1 comprises or consists of the amino acid sequence set forth in SEQ ID NO: 6, or the LCDR1 comprises an amino acid sequence having one, two, or three changes (preferably amino acid replacements, and more preferably conservative replacements) compared with the amino acid sequence set forth in SEQ ID NO: 6.
In some embodiments, the LCDR2 comprises or consists of the amino acid sequence set forth in SEQ ID NO: 7, or the LCDR2 comprises an amino acid sequence having one, two, or three changes (preferably amino acid replacements, and more preferably conservative replacements) compared with the amino acid sequence set forth in SEQ ID NO: 7.
In some embodiments, the LCDR3 comprises or consists of the amino acid sequence set forth in SEQ ID NO: 8, or the LCDR3 comprises an amino acid sequence having one, two, or three changes (preferably amino acid replacements, and more preferably conservative replacements) compared with the amino acid sequence set forth in SEQ ID NO: 8.
In some embodiments, Ab in the formula (I) of the present invention comprises a heavy chain constant region. In some embodiments, Ab in the formula (I) of the present invention comprises a light chain constant region. In some embodiments, Ab in the formula (I) of the present invention further comprises a heavy chain constant region and a light chain constant region.
In some embodiments, the heavy chain constant region HC of the present invention is a heavy chain constant region of IgG1, IgG2, IgG3, or IgG4, preferably a heavy chain constant region of IgG1, such as a heavy chain constant region of a wild-type IgG1. In some embodiments, the antibody light chain constant region LC of the present invention is a lambda or Kappa light chain constant region.
In some preferred embodiments, the heavy chain constant region HC of the present invention

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 5;
(ii) comprises or consists of the amino acid sequence set forth in SEQ ID NO: 5; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 5.

In some embodiments, the antibody light chain constant region LC of the present invention

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 10;

(ii) comprises or consists of the amino acid sequence set forth in SEQ ID NO: 10; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 10.

In some specific embodiments of the present invention, Ab in the formula (I) of the present invention comprises a heavy chain. In some embodiments of the present invention, Ab in the formula (I) of the present invention comprises a light chain. In some embodiments of the present invention, Ab in the formula (I) of the present invention comprises a heavy chain and a light chain. In some specific embodiments of the present invention, the heavy chain of the present invention comprises or consists of a heavy chain variable region and a heavy chain constant region. In some specific embodiments of the present invention, the light chain of the present invention comprises or consists of a light chain variable region and a light chain constant region.
In some specific embodiments, Ab in the formula (I) of the present invention specifically binds to CLDN18.2 and comprises three complementarity determining regions HCDR1, HCDR2, and HCDR3 contained in the VH set forth in SEQ ID NO: 4 and/or three complementarity determining regions LCDR1, LCDR2, and LCDR3 contained in the VL set forth in SEQ ID NO: 9.
In some specific embodiments of the present invention, Ab in the formula (I) of the present invention comprises HCDR1, HCDR2, and HCDR3 having amino acid sequences: set forth in SEQ ID NOs:1, 2, and 3, respectively, and/or LCDR1, LCDR2, and LCDR3 having amino acid sequences set forth in SEQ ID NOs: 6, 7, and 8, respectively.
In some specific embodiments of the present invention, Ab in the formula (I) of the present invention comprises

a VH comprising or consisting of the amino acid sequence set forth in SEQ ID NO: 4 or an amino acid sequence having at least 90%, 91%, 92%, 93%, 94%, 95%, 96%, 97%, 98%, or 99% identity thereto; and/or
a VL comprising or consisting of the amino acid sequence set forth in SEQ ID NO: 9 or an amino acid sequence having at least 90% identity thereto.

In some embodiments of the present invention, Ab in the formula (I) of the present invention comprises a VH and a VL, wherein the amino acid sequence of the VH is set forth in SEQ ID NO: 4 and the amino acid sequence of the VL is set forth in SEQ ID NO: 9.
In some specific embodiments of the present invention, Ab in the formula (I) of the present invention is an IgG antibody, i.e., it comprises a heavy chain and a light chain that bind to CLDN18.2. In some embodiments, Ab in the formula (I) of the present invention is a complete antibody.
In some embodiments, the heavy chain of Ab in the formula (I)

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 11;
(ii) comprises or consists of the amino acid sequence set forth in SEQ ID NO: 11; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 11.

In some embodiments, the light chain of Ab in the formula (I)

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 12;
(ii) comprises or consists of the amino acid sequence set forth in SEQ ID NO: 12; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 12.

In some embodiments of the present invention, Ab in formula (I) of the present invention comprises a heavy chain and a light chain, wherein the amino acid sequence of the heavy chain is set forth in SEQ ID NO: 11 and the amino acid sequence of the light chain is set forth in SEQ ID NO: 12.
In one embodiment of the present invention, the amino acid change described herein includes amino acid replacement, insertion, or deletion. Preferably, the amino acid change described herein is an amino acid replacement,

preferably a conservative replacement. In a preferred embodiment, the amino acid change described herein occurs in a region outside the CDR (e.g., in FR). More preferably, the amino acid change described herein occurs in a region outside the heavy chain variable region and/or outside the light chain variable region. In some embodiments, the amino acid change described herein occurs in the Fc region of the heavy chain constant region of the antibody.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. In certain embodiments, the replacement occurs in the CDRs of the antibody. Generally, the obtained variant has modifications (e.g., improvements) in certain biological properties (e.g., increased affinity) relative to the parent antibody and/or will substantially retain certain biological properties of the parent antibody. Exemplary replacement variants are affinity-matured antibodies.

The antibody Ab in the formula (I) of the present invention may be an antibody with engineered glycosylation. In some embodiments, the antibody is an antibody obtained being enzymatically modified for glycan chains *in vitro* (e.g., the glycan chains are modified by glycosidases (e.g., endoglycosidases or glycosyltransferases)). In some embodiments, the antibody with engineered glycosylation is an antibody in which a glycan chain at a glycosylation site of the antibody is engineered from an N-glycan chain with a non-homogeneous structure to an N-glycan chain with a single structure having a reactive group (e.g., any reactive group capable of reacting with a linker moiety, such as azido, keto, and alkynyl). In a preferred embodiment, the N-glycosylation site of the antibody is a conservative N-glycosylation site, e.g., Asn297, on the Fc domain of the antibody.

Suitable methods for engineering antibody glycosylation of the present invention are described, e.g., in PCT/NL2013/050744, PCT/EP2016/059194, or PCT/EP2017/052792, which are incorporated herein in their entireties.

In a preferred embodiment, the antibody with engineered glycosylation of the present invention is an antibody comprising a GlcNAc-$E(A)_x$ substituent, wherein GlcNAc is N-acetylglucosamine, and E(A)x is a saccharide derivative comprising x functional groups A, wherein A is independently selected from azido, keto, and alkynyl, and x is 1, 2, 3, or 4; wherein the GlcNAc-E(A)x substituent is bonded to the antibody by C1 of the N-acetylglucosamine of the GlcNAc-E(A)x substituent, wherein the N-acetylglucosamine is optionally fucosylated. In the case that N-acetylglucosamine is fucosylated, it is bonded to fucose (Fuc) by C6.

In one embodiment, the antibody with engineered glycosylation of the present invention is an antibody of formula (III), wherein Ab represents an antibody, GlcNAc is N-acetylglucosamine, Fuc is fucose, b is 0 or 1, y is 1-20, and E(A)x is a saccharide derivative comprising x functional groups A, wherein A is azido, keto, and alkynyl, and x is 1, 2, 3, or 4. In a preferred embodiment, y is 1-10; more preferably, y is 1, 2, 3, 4, 5, 6, 7, or 8; even more preferably, y is 1, 2, 3, or 4; most preferably, y is 1 or 2.

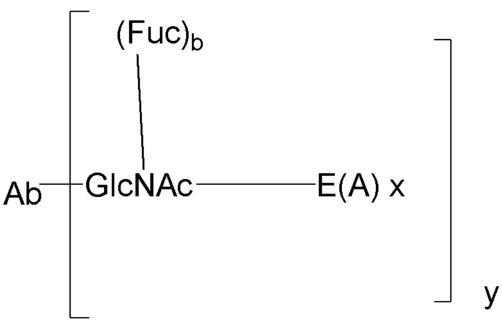

(formula III)

The saccharide derivative E(A)x in the GlcNAc-E(A)x substituent of the antibody with engineered glycosylation may, e.g., be bonded to C4 of the GlcNAc by a P(1,4)-glycosidic bond or to C3 of the GlcNAc by an α (1,3)-glycosidic bond, preferably to C4 of the GlcNAc by a P(1,4)-glycosidic bond. The N-acetylglucosamine of the GlcNAc-E(A)x substituent is bonded to the antibody by C1, preferably being bonded to an amide nitrogen atom in the side chain of the asparagine of the antibody via an N-glycosidic bond (GlcNAcβ1-Asn). The GlcNAc on the GlcNAc-E(A)x substituent is optionally fucosylated. Accordingly, GlcNAc-E in the antibody-drug conjugate, when present, may also be linked as previously described.

In a preferred embodiment, the functional group A is azido. When A is azido, preferably A is bonded to C2, C3, C4, or C6. As described above, one or more azide substituents in E(A)x may be bonded to C2, C3, C4, or C6 of the saccharide or saccharide derivative E to replace the hydroxyl (OH). It should be understood that the bonding position of the functional group A corresponds to the position where the A-containing saccharide is linked to the linker.

In a preferred embodiment, the saccharide derivative E(A)x is derived from a saccharide or a saccharide derivative E. In a preferred embodiment, E is a saccharide or a saccharide derivative and is selected from galactose

(Gal), mannose (Man), N-acetylglucosamine (GlcNAc), glucose (Glc), N-acetylgalactosamine (GalNAc), glucuronic acid (Gcu), fucose (Fuc), and N-acetylneuraminic acid (sialic acid), preferably Gal, GlcNAc, glucose, and GalNAc, and most preferably GalNAc.

In another preferred embodiment, the E(A)x is GalNAc-Ns; preferably, E(A)x is 6-azido-6-deoxy-2-acetamidogalactose.

It should be understood that, without being contradictory, the descriptions and illustrations above for saccharides (including but not limited to the way of linking via a glycosidic bond in GlcNAc-E(A)x) apply equally to corresponding saccharides in the conjugate of formula (II) below, e.g., the way of linking saccharide therein. It will be appreciated by those skilled in the art that although in some embodiments, a reactive group (e.g., a functional group a) is linked to the glycan chain of an antibody in antibody glycosylation engineering, and thus "an antibody with engineered glycosylation" is defined as an antibody comprising the reactive group, but upon the formation of an antibody-drug conjugate, the reactive group may react with the linker moiety, thereby forming a new group with the linker moiety, such that the new group is considered to be part of the linker in the antibody-drug conjugate.

Preferably, the antibody of the present invention is a monoclonal antibody, more preferably an IgG antibody (e.g., a four-chain IgG antibody), and most preferably an IgG1 antibody. In one embodiment, the antibody is a complete antibody.

When the modified antibody is a complete antibody, the antibody preferably comprises two or more, more preferably two, GlcNAc-E(A)x substituents, and the GlcNAc-E(A)x substituents are optionally fucosylated. However, if the modified antibody is an antibody fragment, such as a Fab or an Fc fragment, the antibody may have only one GlcNAc-E(A)x substituent, which is optionally fucosylated. The GlcNAc-E(A)x substituent may be located at any position of an antibody, provided that the substituent does not interfere with the binding of an antigen to the antigen-binding site of the antibody. In one embodiment, the GlcNAc-E(A)x substituent is located in the Fc domain of an antibody, more preferably in the CH2 domain.

As described above, the antibody with engineered glycosylation of the present invention comprises no less than one GlcNAc-E(A)x substituent, e.g., 2 GlcNAc-E(A)x substituents.

In a preferred embodiment, the GlcNAc-E(A)x substituent is present at a native N-glycosylation site (e.g., a native conservative N-glycosylation site) of the antibody, such as a glycosylation site of the Fc region (more preferably, the CH2 domain). In yet another preferred embodiment, the antibody is an IgG antibody and the GlcNAc-E(A)x substituent is present at a native N-glycosylation site (a native conservative N-glycosylation site) of the IgG antibody. In yet another preferred embodiment, the native site is an Asn297-glycosylation site of an IgG antibody. The Asn297-glycosylation site is present in the Fc region of the heavy chain of the IgG antibody. In a preferred embodiment, the GlcNAc-E(A)x substituent is present at Asn297-glycosylation sites of the two heavy chains of the antibody.

In some embodiments, L in the formula (I) of the present invention is a linker. Any linker known in the art can be used for linking to anti-human CLDN18.2 of the present invention, preferably a linker enabling site-specific conjugation of ADCs.

In some embodiments, a linker suitable for use in the present invention may be any linker that enables conjugation of an antibody to a drug. In some embodiments, the linker may be a linker used in techniques that enable site-specific conjugation.

In a preferred embodiment, the linker of the present invention is a linker that is linked to an antibody oligosaccharide. The "linker linked to an antibody oligosaccharide" defined herein refers to any linker that is linked to a reactive group on a glycan chain at a glycosylation site of an antibody to allow the antibody to be conjugated to a drug. The glycan chain on the glycosylation site of the antibody is usually an N-glycan chain and is generally engineered to change an N-glycan chain with a non-homogeneous structure into an N-glycan chain with a single structure having a reactive group, and then the reactive group carried by the glycan chain is utilized to be linked to a "linker", so that the site-specific conjugation of the drug and the antibody is realized, and the antibody-drug conjugate is obtained. In a preferred embodiment, the N-glycosylation site of the antibody is a conservative N-glycosylation site, e.g., Asn297, on the Fc domain of the antibody, preferably the CH2 domain. Thus, in one embodiment, the "linker linked to an antibody oligosaccharide" of the present invention is particularly any linker enabling site-specific conjugation to a reactive group on the N-glycan chain at a conservative N-glycosylation site (e.g., Asn297) on the Fc domain of an antibody, such as the linker described in PCT/NL2013/050744 or the linker of PCT/EP2021/075401, which are incorporated herein in their entireties. In one embodiment, the reactive group of the present invention is azido, keto, or alkynyl. In one embodiment, the linker of the present invention is a linker comprising an alkynyl group. In one embodiment, the reactive group of the present invention is azido, keto, or alkynyl, preferably azido, and the linker of the present invention is a linker comprising an alkynyl group. In referring to such linkers in the present invention, since the reactive group forms a new group upon reaction with the linker group, the group formed upon reaction of the reactive group in the antibody-drug conjugate may also be defined as part of the "linker", e.g., as shown in the formula (II) of the present invention.

Linkers suitable for use in the present invention also include, e.g., cathepsin-degradable linkers, such as Val-Cit linkers (e.g., vc-PAB), cBu-Cit linkers, and CX linkers; non-cleavable linkers, such as an SMCC linker or an MD

linker; acid-sensitive linkers, silyl ether linkers, disulfide-carbamate linkers, MC-GGFG linkers, TRX linkers, galactoside-containing linkers, pyrophosphate linkers, near infrared-sensitive linkers, UV-sensitive linkers such as PC4AP (Antibody-drug conjugates: Recent advances in linker chemistry, Su, Z., Xiao, D., Xie, F., Liu, L., Wang, Y, Fan, S., Li, S. (2021). Antibody-drug conjugates: Recent advances in linker chemistry. Acta Pharmaceutica Sinicα B*)*. Linkers suitable for use in the present invention may also be combinations of one or more linkers. For example, a cathepsin-degradable linker may be combined with other types of linkers to form a new linker. Thus, the "linker" described herein encompasses a single type of linker or a combination of different types of linkers, as long as it enables conjugation of the antibody of the present invention to a drug. Thus, in one embodiment, the linker suitable for use in the present invention is MC-VC-PAB, vc-PAB, SMCC, or MC-GGFG.

D in the formula (I) of the present invention may be any anti-tumor compound and is not particularly limited as long as it is a compound that has an anti-tumor effect and has a substituent or partial structure capable of being linked to a linker structure. For example, the anti-tumor compound may be a pharmaceutically active compound that acts on a tumor. For an anti-tumor compound, it is preferable that a part or all of the linker is cleaved in a tumor cell to release the anti-tumor compound, thereby exhibiting an anti-tumor effect. When the linker is cleaved where it is linked to the drug, the anti-tumor compound is released in an unmodified structure and exhibits its original anti-tumor effect.

In some embodiments, the anti-tumor compound may be, e.g., a cytotoxic agent or a chemotherapeutic agent, such as camptothecin compounds exatecan (topoisomerase I inhibitor exatecan) and Dxd (a novel topoisomerase I inhibitor exatecan derivative), auristatin compounds such as monomethyl auristatin E (MMAE), or maytansinoid compounds such as small molecule microtubule inhibitor DM1. The structures in the examples of the present application show the structures of representative compounds of these anti-tumor compounds.

In one embodiment, the present invention provides an antibody-drug conjugate of (II) or a pharmaceutically acceptable salt or solvate thereof

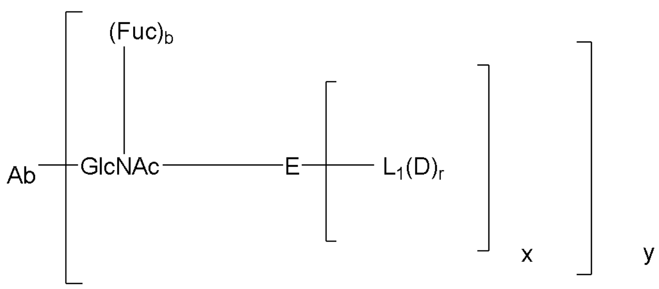

(formula II)

Ab represents an antibody defined herein;
$L_1$ is a linker;
E is a saccharide or a saccharide derivative, such as a saccharide or a saccharide derivative defined above;
GlcNAc is N-acetylglucosamine, and Fuc is fucose;
D and r are as defined above in formula I;
b is 0 or 1, e.g., 0;
x is 1, 2, 3, or 4; preferably 1 or 2;
y is 1-20; for example, y is 1-10; more preferably, y is 1, 2, 3, 4, 5, 6, 7, or 8; even more preferably, y is 1, 2, 3, or 4.

In one embodiment, Fuc is fucose; b is 0 or 1, e.g., 0; and/or x is 1 or 2, more preferably 2; and/or y is 1 or 2, more preferably 2.

In one embodiment, in the formula (II), E is selected from galactose (Gal), mannose (Man), N-acetylglucosamine (GlcNAc), glucose (Glc), N-acetylgalactosamine (GalNAc), glucuronic acid (Gcu), fucose (Fuc), and N-acetylneuraminic acid (sialic acid), preferably Gal, GlcNAc, glucose, and GalNAc, and most preferably GalNAc, e.g., 6-deoxy-2-acetamidogalactose, which is preferably linked to $L_1$ via the C atom at position 6.

In a preferred embodiment, the GlcNAc linked to Ab is present at a native N-glycosylation site (e.g., a native conservative N-glycosylation site) of the antibody, such as a glycosylation site in the Fc region. In yet another preferred embodiment, the antibody is an IgG and the GlcNAc is present at a native N-glycosylation site (e.g., a native conservative N-glycosylation site) of the IgG, e.g., a glycosylation site of the Fc region. In yet another preferred embodiment, the native site is an Asn297-glycosylation site of IgG. The Asn297-glycosylation site is present in the Fc region of the heavy chain of the IgG antibody. In a preferred embodiment, the GlcNAc group is present at Asn297-glycosylation sites of the two heavy chains of the antibody.

In one embodiment, in the formula (II), $L_1$ has the following structure

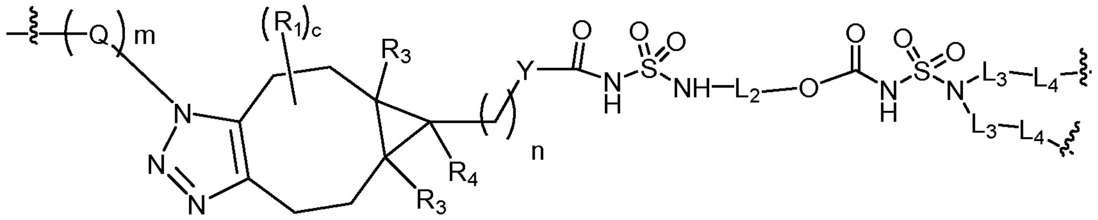

wherein, Q is $-N(H)C(O)CH_2-$ or $-CH_2-$;

$R_1$ is independently selected from hydrogen, halogen, $-OR_2$, $-NO_2$, -CN, $-S(O)_2R_2$, $C_1$-$C_{12}$ alkyl, aryl, heteroaryl, $C_1$-$C_{12}$ alkylaryl, $C_1$-$C_{12}$ alkylheteroaryl, aryl$C_1$-$C_{12}$ alkyl, and heteroaryl$C_1$-$C_{12}$ alkyl, wherein the alkyl, aryl, heteroaryl, alkylaryl, alkylheteroaryl, arylalkyl, and heteroarylalkyl are optionally substituted, wherein two substituents $R_1$ may be linked together to form a fused, bridged, or spiro cycloalkyl or a fused or spiro arene or heteroarene substituent, and wherein $R_2$ is independently selected from hydrogen, halogen, $C_1$-$C_{24}$ alkyl, aryl, heteroaryl, $C_1$-$C_{12}$ alkylaryl, $C_1$-$C_{12}$ alkylheteroaryl, aryl$C_1$-$C_{12}$ alkyl, and heteroaryl$C_1$-$C_{12}$ alkyl;

$R_3$ and $R_4$ are each independently selected from hydrogen, halogen, $C_1$-$C_{24}$ alkyl, aryl, heteroaryl, $C_1$-$C_{12}$ alkylaryl, $C_1$-$C_{12}$ alkylheteroaryl, aryl$C_1$-$C_{12}$ alkyl, and heteroaryl$C_1$-$C_{12}$ alkyl;

c is 0, 1, 2, 3, or 4;

m is 0 or 1;

n is 0, 1, 2, 3, 4, 5, 6, 7, 8, 9, or 10;

Y is O, S, or $NR_1$;

$L_2$ and $L_3$ are each independently $C_1$-$C_{12}$ alkyl, wherein one or more (e.g., 1, 2, 3, or 4) carbon atoms in the alkyl are optionally replaced by a heteroatom selected from O, N, and S, provided that the O, N, and/or S are not directly linked to each other;

$L_4$ are each independently a cleavable linker.

It should be understood that when m is 0, it means that Q is absent and the relevant nitrogen atom of the triazole is therefore directly linked to the E moiety via a covalent single bond.

In one embodiment, $L_4$ are each independently

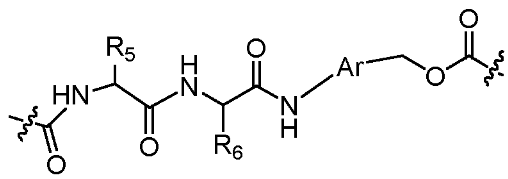

,

wherein, $R_5$ and $R_6$ are each independently selected from hydrogen and $C_1$-$C_{12}$ alkyl;
Ar is selected from aryl, preferably phenyl;

In one embodiment, $L_4$ are each independently

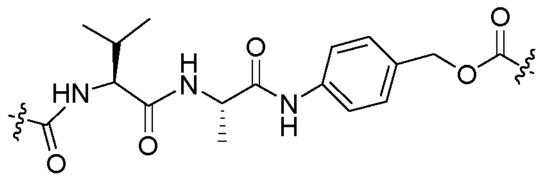

.

In one embodiment, $-L_1-$ has the following structure

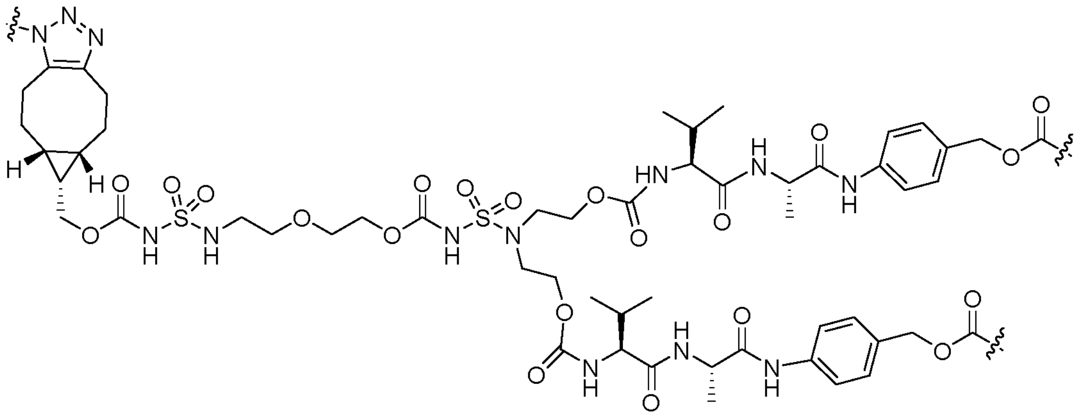

In one embodiment, the antibody-drug conjugate has an average DAR of 1-15, e.g., 1-10, 2-8, 2-6, or 3-5.

In one embodiment, the antibody-drug conjugate of the present invention is selected from:

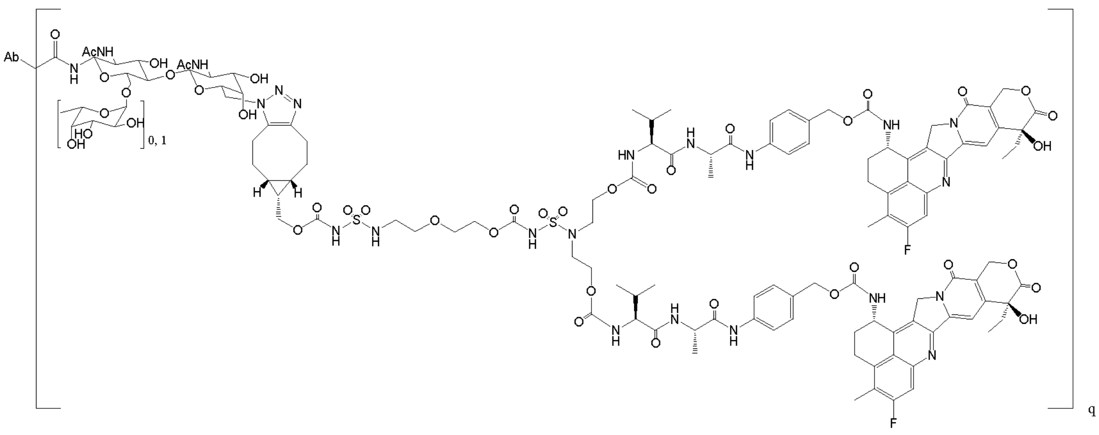

IEX019-02

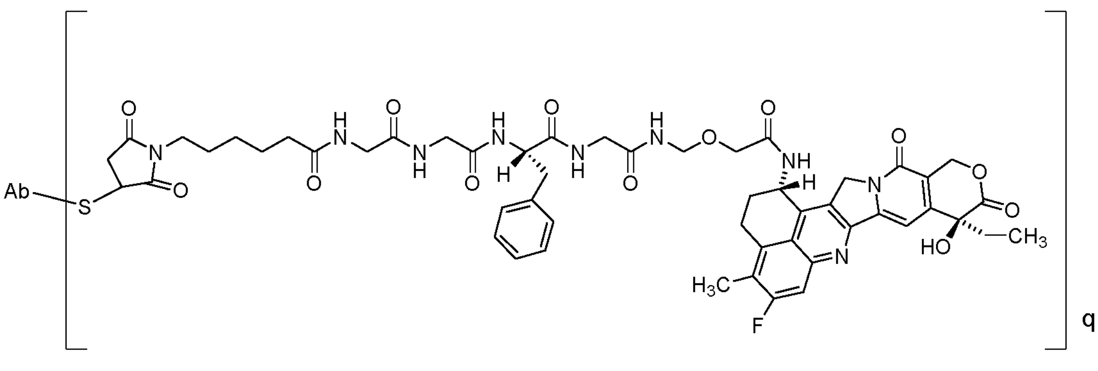

IEX019-03

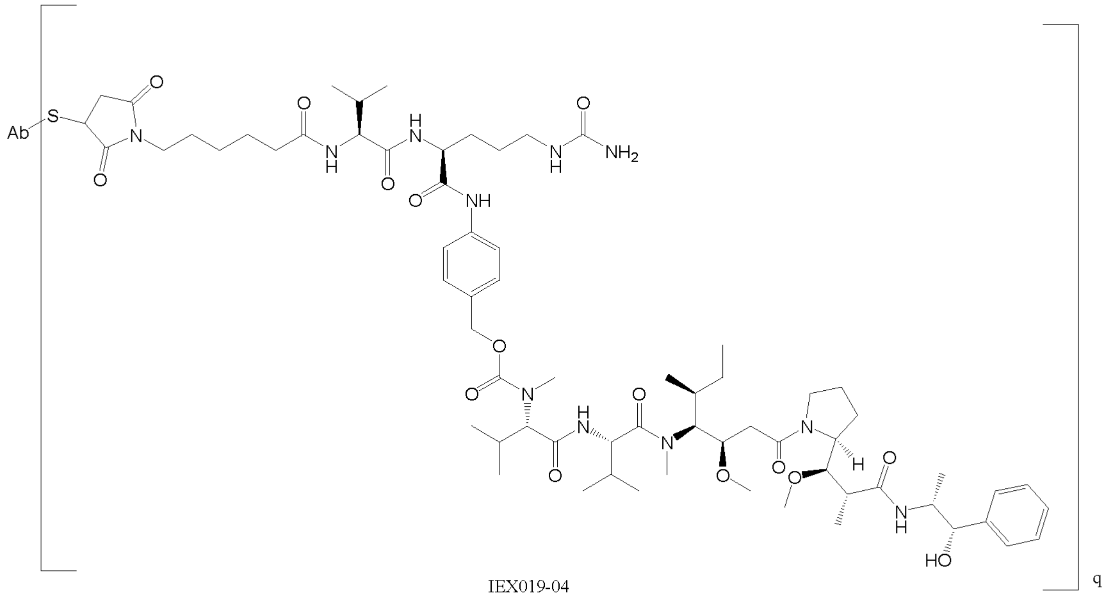

IEX019-04

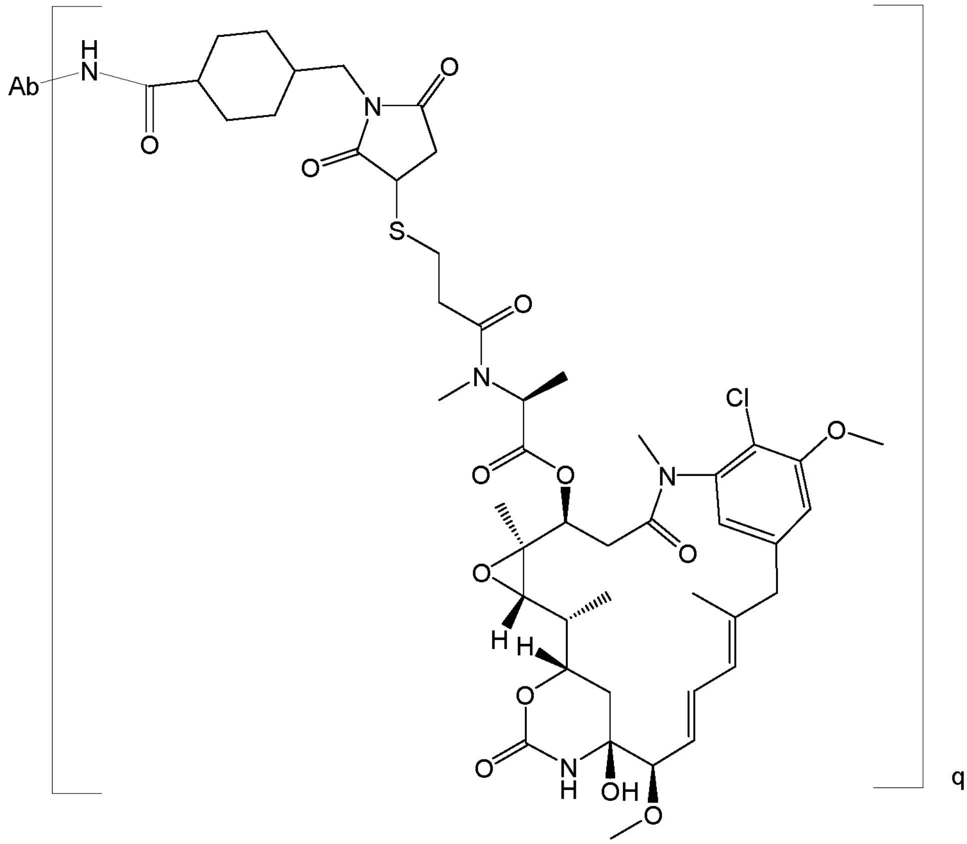

IEX019-05

wherein, Ab is HB37A6;

q represents an average DAR;
in IEX019-02, IEX019-04, and IEX019-05, q is 2-5, such as 3-5, 3-4, or 3.5-4.5;
in IEX019-03, q is 5-11, such as 6-10, 7-9, or 7.5-8.5.

**III. Preparation of the ADC molecule of the present invention**

In one aspect, the present invention provides a method for preparing an antibody with engineered glycosylation, which comprises

(1) preparation of a glycosylated antibody: culturing a host cell comprising a nucleic acid encoding the antibody (e.g., any one and/or more polypeptide chains) or an expression vector comprising the nucleic acid, as provided above, under conditions suitable for expressing antibodies, and optionally isolating the antibody from the host cell (or a host cell medium) to obtain an antibody comprising a core N-acetylglucosamine substituent (core-GlcNAc substituent), wherein the core N-acetylglucosamine and the core N-acetylglucosamine substituent are optionally fucosylated;

(2) preparation of a trimmed antibody: deglycosylating the antibody prepared in step (1) in the presence of an endoglycosidase to obtain an antibody comprising a core N-acetylglucosamine substituent, wherein the core N-acetylglucosamine and the core N-acetylglucosamine substituent are optionally fucosylated;

(3) allowing the trimmed antibody obtained in (2) to be in contact with a compound of formula E(A)x-P in the presence of a suitable catalyst to obtain an antibody comprising a GlcNAc-E(A)x substituent, the GlcNAc-E(A)x substituent being bonded to the antibody by C1 of the N-acetylglucosamine of the GlcNAc-E(A)x substituent, wherein the catalyst is a glycosyltransferase, and P is selected from uridine diphosphate (UDP), guanosine diphosphate (GDP), and cytidine diphosphate (CDP).

For carrying out step (1), a nucleic acid encoding the antibody (e.g., the antibody described above, e.g., any one and/or more polypeptide chains) is isolated and inserted into one or more vectors for further cloning and/or expressing in the host cells. Such nucleic acids can be easily isolated and sequenced by using conventional procedures (e.g., by using oligonucleotide probes that are capable of specifically binding to genes encoding heavy and light chains of antibodies).

The endoglycosidase in step (2) may be selected according to the properties of the glycosylated antibody, e.g., selected from Endo S, Endo A, Endo F, Endo M, Endo D, and Endo H enzymes and/or combinations thereof, and is also, e.g., Endo S, Endo S49, Endo F, or a combination thereof. In a preferred embodiment, the endoglycosidase is an endoglycosidase described in PCT/EP2017/052792, most preferably an Endo SH in PCT/EP2017/052792.

The glycosyltransferase in step (3) is preferably a P-(1,4)-N-acetylgalactosamine transferase or a glycosyltransferase derived therefrom, and is more preferably any β-(1,4)-GalNAcT enzyme described in PCT/EP2016/059194. In some embodiments, the β-(1,4)-GalNAcT enzyme is or is derived from an invertebrate β-(1,4)-GalNAcT enzyme. The β-(1,4)-GalNAcT enzyme may be, or may be derived from, any invertebrate β-(1,4)-GalNAcT enzyme known to those skilled in the art. Preferably, the β-(1,4)-GalNAcT enzyme is, or is derived from, a β-(1,4)-GalNAcT enzyme that originates from the phylum of Nematoda, preferably the class of Chromadorea or Secernentea, or from the phylum of Arthropoda, preferably the class of Insecta. More preferably, the β-(1,4)-GalNAcT enzyme is, or is derived from, a β-(1,4)-GalNAcT enzyme that originates from *Caenorhabditis elegans, Ascaris suum, Trichoplusia ni, Drosophila melanogaster, Caenorhabditis remanei,* Caenorhabditis briggsae, *Wuchereria bancrofti, Loa loa, Cerapachys biroi, Zootermopsis nevadensis, Camponotus florid anus. Crassostrea gigas,* or *Danaus plexippus.* Preferably, the glycosyltransferase suitable for use in step (3) is a *Trichoplusia ni* β-(1,4)-GalNAcT enzyme designated TnGalNAcT disclosed in PCT/EP2016/059194 (e.g., His-TnGalNacT).

In another aspect, the present invention provides a method for preparing an ADC by using the antibody of the present invention. The "ADC" in the present invention is defined as an antibody conjugated to an active substance (D) having biological and/or pharmaceutical activity via a linker (L). The method comprises allowing the antibody of the present invention to be conjugated to one or more active substance D via one or more linkers (L) defined herein. Preferably, the linker-active agent is site-specifically conjugated to the antibody.

In one embodiment, provided is a method for preparing an ADC of the present invention, which comprises allowing the glycosylated antibody of formula (III)

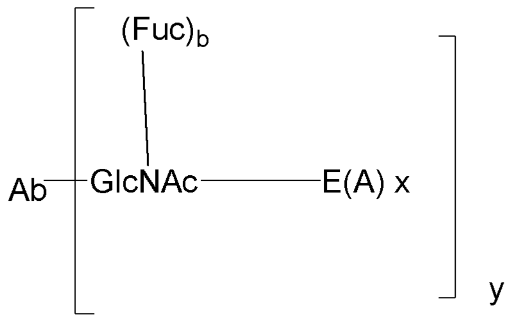

(formula III)

(wherein, the meaning of each variable or symbol is as defined above)

to react with a linker-payload containing an alkynyl group and one or more (e.g., 1, 2, 3, or 4) drug molecules to generate the antibody-drug conjugate of formula (II)

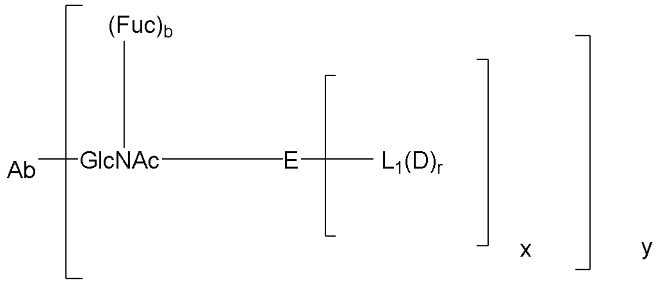

(formula II)

(wherein, the meaning of each variable or symbol is as defined above).

In one embodiment, the linker-payload has a structure of the following formula:

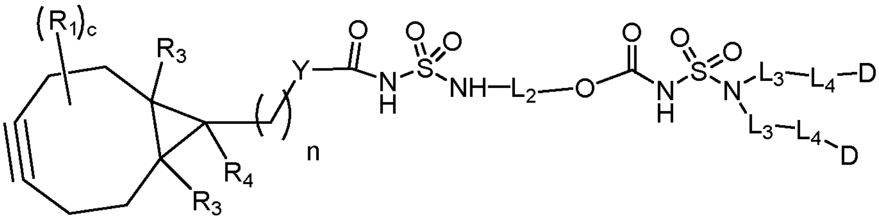

wherein, the variables are as defined above.

IV. Pharmaceutical composition

In some embodiments, the present invention provides a composition comprising any ADC molecule or the pharmaceutically acceptable salt thereof described herein, wherein preferably, the composition is a pharmaceutical composition or a pharmaceutical formulation. In one embodiment, the composition further comprises a pharmaceutical supplementary material. In one embodiment, the composition, e.g., the pharmaceutical composition, comprises the ADC molecule of the present invention and a combination of one or more additional therapeutic agents.

the present invention further comprises a composition (including a pharmaceutical composition) comprising the ADC molecule of the present invention or the pharmaceutically acceptable salt thereof. Such compositions can further comprise a suitable pharmaceutical supplementary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible.

For use and application of pharmaceutical supplementary materials, see Handbook of Pharmaceutical Excipients, 8th Ed., R. C. Rowe, P. J. Seskey and S. C. Owen, Pharmaceutical Press, London, Chicago.

The compositions of the present invention may be in a variety of forms. These forms include, for example, liquid, semi-solid and solid dosage forms, such as liquid solutions (e.g., injectable solutions and infusible solutions), pulvis or suspensions, liposomes, and suppositories. The preferred form depends on the intended mode of administration and therapeutic use.

The drug, preferably in the form of a lyophilized formulation or an aqueous solution, comprising the ADC described herein can be prepared by mixing the ADC molecule of a desired purity of the present invention with one or more optional pharmaceutical supplementary materials.

The pharmaceutical composition or formulation of the present invention may further comprise more than one active ingredient required by a particular indication treated, preferably those having complementarity activity without adversely affecting one another. For example, it may be desirable to also provide other therapeutic agents, including chemotherapeutic agents, angiogenesis inhibitors, cytokines, cytotoxic agents, other antibodies, small molecule drugs, immunomodulatory agents (e.g., immune checkpoint inhibitors or agonists), and the like. The active ingredients are suitably combined in an amount effective for an intended purpose.

A sustained release formulation can be prepared. Suitable examples of the sustained release formulation include a semipermeable matrix of a solid hydrophobic polymer containing an antibody. The matrix is in the form of a shaped article, such as a film or a microcapsule.

## VII. Pharmaceutical combination and kit

In some embodiments, the present invention further provides a pharmaceutical combination or a pharmaceutical combination product comprising the ADC molecule of the present invention, and one or more other therapeutic agents (e.g., therapeutic agents, including chemotherapeutic agents, angiogenesis inhibitors, cytokines, cytotoxic agents, other antibodies, small molecule drugs, immunomodulatory agents (e.g., immune checkpoint inhibitors or agonists), and the like).

Another object of the present invention is to provide a kit of parts comprising the pharmaceutical combination of the present invention; preferably, the kit is in the form of a pharmaceutical dose unit. Dose units may thus be provided according to the administration regimen or the interval between drug administrations.

In one embodiment, the kit of parts of the present invention comprises, in the same package,

- a first container containing a pharmaceutical composition comprising the ADC molecule of the present invention; and
- a second container containing a pharmaceutical composition comprising other therapeutic agents.

## VIII. Use and method

In one aspect, the present invention provides a method for preventing or treating a tumor (e.g., cancer) in a subject, which comprises administering to the subject an effective amount of the ADC molecule, the pharmaceutical composition, the pharmaceutical combination, or the kit of the present invention.

In some embodiments, the tumor (e.g., cancer) patient has CLDN18.2 (e.g., at an elevated level, e.g., at a nucleic acid or protein level). In some embodiments, tumor cells in the patient express CLDN18.2, e.g., moderately express CLDN18.2, preferably highly express CLDN18.2.

In some embodiments, the tumor, e.g., cancer, includes solid tumors and hematological tumors as well as metastatic lesions. In one embodiment, examples of the solid tumors include malignant tumors. The cancer may be at an early, intermediate, or advanced stage, or may be a metastatic cancer.

In one specific embodiment, the ADC molecule of the present invention is capable of killing tumor cells, and/or inhibiting tumor cell proliferation, such as tumor cells expressing CLDN18.2, e.g., gastrointestinal tumor cells, e.g., gastric cancer cells, pancreatic cancer cells, colon cancer cells, or colorectal cancer cells.

In some embodiments, the tumor is a tumor immune escape.

In some embodiments, the tumor is cancer, e.g., epithelial tumor, e.g., gastrointestinal tumor, e.g., epithelial cancer or gastrointestinal cancer, such as gastric cancer, gastroesophageal junction cancer, pancreatic cancer, colorectal cancer, or colon cancer.

The subject may be a mammal, e.g., a primate, preferably a higher primate, e.g., a human (e.g., an individual suffering from or at risk of suffering from the disease described herein). In one embodiment, the subject suffers from or is at risk of suffering from the disease described herein (e.g., cancer). In certain embodiments, the subject is receiving or has received other therapies, e.g., chemotherapy and/or radiotherapy. In some embodiments, the subject has previously received or is receiving immunotherapy.

In other aspects, the present invention provides use of the ADC molecule, the pharmaceutical composition, the pharmaceutical combination, or the kit in producing or preparing a drug for the use described herein, e.g., for use in preventing or treating a related disease or condition mentioned herein.

In some embodiments, the ADC molecule, the pharmaceutical composition, the pharmaceutical combination, or the kit of the present invention delays the onset of the condition and/or symptoms associated with the condition.

In some embodiments, the ADC molecule or the pharmaceutical composition of the present invention can also be administered in combination with one or more other therapies, e.g., therapeutic modalities and/or other therapeutic agents, for the use described herein, e.g., for use in preventing and/or treating a related disease or condition mentioned herein.

In some embodiments, the therapeutic modality includes surgery, radiotherapy, partial irradiation, focused irradiation, or the like.

In some embodiments, the therapeutic agent is selected from chemotherapeutic agents, angiogenesis inhibitors, cytokines, cytotoxic agents, other antibodies, small molecule drugs, or immunomodulatory agents (e.g., immune checkpoint inhibitors or agonists).

Examples of other antibodies include antibodies that specifically bind to immune checkpoints.

The combination therapy of the present invention encompasses both co-administration (e.g., two or more therapeutic agents are contained in the same formulation or separate formulations) and separate administrations in which the ADC molecule of the present invention can be administered prior to, concurrently with, and/or after the administration of other therapeutic agents and/or pharmaceuticals.

The route of administration of the pharmaceutical composition is based on a known method, for example, oral, intravenous, intraperitoneal, intracerebral (intraparenchymal), intraventricular, intramuscular, intraocular, intraarterial, intraportal or intralesional route, via sustained release systems or via implanted devices. In certain embodiments, the composition can be administered by bolus injection or by continuous infusion or by an implanted device.

The composition can also be administered topically via an implanted membrane, sponge, or another suitable material that absorbs or encapsulates the desired molecule. In certain embodiments, when an implanted device is used, the device can be implanted into any suitable tissue or organ, and the desired molecule can be delivered via diffusion, time-released bolus, or continuous administration.

These and other aspects and embodiments of the present invention are illustrated in the drawings (brief description of the drawings follows) and in the following detailed description of the present invention and are described in the following examples. Any or all of the features described above and throughout the present invention may be combined in various embodiments of the present invention. The following examples further illustrate the present invention. However, it should be understood that the examples are described by way of illustration rather than limitation, and various modifications may be made by those skilled in the art.

## Examples

### Example 1.1: Construction of Stably Expressing Cell Lines

Preparation of cell lines overexpressing human CLDN18.2

According to the manufacturer's instructions, the Freedom® CHO-S® kit (Invitrogen, A1369601) was used to construct cell lines stably expressing human Claudin18.2 (abbreviated as CLDN18.2, the same applies hereinafter). Firstly, the full-length gene of human CLDN18.2 (UniProt ID: P56856-2) was introduced into a vector pCHO1.0 to construct a plasmid, which was transfected into CHO-S cells (Invitrogen, A1369601) and HEK293 cells (Invitrogen, A14527) separately by chemical transfection and electrotransfection, and the transfected cells were subjected to two rounds of pressurized screening to obtain cell pools expressing CLDN18.2. Then, the cells highly expressing CLDN18.2 were sorted out by using a flow cytometric sorter (MoFlo XDP, Beckman Coulter), and the monoclonal cell lines CHO-hCLDN18.2 and HEK293-hCLDN18.2 that stably express CLDN18.2 were obtained by dilution.

Preparation of cell line overexpressing human CLDN18.1

According to the manufacturer's instructions, the Freedom® CHO-S® kit (Invitrogen, A1369601) was used to construct cell lines stably expressing human Claudin18.1 (abbreviated as CLDN18.1, the same applies hereinafter). Firstly, the full-length gene of human CLDN18.1 (UniProt ID: P56856-1) was introduced into a vector pCHO1.0 (Invitrogen, A1369601) to construct a plasmid, which was transfected into CHO-S cells (Invitrogen, A1369601) by chemical transfection, and the transfected cells were subjected to two rounds of pressurized screening to obtain the cell pool expressing CLDN18.1. Then, the cells highly expressing CLDN18.1 were sorted out by using a flow cytometric sorter (MoFlo XDP, Beckman Coulter), and the monoclonal cell line CHO-hCLDN18.1 stably expressing CLDN18.1 was obtained by dilution.

Construction of tumor cell lines overexpressing CLDN18.2

The full-length gene of human CLDN18.2 (UniProt ID: P56856-2) was introduced into a vector pWPT-GFP (Addgene, 12255) to replace the GFP sequence therein, and the construct was transfected, together with the packaging vectors psPAX2 (Addgene, 12260) and pMD2.G (Addgene, 12259) of the lentivirus, into HEK293T (ATCC, CRL-3216) cells for virus packaging. The culture supernatants cultured for 48 h and 72 h were each collected, and the lentivirus was concentrated using PEG8000. The pancreatic cancer DAN-G cells (CLS Cell Lines Service GmbH, 300162) and gastric cancer KATO III cells (ATCC, HTB-103) were transfected with the concentrated virus, and then the cells expressing CLDN18.2 were sorted out by using a flow cytometric sorter (MoFlo XDP, Beckman Coulter) to obtain the tumor cell lines DAN-G-hCLDN18.2 and KATO III-hCLDN18.2 which were stably transfected with CLDN18.2.

**Example 1.2: Generation of CLDN18.2 Monoclone**

In the present invention, H2L2 fully human antibody transgenic mice (purchased from Harbour BioMed) were immunized with the cells obtained in Example 1 (CHO-hCLDN18.2) by using the hybridoma technology. Spleen cells from the mice were then harvested and electrofused with myeloma cells. Then, the supernatant was collected, hybridoma cells specifically expressing the anti-CLDN18.2 antibody were selected by a flow cytometer (FACS), and the secreted antibody did not bind to CLDN18.1. The test cells (HEK293-hCLDN18.2) obtained in Example 1 were counted, diluted to $1 \times 10^6$ cells/mL, and then added to a U-bottom 96-well plate at 100 μL/well. The cells were centrifuged at 500 g for 5 min, and the cell medium was removed. The hybridoma culture supernatant in a 96-well plate was added to the U-bottom plate at 100 μL/well, the cells were resuspended, and the suspension was left to stand on ice for 30 min. The suspension was centrifuged at 500 g for 5 min, the supernatant was removed, and the cells were washed once with PBS. 100 μL of FITC-labeled anti-mouse Fab secondary antibody (1:500 dilution in PBS) was added to each well, and 100 μL of FITC-labeled anti-human Fab secondary antibody was added to the positive control antibody. The resulting mixture was incubated on ice in the dark for 30 min. The mixture was centrifuged at 500 g for 5 min, the supernatant was removed, and the cells were washed once with PBS. The cells were resuspended in 50 μL of 1× PBS, and the suspension was loaded for FACS assay. The positive clones were rescreened for CHO-hCLDN18.1 in the same manner as described above. After two rounds of screening, the fully human antibody clone HB37A6 was obtained.

**Example 1.3: Preparation of Recombinant CLDN18.2 Monoclonal Antibody**

The anti-CLDN18.2 monoclonal antibody HB37A6 (see CN202010570517.X) and the control antibody zolbetuximab (abbreviated as Zmab, sequence derived from INN117) were expressed as full-length monoclonal antibodies in HEK293 cells (Invitrogen, A14527).

Expression vectors were first constructed. The heavy chain variable region and light chain variable region of HB37A6 and those of the control antibody (see sequence listing) were separately placed at the N-terminus of the human IgG1 heavy chain constant region (SEQ ID NO: 5) and the light chain kappa constant region (SEQ ID NO: 10). Then, the obtained sequences were introduced into pcDNA3.1 expression vectors with N-terminal signal peptide to obtain the light and heavy chain expression vectors. The obtained light and heavy chain expression vectors were co-transfected into HEK293 cells by PEI (Polysciences Inc, 23966), and the medium supernatant was collected after 7 days of culturing. The supernatant was purified by a Protein A column (Hitrap Mabselect Sure, GE 11-0034-95), followed by ultrafiltration and buffer-exchange into PBS (Gibco, 70011-044). The concentration was determined by the A280 method and the purity was determined by the SEC-HPLC method to obtain an antibody solution with a purity of greater than 95%, thus obtaining the recombinant CLDN18.2 monoclonal antibody HB37A6.

The specific transfection and purification procedures were as follows:
Expi293 cells (Invitrogen, A14527) were passaged according to a desired transfection volume.

The cell density was adjusted to $1.5 \times 10^6$ cells/mL the day before transfection. The cell density on the day of transfection was approximately $3 \times 10^6$ cells/mL. The Opti-MEM culture medium (Gibco, 31985-070) at a volume 1/10 of the final volume was used as the transfection buffer, the plasmid was added at 1.0 μg/mL transfected cells, and the mixture was well mixed. An appropriate amount of polyethylenimine (PEI) (Polysciences, 23966) was added to the plasmid (the ratio of plasmid to PEI was 1:3 in the 293F cells), and the mixture was well mixed and incubated at room temperature for 20 min, thus obtaining a DNA/PEI mixture. The DNA/PEI mixture was added slowly to the cells while gently shaking the shake flask, and then the resulting mixture was cultured in an incubator at 36.5 °C and 8% $CO_2$; after culturing for seven days, a cell suspension was obtained, and the cell supernatant was collected for purification.

The Protein A column (Hitrap Mabselect Sure, GE, 11-0034-95) used for purification was treated with 0.1 M NaOH for 2 h, and the glass bottles or the like were washed with distilled water and then dried at 180 °C for 4 h. The collected cell suspension was centrifuged at 4500 rpm for 30 min before purification, and the supernatant was filtered using a 0.22 μm filter. The Protein A column was equilibrated with 10 column volumes of binding buffer (20 mM sodium

phosphate, 150 mM NaCl, pH 7.0). The filtered supernatant was loaded to the purification column, which was then equilibrated with 10 column volumes of binding buffer. 5 mL of elution buffer (0.1 M citric acid + sodium citrate, pH 3.5) was added. The eluate was collected, and 80 μL of 2 M Tris-HCl was added per mL of eluate. The collected antibody was buffer-exchanged into PBS (Gibco, 70011-044) by ultrafiltration concentration, and the concentration was measured.

**Example 1.4: Determination of Affinity of CLDN18.2 Antibody by SPR**

The equilibrium dissociation constant ($K_D$) for binding of HB37A6 to human CLDN18.2 was determined by surface plasmon resonance (SPR). According to the manufacturer's instructions, human Claudin18.2 (GenScrip, P50251802) was coupled to the surface of a CM5 chip (GE Healthcare, 29-1496-03) using the amino coupling kit (GE Healthcare, BR-1006-33), and the remaining activation sites were blocked by injection of 1 M ethanolamine after coupling. According to the manufacturer's instructions, the binding and dissociation between the antigen on the chip surface and antibody in the mobile phase were detected by Biacore (GE Healthcare, T200) to obtain affinity and kinetic constants. The antibodies (0-100 nM) obtained by gradient dilution flowed over the chip surface in an order from low concentration to high concentration, with the binding time of 180 s and the dissociation time of 600 s. Finally, the chip was regenerated using 10 mM Glycine pH 1.5 (GE Healthcare, BR-1003-54). Kinetic analysis of the data results was performed using Biacore T200 analysis software with a 1:1 binding model. As shown in Table 1, the affinity of HB37A6 was better than that of the control antibody Zmab.

Table 1. Determination of affinity constants (equilibrium dissociation constants) of CLDN18.2 antibodies by SPR

| Antibody | ka (1/Ms) | kd (1/s) | KD (M) |
|---|---|---|---|
| Zmab | 3.482E+5 | 7.894E-4 | 2.267E-9 |
| HB37A6 | 4.252E+5 | 3.063E-4 | 7.203E-10 |

Example 1.5: Binding Specificity of CLDN18.2 Antibodies for CLDN18 Cells

The binding of the anti-CLDN18.2 monoclonal antibody HB37A6 described above and the control antibody Zmab to the CHO-S cell lines stably transfected with human CLDN18.2 and human CLDN18.1 (i.e., CHO-hCLDN18.2 and CHO-hCLDN18.1 prepared as described in Example 1) obtained in Example 1 was determined by flow cytometry (FACS).

Specifically, the test cells (CHO-hCLDN18.2 and CHO-hCLDN18.1) obtained in Example 1 were counted, diluted to $1 \times 10^6$ cells/mL, and then added to a U-bottom 96-well plate at 100 μL/well. The cells were centrifuged at 500 g for 5 min, and the cell medium was removed. The anti-CLDN18.2 monoclonal antibody HB37A6 and the control antibody Zmab were each added to the U-bottom plate at 100 μL/well, and the cells were resuspended; the initial concentration of the antibody was 900 nM, and then 3-fold dilution was performed to obtain a total of 10 concentration points. The suspension was left to stand on ice for 30 min. The suspension was centrifuged at 500 g for 5 min, the supernatant was removed, and the cells were washed once with PBS. The goat anti-human Fc PE-labeled secondary antibody (SouthernBiotech, J2815-5H87B) was added at 100 μL/well. The resulting mixture was incubated on ice in the dark for 30 min. The mixture was centrifuged at 500 g for 5 min, the supernatant was removed, and the cells were washed once with PBS. The cells were resuspended in 50 μL of 1× PBS, and the suspension was loaded for FACS assay. The experimental data were analyzed using GraphPad Prism software, and FIG. 1 and FIG. 2 were obtained. As shown in FIG. 1 and FIG. 2, all the antibodies specifically bound to human CLDN18.2 (FIG. 1) but did not bind to human CLDN18.1 (FIG. 2).

**Example 1.6: Binding of CLDN18.2 Antibodies to Tumor Cell Lines**

Referring to Example 1.5, the binding of HB37A6 to the gastric cancer cell line NUGC-4 (JCRB, JCRB0834), the gastric cancer cell line KATO III-hCLDN18.2, and the pancreatic cancer cell line DAN-G-hCLDN18.2 was determined by FACS. FIG. 3 shows that the human antibody HB37A6 has good tumor cell specific binding, which was better than that of the control antibody Zmab.

Example 1.7: *In Vivo* Anti-Tumor Effect of CLDN18.2 Antibodies

1. Activity of antibodies on the DAN-G-CLDN18.2 tumor-bearing mouse model

The HB37A6 antibody was used to test its anti-tumor effect in NOD-SCID mice (female NOD-SCID mice (15-18

g) purchased from Beijing Vital River Laboratory Animal Technology Co., Ltd.) with human pancreatic cancer. The human pancreatic cancer cells DAN-G-hCLDN18.2 constructed in Example 1 were subcultured conventionally for subsequent *in vivo* experiments. The cells were collected by centrifugation, and the DAN-G-hCLDN18.2 cells were dispersed with PBS (1×) to obtain a suspension with a cell density of 12 × $10^5$ cells/mL. The cell suspension was mixed with matrigel gel at 1:1 to prepare a cell suspension with a cell concentration of 6 × $10^5$ cells/mL. On day 0, 0.2 mL of the cell suspension was subcutaneously inoculated into the right abdominal region of each of the NOD-SCID mice to establish DAN-G-CLDN18.2 tumor-bearing mouse models.

The tumor volume of each mouse was measured 5 days after tumor cell inoculation, and the mice with the tumor volume in the range of 43.36 $mm^3$ to 89.47 $mm^3$ were selected and divided into groups in a serpentine manner according to the size of the tumor volume (8 mice in each group). The hIgG (Equitech-Bio, batch No. 160308-02), HB37A6, and the control antibody Zmab were administered, at a dose of 10 mg/kg each time, to each mouse on days 5, 9, 12, and 16 after inoculation, and the tumor volume of the mice was monitored 2-3 times per week. Tumor volume measurement: The maximum length of the major axis (L) and the maximum length of the minor axis (W) of tumors were measured with a vernier caliper, and tumor volume was calculated by the following formula: $V = L \times W^2/2$. The mice were weighed using an electronic balance.

2. Activity of antibody on the NUCG-4 tumor-bearing mouse model

The HB37A6 antibody was selected to test its anti-tumor effect in NOG mice (female NOG mice (15-18 g) purchased from Beijing Vital River Laboratory Animal Technology Co., Ltd.) with human gastric cancer. The PBMC cells (Allcells) were thawed and centrifuged to collect cells. The PBMC cells were dispersed with PBS (1×) to obtain a cell suspension with a cell density of 2.5 × $10^6$ cells/mL. On day 0, 0.2 mL of the cell suspension was injected into each of the NOG mice via the ophthalmic vein to establish the NOG humanized mouse models.

The NUGC-4 cells were thawed and subcultured conventionally for subsequent *in vivo* experiments. The cells were collected by centrifugation, and the NUGC-4 cells were dispersed with PBS (1×) to obtain a suspension with a cell density of 12 × $10^6$/mL. The suspension was mixed with matrigel at 1:1 to prepare a cell suspension with a cell concentration of 6 × $10^6$/mL. On day 5, 0.2 mL of the cell suspension was subcutaneously inoculated into the right abdominal region of each of the NOG humanized mice to establish the NUCG-4 tumor-bearing mouse models.

The mice were randomly divided into groups on day 1 after inoculation with tumor cells (7 mice per group). hIgG (Equitech-Bio, batch No. 160308-02, control group), HB37A6 (treatment group), and the positive control antibody Zmab (treatment group) were administered, at a dose of 10 mg/kg each time, to each mouse on days 1, 5, 8, and 12 after inoculation, and the tumor volume and body weight of the mice were monitored 2-3 times per week. Tumor volume measurement: The maximum length of the major axis (L) and the maximum length of the minor axis (W) of tumors were measured with a vernier caliper, and tumor volume was calculated by the following formula: $V = L \times W^2/2$. The mice were weighed using an electronic balance. The relative tumor growth inhibition (TGI%) was calculated on day 26 after inoculation, and the calculation formula is as follows:

TGI% = 100% × (tumor volume of the control group - tumor volume of the treatment group)/(tumor volume of the control group - tumor volume of the control group before administration).

3. Results

The results are shown in FIG. 4. Both HB37A6 and the control antibody Zmab were capable of inhibiting tumor growth in the human pancreatic cancer DAN-G-CLDN18.2 mouse models, with the TGI values of the two being 28% and 24%, respectively. As shown in FIG. 5, HB37A6 showed better anti-tumor effect than the control antibody Zmab in human gastric cancer NUGC-4 mouse models, with the TGI values of the two being 31% and 0%, respectively.

**Example 2.1: Synthesis of IEX019 ADC Molecules**

**IEX019**

Based on HB37A6, ADCs featuring conjugation to different small molecule compounds were further designed and synthesized. The specific procedure is as follows:

**Example 2.1.1: Preparation of IEX019-02**

**1) Preparation of compound 6**

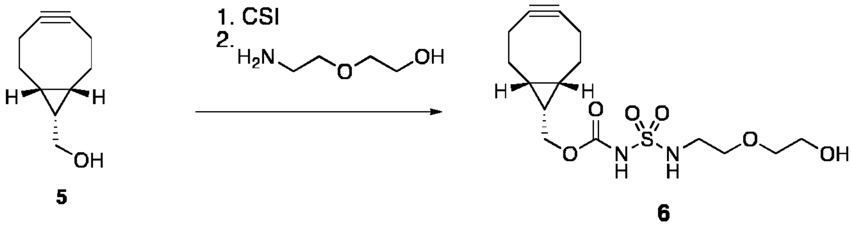


5

6

In $N_2$ atmosphere, chlorosulfonyl isocyanate (CSI) (0.87 mL, 1.4 g, 10 mmol), $Et_3N$ (2.8 mL, 2.0 g, 20 mmol), and 2-(2-aminoethoxy)ethanol (1.2 mL, 1.26 g, 12 mmol) were added to a solution of BCN-OH (5, 1.5 g, 10 mmol) in DCM (150 mL). The mixture was stirred for 10 min, and then aqueous $NH_4Cl$ solution (saturated, 150 mL) was added to quench the reaction. After separation, the aqueous layer was extracted with DCM (150 mL). The combined organic layers were dried ($Na_2SO_4$) and concentrated. The residue was purified by column chromatography to give product 6 (2.06 g, 5.72 mmol, 57%) in the form of a pale yellow thick oil. $^1H$ NMR (400 MHz, $CDCl_3$) δ (ppm) 6.0 (bs, 1H), 4.28 (d, J = 8.2 Hz, 2H), 3.78-3.73 (m, 2H), 3.66-3.61 (m, 2H), 3.61-3.55 (m, 2H), 3.34 (t, J = 4.9 Hz, 2H), 2.37-2.15 (m, 6H), 1.64-1.48 (m, 2H), 1.40 (quintet, J = 8.7 Hz, 1H), 1.05-0.92 (m, 2H).

2) Preparation of compound 7

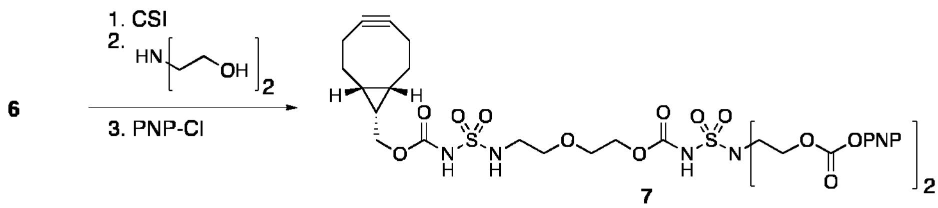


6

7

CSI (11 μL, 18 mg, 0.13 mmol) was added to a stirred solution of 6 (47 mg, 0.13 mmol) in DCM (10 mL). 30 min later, a solution of $Et_3N$ (91 μL, 66 mg, 0.65 mmol) and diethanolamine (16 mg, 0.16 mmol) in DMF (0.5 mL) was added. 30 min later, p-nitrophenyl chloroformate (52 mg, 0.26 mmol) and $Et_3N$ (54 μL, 39 mg, 0.39 mmol) were added. 4.5 h later, the reaction mixture was concentrated, and the residue was purified by gradient column chromatography (33 → 66% EtOAc/heptane (1% AcOH)) to give 7 (88 mg, 0.098 mmol, 75%) in the form of a colorless oil. $^1H$ NMR (400 MHz, $CDCl_3$) δ (ppm) 8.28-8.23 (m, 4H), 7.42-7.35 (m, 4H), 4.52 (t, J = 5.4 Hz, 4H), 4.30 (d, J = 8.3 Hz, 2H), 4.27-4.22 (m, 2H), 3.86 (t, J = 5.3 Hz, 4H), 3.69-3.65 (m, 2H), 3.64-3.59 (m, 2H), 3.30-3.22 (m, 2H), 2.34-2.14 (m, 6H), 1.62-1.46 (m, 2H), 1.38 (quintet, J = 8.7 Hz, 1H), 1.04-0.92 (m, 2H).

**3) Preparation of compound 9**

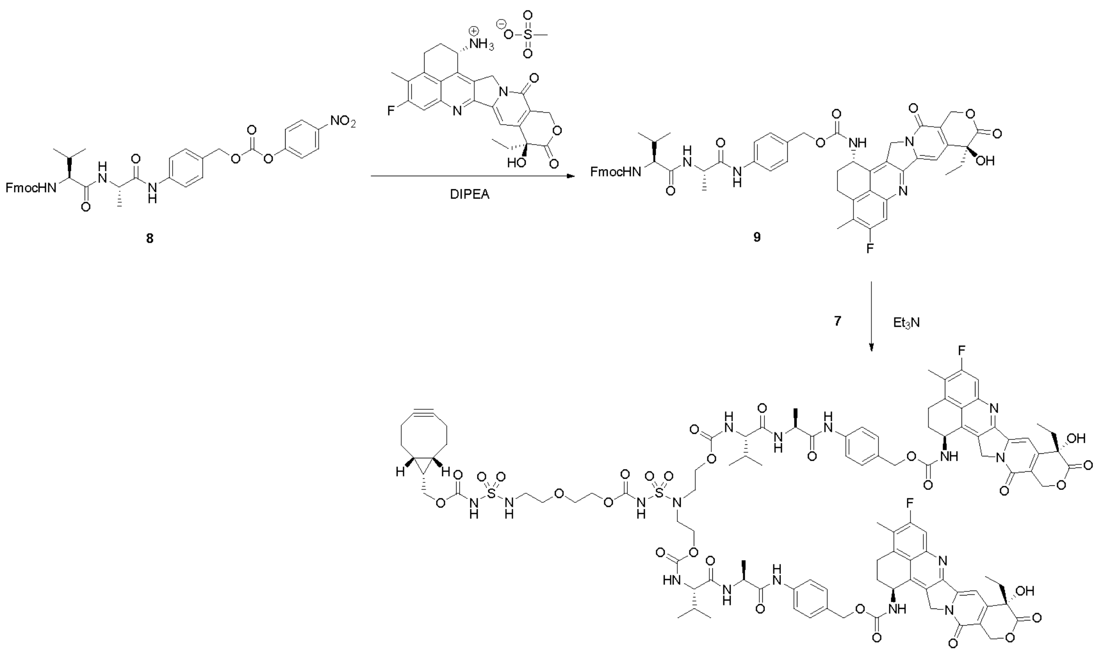


8

9

1

Compound **8** (163 mg, 240 μmol) was added to a mixture of exatecan mesylate (125 mg, 235 μmol) and DIPEA (61 mg, 82 μL, 0.47 mmol) in dry DMF (0.9 mL). 20 h later, the reaction mixture was diluted with 9 mL of DCM and purified by gradient column chromatography (0 → 40% MeOH/DCM) to give 9 (155 mg, 159 μmol, 68%). LCMS (ESI+) $C_{55}H_{54}FN_6O_{10}^+$ $(M+H)^+$ calculated: 977.39; found: 977.80.

**4) Preparation of compound 1**

A solution of $Et_3N$ (73 mg, 101 μL, 0.72 mmol) and compound 7 (65 mg, 72 μmol) in DMF (1.4 mL) was added to a solution of compound 9 (155 mg, 159 μmol) in DMF (1.6 mL). The reaction mixture was stirred for 24 h, diluted with DCM (20 mL), and purified by gradient column chromatography (0 → 40% MeOH/DCM) to give compound 1 (94 mg, 44 μmol, 28%) in the form of a pale yellow solid. LCMS (ESI+) $C_{102}H_{118}F_2N_{16}O_{29}S_2^{2+}$ $(M/2+H)^+$ calculated: 1066.88; found: 1067.12.

**5)** Enzymatic reconstitution of HB37A6 into HB37A6-(GlcNAc(Fuc)$_1$-6-$N_3$-GalNAc)$_2$ HB37A6 was expressed and purified in HEK293 cells according to Example 1.3. The obtained HB37A6 (16.4 mg/mL) was incubated with EndoSH (1% w/w) as described in PCT/EP2017/052792, thus obtaining a trimmed HB37A6 having -GlcNAc or GlcNAc(Fuc) at position Asn297. The trimmed HB37A6 was incubated with the enzyme His-TnGalNAcT disclosed in PCT/EP2016/059194 (4.5% w/w) and the 6-azido-GalNAc-UDP disclosed in PCT/EP2016/059194 (25 eq (equivalents) as compared with the antibody) (in a histidine (20 mM) + NaCl (150 mM) solution containing 6 mM $MnCl_2$) at 30 °C for 16 h.

Then, the incubation mixture obtained above was purified using a 50 mL protA column (Hitrap Mabselect Sure, GE, 11-0034-95). The incubation mixture obtained above was loaded to the column, which was washed with TBS + 0.2% Triton and TBS. The column was then eluted with 0.1 M acetate buffer (pH 2.9) and neutralized with 2.5 M Tris-HCl (pH 7.2). After three times of dialysis with PBS, the obtained (modified) glycosylated antibody was concentrated to 32.6 mg/mL using a Vivaspin Turbo 15 ultrafiltration device (Sartorius).

The obtained glycosylated antibody was analyzed by mass spectrometry, and the main steps are as follows: prior to mass spectrometry, the glycosylated antibody was treated with IdeS, so that the Fc/2 fragment could be analyzed. A solution (a total volume of 10 μL) of 20 μg of (modified) glycosylated antibody solution and IdeS (Fabricator™) (1.25 U/μL) in PBS (pH 6.6) was incubated for 1 h at 37 °C. The sample was diluted to 80 μL and then analyzed on JEOL AccuTOF (ESI-TOF), and deconvolution spectra were obtained using the Magtran software. Mass spectrometry analysis of the IdeS-digested sample showed that the major product corresponded to HB37A6-(GlcNAc(Fuc)$_1$-6-$N_3$-GalNAc)$_2$ obtained, with an observed mass of 24330.4.

Thus, the above results demonstrate that HB37A6-(GlcNAc(Fuc)$_1$-6-$N_3$-GalNAc)$_2$ in which GlcNAc at Asn297 of both heavy chains was substituted with 6-azido-GalNAc (substitution at position 4) was obtained.

**6) Preparation of HB37A6-SYNtecan E conjugate (IEX019-02)**

The bioconjugate IEX019-02 according to the present invention was prepared by conjugating compound 1 (linker-payload) as the linker-conjugate to azide-modified HB37A6-(GlcNAc(Fuc)$_1$-6-N$_3$-GalNAc)$_2$ as the biomolecule. Thus, PBS (pH 7.4, 808 μL), 1,2-propanediol (11.3 mL), and compound 1 (350 μL, 40 mM DMF solution) were added to an HB37A6-(GlcNAc(Fuc)$_1$-6-N$_3$-GalNAc)$_2$ solution (10.8 mL, 350 mg, 32.6 mg/mL, PBS pH 7.4). The reaction mixture was incubated overnight at room temperature and then filtered and dialyzed against PBS (pH 7.4). The remaining free payload was removed by adding charcoal (350 mg) and then spinning overnight at room temperature. Charcoal was removed by centrifugation and filtration, and ADC was purified on AKTA Purifier-10 (GE Healthcare) with a Superdex200 26/600 SEC (GE Healthcare) column.

Mass spectrometry analysis of IdeS-digested sample showed that both major products corresponded to the ADC obtained, i.e., HB37A6-SYNtecan E conjugate. First peak: the observed mass was 26469 Da (calculated mass was 26465 Da), corresponding to the conjugated Fc/2 fragment (2× closed lactone form of payload). Second peak: the observed mass was 26499 Da (calculated mass was 26501 Da), corresponding to the bound Fc/2 fragment (2× open carboxylate form of payload).

The resulting structure is as follows. The concentration of ADC product, DAR value, and SEC purity were determined by UV, SEC, RP-HPLC, and LC-MS. The purity of the monomer measured by SE-HPLC was >99% and the concentration was 6.12 mg/mL.

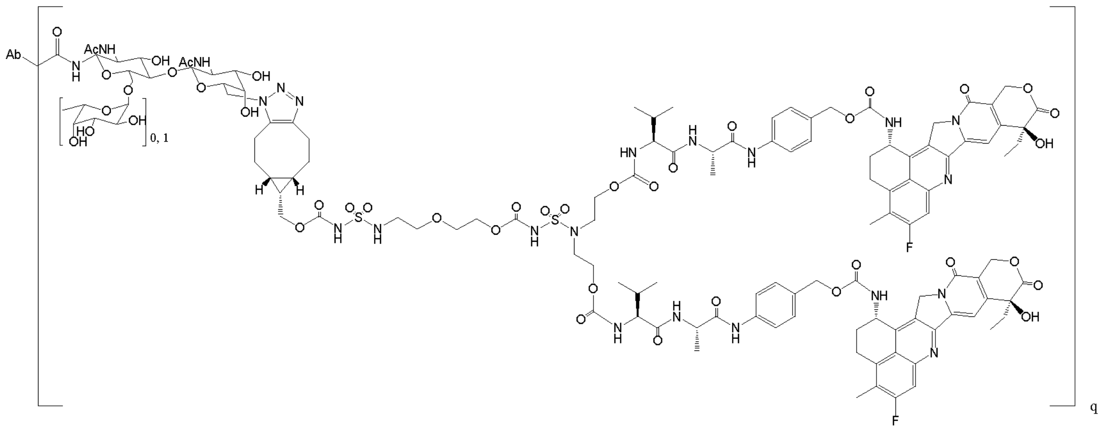

In the structure, q represents an average DAR of, e.g., 3-5, 3.2-4.8, or 3.5-4.5, such as 3.52 as determined in Table 2;
Ab is HB37A6.

Example 2.1.2: Preparation of IEX019-03

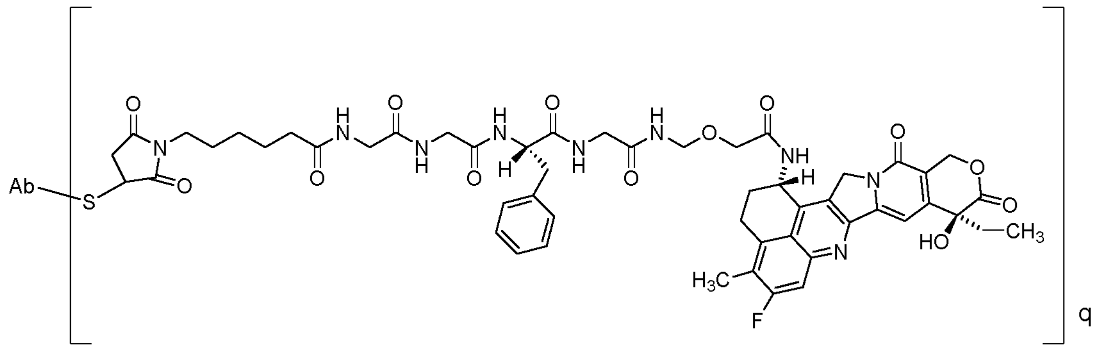

IEX019-03

In the structure, q represents an average DAR of, e.g., 5-11, 6-10, 7-9, or 7.5-8.5, such as 7.9 as determined

in Table 2;
Ab is HB37A6.

(a) A reducing agent solution (TCEP (Sigma, C4706), dissolved in water) was added to an HB37A6 solution (dissolving antibody HB37A6 in PBS buffer (Thermo, 10010023)), and the reaction mixture was shaken in a shaker for 2-4 h, wherein

(i) the optimal concentration of HB37A6 was 5-10 mg/mL,
(ii) the optimal molar ratio of TCEP/mAb was 4.5-6.5,
(iii) the optimal temperature for the reaction was 20-37 °C, and
(iv) the optimal pH for the reaction was generally between 6.5 and 8.0.

(b) An excess of linker-toxin MC-GGFG-DXD (purchased from Levena biopharma, SET0218, structure shown below) was dissolved in DMSO, and the solution was allowed to react with the thiol group of the antibody reduced in step (a). The reaction mixture was shaken in a shaker for 1-2 h, wherein

(i) the optimal molar ratio of DXD/mAb was 10.0-12.0, and
(ii) the optimal temperature for the reaction was 20-37 °C,
thus obtaining the ADC crude product.

(c) The obtained ADC crude product was purified by spin desalting, ultrafiltration, or dialysis to obtain the final ADC product IEX019-03.
(d) The ADC product was detected by HIC, LC-MS, and SEC HPLC to determine the average DAR and SEC purity.

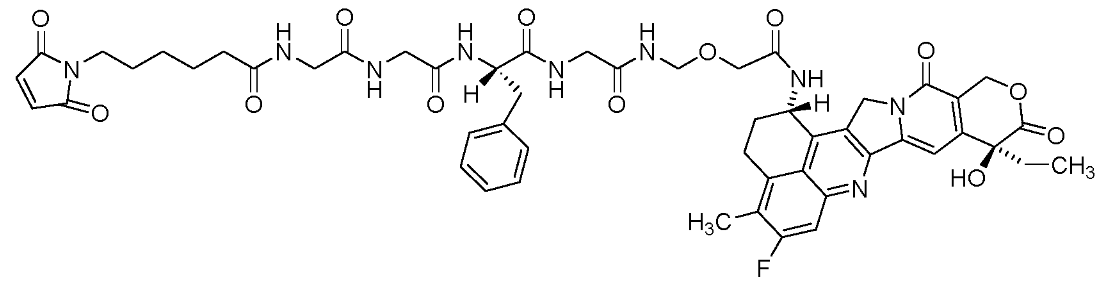

MC-GGFG-DXD

**Example 2.1.3:** Preparation of IEX019-04

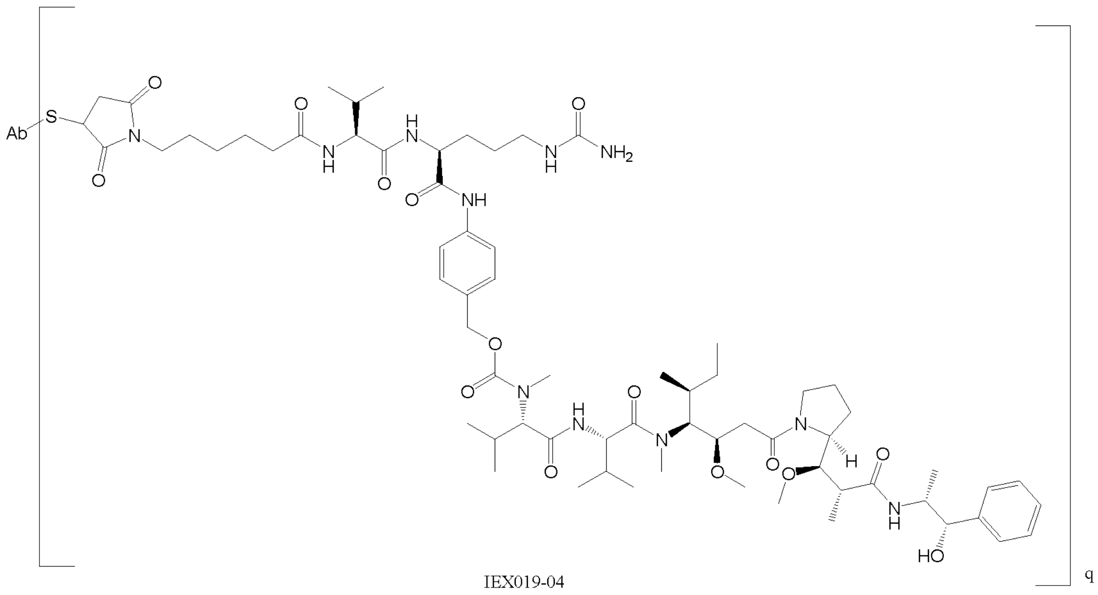

In the structure, q represents an average DAR of, e.g., 3-5, 3.2-4.8, or 3.0-4.0, such as 3.5 as determined in Table 2;
Ab is HB37A6.

This molecule was prepared as follows.

(a) A reducing agent solution (TCEP (Sigma, C4706), dissolved in water) was added to an antibody HB37A6 solution (dissolving antibody HB37A6 in PB buffer), and after the addition was completed, the reaction mixture was shaken in a shaker for 2-4 h, wherein

(i) the optimal concentration of antibody HB37A6 was 5-10 mg/mL,
(ii) the optimal molar ratio of TCEP/mAb was 1.9-2.7,
(iii) the optimum temperature for the reduction reaction was 20-37 °C, and
(iv) the optimal pH for the reaction was generally between 6.5 and 8.0.

(b) An excess of linker-payload MC-VC-PAB-MMAE (purchased from Levena Biopharma, SET0201) with a reactive group (maleimide linked drug) was dissolved in the organic solvent DMSO, and the solution was allowed to react with the reducing thiol group generated in step (a). The reaction mixture was shaken in a shaker for 1-2 h., wherein

(i) the optimal molar ratio of MC-VC-PAB-MMAE/mAb was 8.0-10.0, and
(ii) the optimal temperature for the binding reaction was 20-37 °C.

(c) After the binding reaction was completed, an acetylcysteine solution was added to terminate the reaction of step (b). The mixture was incubated at 20-25 °C for 5-15 min.
(d) The obtained ADC crude product was purified by spin desalting, ultrafiltration, or dialysis to obtain the final ADC product IEX019-04.
(e) The ADC product was detected by HIC and SEC- HPLC to determine the average DAR and SEC purity.

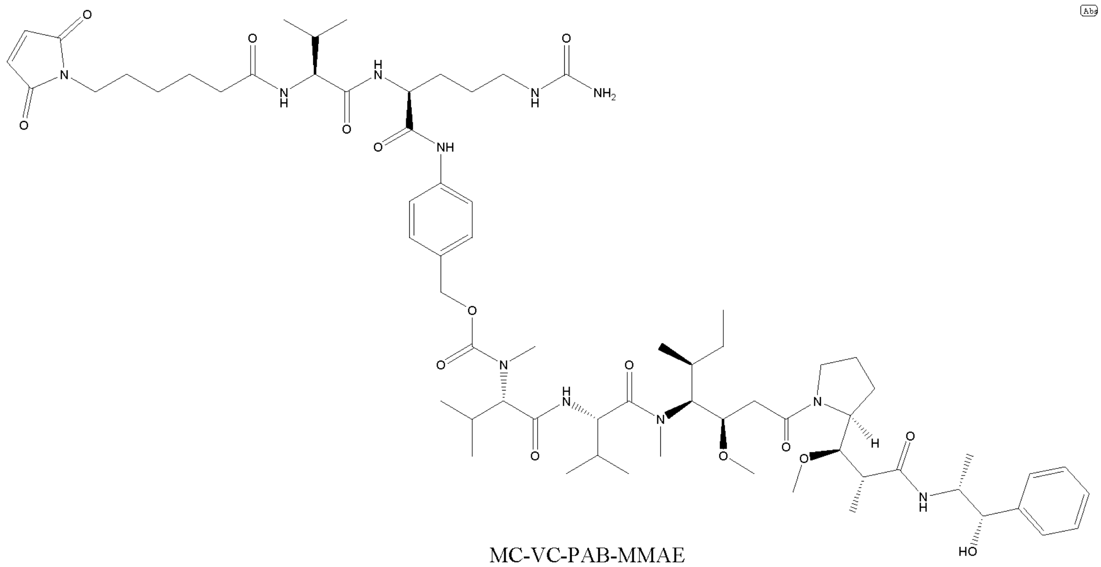

MC-VC-PAB-MMAE

**Example 2.1.4: Preparation of IEX019-05**

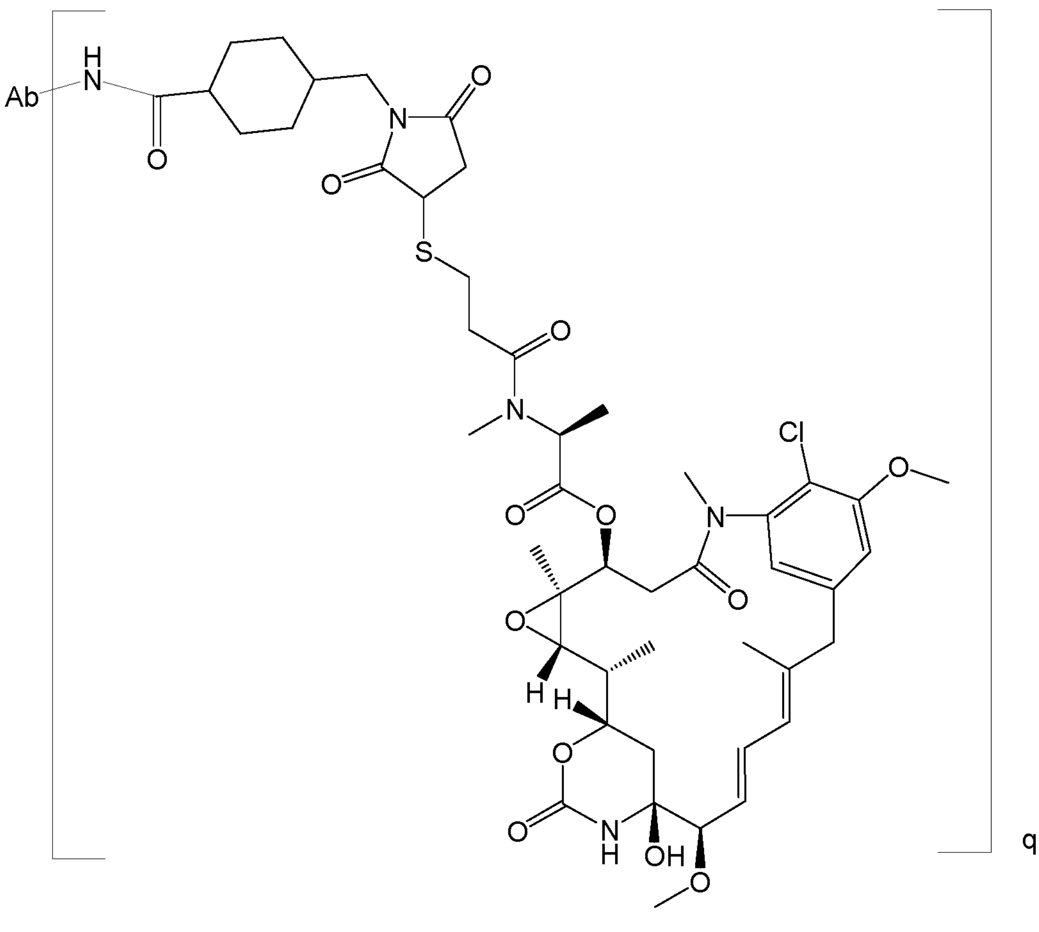

IEX019-05

In the structure, q represents an average DAR of, e.g., 3-5, 3.2-4.8, or 3.0-4.5, such as 3.3 as determined in Table 2;

Ab is HB37A6.

This molecule was prepared as follows.

(a) The linker-payload SMCC-DM1 solution (purchased from Levena Biopharma, SET0101, dissolved in DMSO or other organic solvent) was added to an antibody HB37A6 solution (dissolving antibody HB37A6 in PB buffer), and after the addition, the reaction mixture was shaken in a shaker for 2-5 h, wherein

(i) the optimal concentration of the antibody HB37A6 was 5-10 mg/mL,
(ii) the optimal molar ratio of the linker-payload/mAb was 5.5-6.5,
(iii) the optimum pH for the reaction was generally between 6.5 and 8.0, and
(iv) the optimal temperature for the reaction was 20-37 °C,
thus obtaining the ADC crude product.

(b) The obtained ADC crude product was purified by spin desalting, ultrafiltration, or dialysis to obtain the final ADC product IEX019-05.
(c) The average DAR and SEC purity of the ADC product was determined by using ultraviolet spectrophotometry and SEC high performance liquid chromatography.

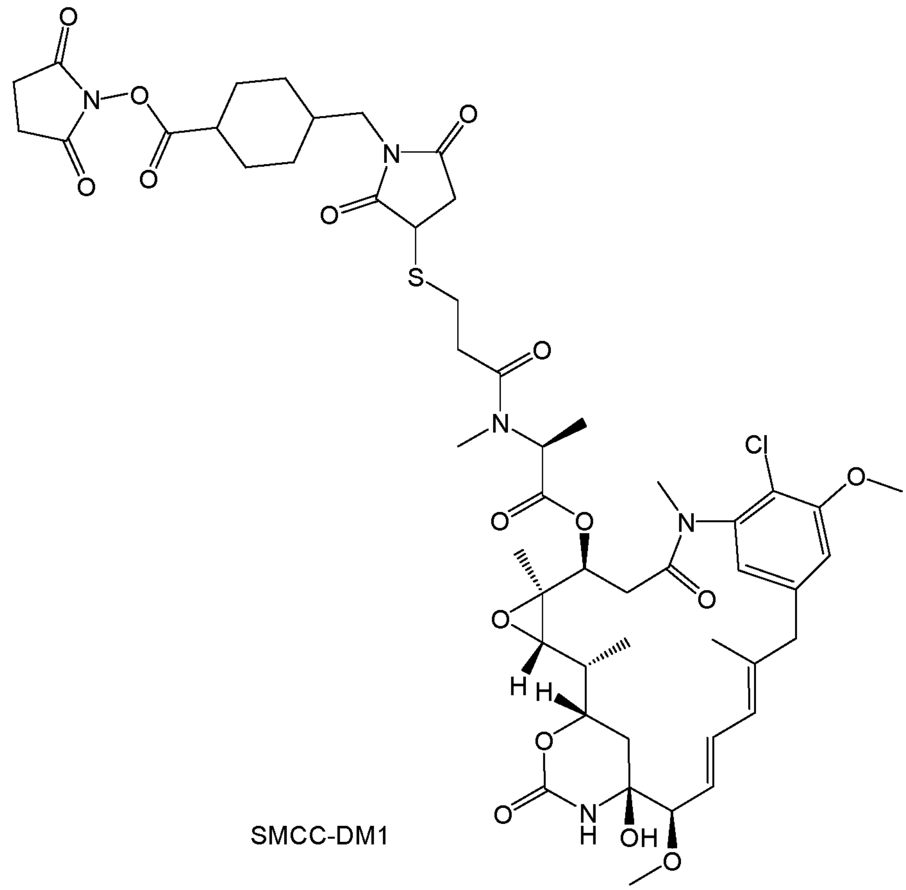

SMCC-DM1

**Example 2.1.5:** Preparation of IEX019-06

The preparation process was similar to that of IEX019-02, except that the HB37A6 monoclonal antibody was replaced by the control antibody IgG.

**Example 2.1.6:** Preparation of IEX019-07

The preparation process was similar to that of IEX019-04, except that the HB37A6 monoclonal antibody was replaced by the control antibody IgG.

The information for all monoclonal antibodies and ADCs is summarized in Table 2.

| | Representative molecule | Note | Linker type | Antibody | Payload | Average DAR (drug-antibody ratio) | SEC purity |
|---|---|---|---|---|---|---|---|
| IEX019-01 | HB37A6 | Monoclonal antibody | N/A | Anti-hCLDN18.2 antibody HB37A6 | None | N.A | >99% |
| IEX019-02 | SYN-HB37A6-Ex | Glycosyl modification-based site-specific conjugation technique | Cleavable | | Exatecan | 3.52 (RP-HPLC) | 99% |
| IEX019-03 | DS-HB37A6-Dxd | Thiol reaction-based coupling technique | Cleavable | | Dxd | 7.9 (HIC) | 98.92 % |
| IEX019-04 | SG-HB37A6-MM AE | Thiol reaction-based random coupling technique | Cleavable | | MMAE | 3.5(HIC) | 99.5% |
| IEX019-05 | IMGN-HB37A6-D M1 | Amine reaction-based random coupling technique | Non-cleavabl e | | DMI | 3.3(SEC) | >95% |
| IEX019-06 | SYN-IgG1-Ex | Glycosyl modification-based site-specific conjugation technique | Cleavable | Control IgG* | Exatecan | 3.74(RP-HP LC) | >99% |
| IEX019-07 | SG-IgG1-MMAE | Thiol reaction-based random coupling technique | Cleavable | Control IgG* | MMAE | 3.7(HIC) | >95% |

*the heavy chain of IgG is SEQ ID NO: 21, and the light chain is SEQ ID NO: 22.

**Example 2.2: Cell Binding Assay of IEX019 Molecules**

To determine whether the binding property of the IEX019-01 monoclonal antibody for target cells was altered in the case of small molecule conjugation, the affinity of IEX019-01 and IEX019-02 for the target was detected by flow cytometry using the DAN-G cell line (hCLDN18.2-negative) and the DAN-G-hCLDN18.2 cell line prepared in Example 1 (over-expressing hCLDN18.2), and the experimental procedure was the same as that in Example 1.5.

IEX019-01 and IEX019-02 did not bind to non-target cell DAN-G, but had a strong affinity for DANG-hCLDN18.2, indicating that antibody binding was dependent on the expression specificity of the target and that conjugation of exatecan did not affect antibody binding. Meanwhile, the control molecule IEX019-06 (the negative control with IgG as the monoclonal antibody, IgG being conjugated to exatecan toxin using the same technique) did not bind to the target cells (FIG. 6).

**Example 2.3: Endocytosis Experiment of IEX019 Molecules**

Strong endocytosis is one of the important properties of ADC drugs. When an ADC binds to the antigen on the surface of the cell membrane, the ADC-antigen complex enters the cell through endocytosis and kills the target cell.

Therefore, the endocytosis efficiency of ADCs is one of the important indicators for determining the tumor inhibition effect.

To detect the endocytosis efficiency of the antibody after being conjugated to a small molecule compound, the endocytosis of different IEX019 molecules on the DANG-hCLDNA18.2 was detected by flow cytometry. After the DANG-hCLDNA18.2 cells were digested, the cell density was adjusted and the cells were plated on a 96-well plate at $1 \times 10^5$ cells/well. The cells were centrifuged at 500 g for 3 min, and the supernatant was discarded. 100 μL of the test molecule was taken to resuspend the cells (molecular concentration: 50 nM), and 5 replicates were set for each sample (i.e., endocytosis time: 0 h, 1 h, 2 h, 3 h, and 4 h). The mixture was placed on ice and incubated for 1 h. 1 h later, the mixture was centrifuged at 500 g for 3 min, and the supernatant was discarded. The FACS buffer (1% FBS, 1× PBS) was added at 200 μL/well to rinse the plate twice. One group of samples were taken, transferred to a new 96-well plate, and incubated at 37 °C for 4 h. The rest of the samples were continued to be incubated on ice. The previous operations were repeated, and samples were incubated at 37 °C for 3 h, 2 h, 1 h, and 0 h, separately. After a specified incubation time, the mixture was centrifuged at 500 g for 3 min, and the supernatant was discarded. The anti-hFC-PE antibody (SouthernBiotech) diluted at a ratio of 1:400 was added at 100 μL/well, and the mixture was incubated in the dark on ice for 30 min. After the incubation of the secondary antibody was completed, the mixture was centrifuged at 500 g for 3 min, and the supernatant was discarded. The FACS buffer was added at 200 μL/well to rinse the plate twice. Finally, the cells were resuspended in 100 μL of PBS, and the suspension was assayed.

The experimental results are shown in FIG. 7. With incubation at 37 °C for 0 h as the zero point of endocytosis, after incubation for 2 h, all molecules reached the maximum limit of endocytosis, about 60%, indicating that after binding to tumor cells, the ADC molecules designed and synthesized based on IEX019-01 maintained strong ability of being endocytosed consistent with that of the monoclonal antibody.

### Example 2.4: *In Vitro* Cell Killing Effect of IEX019 Molecules

The effect of ADCs on cell viability was detected on various cell lines expressing hCLDNA8.2 at different levels (Table 3) using the CellTiter-Glo (Promega, G9242) assay kit.

Table 3. Expression yield of hCLDN18.2 in the cell lines used

| | Origin | IEX019-01 binding strength (relative fluorescence intensity) |
|---|---|---|
| DAN-G | CLS 300162 | 1.643791 |
| SNU620 | KCLB 00620 | 10.47778 |
| NUGC-4 | JCRB0834 | 109.948 |
| DAN-G-hCLDN18.2 | Prepared in Example 1 | 1418.80 |

The cells used were digested with EDTA/Trypsin, the density was adjusted, then the cells were uniformly plated in a 96-well plate (Table 4). Diluted IEX019 molecules (IEX019-02, IEX019-03, and IEX019-04, initial concentration for dilution of 100 nM, dilution factor of 3) at a specific concentration were each added, and wells to which no IEX019 molecule was added were used as control. The mixture was incubated at 37 °C for 5 days. 5 days later, the CellTiter-Glo detection reagent was added at 100 μL/well, and the mixture was incubated for 30 min at room temperature and detected by using a microplate reader. The relative cell viability was calculated (relative cell viability = sample/control × 100%), and curves were fitted using Graph Pad Prism 8.0.

As shown in FIG. 8, the killing of cell lines by ADC molecules was dependent on the expression level of hCLDN18.2 on the surface. In hCLDNA8.2-negative DANG, IEX019 molecules had no significant effect on cell viability (FIG. 8A); in cell lines with moderate expression level (NUGC-4 and SNU620), IEX019 molecules showed a certain degree of cell killing effect (FIG. 8B); IEX019 molecules all had remarkable killing effect in a high-expression cell line DAN-G-hCLDN18.2 (FIG. 8C). This indicated that IEX019 molecules had good selectivity and effectiveness.

Table 4. Cell plating density

| Cell line | Number of cells per well (cell killing experiment) |
|---|---|
| DANG | 3500 |
| SNU620 | 6000 |
| NUGC-4 | 4000 |
| DAN-G-hCLDN18.2 | 3500 |

### Example 2.5: Bystander Killing Effect

During the synthesis of an ADC drug, a small molecule compound can be linked to the antibody via a cleavable linker; after endocytosis of the ADC into the cell membrane, the linker is cleaved, and the small molecule is released to kill the target cell. After the target cell dies, the small molecule compound is released from the target cell and enters the intercellular space to further kill non-target cells within a certain range. This effect is known as the bystander killing effect. Since cells within a tumor can vary greatly in target expression levels (tumor heterogeneity), the bystander killing effect is important for efficient killing of tumor cells and inhibition of tumor growth.

In the present invention, the non-target cell DAN-G and the target cell DANG-hCLDN18.2 were used to detect the bystander killing effect of IEX019 molecules.

After the cells were digested with EDTA/Trypsin, the density was adjusted. A six-well cell culture plate was used, the DANG cells and the DANG-hCLDN18.2 were each added at $7.5 \times 10^4$ cells/well, and the 2 types of cells were cultured together. The test sample (IgG (SEQ ID NO: 21, SEQ ID NO: 22), IEX019-02, IEX019-05, IEX019-06, exatecan (Macklin, E881532) was added at 200 μL/well; the final concentration was 50 nM, with 3 replicates set for each sample. The cell culture plate was placed in a 37 °C incubator and cultured for 5 days. 5 days later, the culture supernatant was discarded, the plate was rinsed with PBS, and then Trypsin-EDTA was added to digest the cells. All the digested cells were collected and transferred to a 96-well plate. According to the antibody incubation process of the flow cytometry, the cells were incubated with the primary antibody (IEX019-01, 100 nM) and the secondary antibody (anti-hFc-PE, SouthernBiotech) at 4 °C for 1 h and 0.5 h, respectively. After the plate was rinsed with PBS, the live/dead Violet dye (Thermo, L34964) was diluted at 1:1000 and added at 100 μL/well, and the mixture was incubated at 4 °C for 20 min. After the plate was rinsed with PBS, the cells were resuspended in 100 μL of PBS, and the suspension was assayed. The cell groups in each sample were distinguished using the five/dead dye, where the IEX019-01-negative (i.e., hCLDN18.2-negative) group was the DAN-G cells and the IEX019-01-positive (i.e., hCLDN18.2-positive) cells were DAN-G-hCLDN18.2. The number of the two types of cells in each sample was counted, the relative cell viability of each type of cell was calculated according to the following equations, and the curves were fitted using Graph Pad Prism 8.0.

Relative viability of DANG cells = number of DNAG cells in sample group/number of DNAG cells in IgG group × 100%

Relative viability of DANG-hCLDN18.2 cells = number of DNAG-18.2 cells in sample group/number of DNAG-18.2 cells in IgG group × 100%

The cell viability in the IgG group was set at 100%.

As shown in FIG. 9, the negative control sample IEX019-06 had no killing effect on both cells; for the IEX019-05 molecule, the DM1 toxin was linked to the IEX019-01 monoclonal antibody via a non-cleavable linker, and this molecule could not kill surrounding non-target cells; therefore, it was only able to kill the DANG-hCLDNA8.2 cells, but had no effect on the DANG cells. Only IEX019-02 had a significant bystander killing effect and was able to kill both target and non-target cells.

### Example 2.6: Anti-Tumor Efficacy of IEX019 Molecules in DAN-G-hCLDN18.2 Mouse

### Xenograft Tumor Model

In order to prove the *in vivo* efficacy of the IEX019 molecules, CB-17-SCID mice were inoculated with the DANG-hCLDN18.2 cells to determine the anti-tumor efficacy of the antibody molecules of the present invention. SPF female CB-17-SCID mice (14-17 g, purchased from Beijing Vital River Laboratory Animal Technology Co., Ltd.) with certificate No. 110011201108225246 were used in the experiment.

DANG-hCLDN18.2 cells were subcultured conventionally for subsequent *in vivo* experiments. The DANG-hCLDN18.2 cells were collected by centrifugation and dispersed in PBS (1×) to form a cell suspension with a cell concentration of $3 \times 10^6$ cells/mL. On day 0, 0.2 mL of the cell suspension was subcutaneously inoculated into the right abdominal region of each of the CB-17 SCID mice to establish the DANG-hCLDN18.2 tumor-bearing mouse models.

On day 5 after tumor cell inoculation, all the mice with the tumor volume in the range of 50.16 $mm^3$ to 136.68 $mm^3$ were divided into groups (6 mice per group) in a serpentine manner, and the dose and route of administration are shown in Table 5. On day 5 after inoculation, the administration was performed. The mice were monitored twice a week for tumor volume and body weight, as shown in FIG. 10a and FIG. 10b, and the monitoring ended on day 92.

The relative tumor growth inhibition (TGI%) was calculated on day 50 after inoculation, and the calculation formula is as follows:

TGI% = 100% × (tumor volume of the control group - tumor volume of the treatment group)/(tumor volume of the control group - tumor volume of the control group before administration).

Tumor volume measurement: the maximum length of major axis (L) and maximum length of minor axis (W) of tumors were measured with a vernier caliper, and tumor volume was calculated using the following formula:

$$V = L \times W^2/2.$$

The mice were weighed using an electronic balance.

Table 5. Experimental design (note: single administration, once in total)

| Group | Dose of administration | Administration frequency | Route of administration |
|---|---|---|---|
| IEX019-01 (control group) | 10 mg/kg | Single administration | Intraperitoneal injection |
| IEX019-02 (treatment group) | 10 mg/kg | Single administration | Intraperitoneal injection |
| IEX019-03 (treatment group) | 10 mg/kg | Single administration | Intraperitoneal injection |
| IEX019-04 (treatment group) | 10 mg/kg | Single administration | Intraperitoneal injection |

The results of tumor growth inhibition are shown in Table 6 and FIG. 10A: on day 50 after inoculation, compared with the IEX019-01 monoclonal antibody, the tumor growth inhibition of IEX019-02 after single administration reached 103.60%, which was significantly better than those of IEX019-03 and IEX019-04, which were 93.70% and 35.20% respectively. 82 days after inoculation, 100% of the mice in the IEX019-02 group showed complete regression of tumors. Meanwhile, the body weight of the mice was measured. As shown in FIG 10B, there was no significant difference in body weight of mice.

Table 6. Tumor growth inhibition on day 50

| Group | Tumor volume ($mm^3$) | Tumor growth inhibition (%) at day 50 | Complete regression of tumor at day 82 |
|---|---|---|---|
| IEX019-01, 10 mg/kg | 2162.19 | Control group, set as base value | CR=0/6 |
| IEX019-02, 10 mg/kg | 11.69 | 103.60 | CR=6/6 |
| IEX019-03, 10 mg/kg | 154.95 | 93.70 | CR=0/6 |
| IEX019-04, 10 mg/kg | 1431.43 | 35.20 | CR=0/6 |

**Example 2.7: Anti-Tumor Efficacy of IEX019 Molecules in NUGC-4 Mouse Xenograft Tumor Model**

In order to prove the *in vivo* efficacy of the IEX019 molecules, CB-17-SCID mice were inoculated with the NUGC-4 cells to determine the anti-tumor efficacy of the antibody molecules of the present invention.

SPF female CB-17-SCID mice (14-17 g, purchased from Beijing Vital River Laboratory Animal Technology Co., Ltd.) with certificate No. 110011201109348141 were used in the experiment.

The NUGC-4 cells were subcultured conventionally for subsequent *in vivo* experiments. The NUGC-4 cells were collected by centrifugation and dispersed in PBS (1×) to form a cell suspension with a cell concentration of $3 \times 10^7$ cells/mL. On day 0, 0.2 mL of the cell suspension was subcutaneously inoculated into the right abdominal region of each of the CB-17 SCID mice to establish the NUGC-4 tumor-bearing mouse models.

On day 5 after tumor cell inoculation, all the mice with the tumor volume in the range of 72.25 $mm^3$ to 140.50 $mm^3$ were divided into groups (6 mice per group) in a serpentine manner, and the dose and route of administration are shown in Table 7. On day 5 after inoculation, the administration was performed. The mice were monitored twice a week for tumor volume and body weight, as shown in FIG. 11A and FIG. 11B, and the monitoring ended on day 40.

The relative tumor growth inhibition (TGI%) was calculated on day 33 after inoculation, and the calculation formula is as follows:

TGI% = 100% × (tumor volume of the control group - tumor volume of the treatment group)/(tumor volume of the control group - tumor volume of the control group before administration).

Tumor volume measurement: the maximum length of major axis (L) and maximum length of minor axis (W) of tumors were measured with a vernier caliper, and tumor volume was calculated using the following formula:

$$V = L \times W^2/2.$$

The mice were weighed using an electronic balance.

Table 7. Experimental design (note: single administration, once in total)

| Group | Dose of administration | Administration frequency | Route of administration |
|---|---|---|---|
| IEX019-06 (control group) | 10 mg/kg | Single administration | Intraperitoneal injection |
| IEX019-02 (treatment group) | 10 mg/kg | Single administration | Intraperitoneal injection |
| IEX019-03 (treatment group) | 10 mg/kg | Single administration | Intraperitoneal injection |

The results of tumor growth inhibition are shown in Tables 8 and FIG. 11A: on day 33 after inoculation, compared with the negative control IEX019-06, the tumor growth inhibition of IEX019-02 and that of IEX019-03 after single administration were 80.04% and 54.31%, respectively. Meanwhile, the body weight of the mice was measured. As shown in FIG 11B, there was no significant difference in body weight of mice.

Table 8. Tumor growth inhibition on day 33

| Group | Tumor volume ($mm^3$) | Tumor growth inhibition (%) |
|---|---|---|
| IEX019-06, 10 mg/kg | 1068.20 | Control group, set as base value |
| IEX019-02, 10 mg/kg | 423.11 | 80.04 |
| IEX019-03, 10 mg/kg | 839.83 | 54.31 |

**Example 2.8: Anti-Tumor Efficacy of IEX019 Molecules in SNU620 Mouse Xenograft Tumor Model**

In order to prove the *in vivo* efficacy of the IEX019 molecules, CB-17-SCID mice were inoculated with the SNU620 cells to determine the anti-tumor efficacy of the molecule (IEX019-02) of the present invention. SPF female CB-17-SCID mice (18-20 g, purchased from Beijing Vital River Laboratory Animal Technology Co., Ltd.) with certificate No. 110011211102179364 were used in the experiment.

The SNU620 cells were subcultured conventionally for subsequent *in vivo* experiments. The SNU620 cells were collected by centrifugation and dispersed in PBS (1×) to form a cell suspension with a cell concentration of $3 \times 10^7$ cells/mL. On day 0, 0.2 mL of the cell suspension was subcutaneously inoculated into the right abdominal region of each of the CB-17 SCID mice to establish the SNU620 tumor-bearing mouse models.

On day 7 after tumor cell inoculation, all the mice with the tumor volume in the range of 58.1 $mm^3$ to 117.3 $mm^3$ were divided into groups (6 mice per group) in a serpentine manner, and the dose and route of administration are shown in Table 9. On day 7 after inoculation, the administration was performed. The mice were monitored twice a week for tumor volume and body weight, as shown in FIG. 12a and FIG. 12b, and the monitoring ended on day 39.

The relative tumor growth inhibition (TGI%) was calculated on day 39 after inoculation, and the calculation formula is as follows:

TGI% = 100% × (tumor volume of the control group - tumor volume of the treatment group)/(tumor volume of the control group - tumor volume of the control group before administration).

Tumor volume measurement: the maximum length of major axis (L) and maximum length of minor axis (W) of tumors were measured with a vernier caliper, and tumor volume was calculated using the following formula:

$$V = L \times W^2/2.$$

The mice were weighed using an electronic balance.

Table 9. Experimental design (note: single administration, once in total)

| Group | Dose of administration | Administration frequency | Route of administration |
|---|---|---|---|
| hIgG (control group, SEQ ID NO: 21, SEQ ID NO: 22) | 10 mg/kg | Single administration | Intraperitoneal injection |
| IEX019-06 (control group) | 10 mg/kg | Single administration | Intraperitoneal injection |
| IEX019-02 (treatment group) | 10 mg/kg | Single administration | Intraperitoneal injection |

The results of tumor growth inhibition are shown in Table 10 and FIG. 12A: on day 39 after inoculation, compared with hIgG, the tumor growth inhibition of IEX019-02 after single administration at 10 mg/kg was 143.77%, and 100% of the mice showed complete regression of tumors. The body weight of mice in each administration group was not significantly different from that in the hIgG control group (FIG. 12B).

Table 10. Tumor growth inhibition on day 39

| Group | Tumor volume ($mm^3$) | Tumor growth inhibition (%) | Complete regression of tumor |
|---|---|---|---|
| hIgG, 10 mg/kg | 275.51 | Control group, set as base value | CR=0/7 |
| IEX019-06, 10 mg/kg | 291.82 | N/A | CR=0/7 |
| IEX019-02, 10 mg/kg | 0.00 | 143.77 | CR=7/7 |

Sequence information:

| SEQ ID NO | Name of antibody | HB37A6 (HCDR1 defined by the AbM rules, and HCDR2, HCDR3, LCDR1, LCDR2, and LCDR3 defined by the Kabat rules) |
|---|---|---|
| 1 | HCDR1 | GFTFSSYVMS |
| 2 | HCDR2 | TISHSGGSTYYADSVKG |
| 3 | HCDR3 | DAPYYDILTGYRY |
| 4 | Heavy chain variable region (VH) | EVQLLDSGGGLVQPGGSLRLSCAASGFTFSSYVMSWVRQAPGK GLNWVSTISHSGGSTYYADSVKGRFTISRDNSKNTLYLQMNSLR AEDTAVYYCAIDAPYYDILTGYRYWGQGTLVTVSS |
| 5 | Heavy chain constant region (HC) | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTIS KAKGQPREPQVYTLPPSRDELTKNQVSLTCLVKGFYPSDIAVEW ESNGQPENNYKTTPPVLDSDGSFFLYSKLTVDKSRWQQGNVFS CSVMHEALHNHYTQKSLSLSPGK |

(continued)

| SEQ ID NO | Name of antibody | HB37A6 (HCDR1 defined by the AbM rules, and HCDR2, HCDR3, LCDR1, LCDR2, and LCDR3 defined by the Kabat rules) |
|---|---|---|
| 11 | Heavy chain | EVQLLDSGGGLVQPGGSLRLSCAASGFTFSSYVMSWVRQAPGK GLNWVSTISHSGGSTYYADSVKGRFTISRDNSKNTLYLQMNSLR AEDTAVYYCAIDAPYYDILTGYRYWGQGTLVTVSSASTKGPSV FPLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQP REPQVYTLPPSRDELTKNQVSLTCLVKGFYPSDIAVEWESNGQP ENNYKTTPPVLDSDGSFFLYSKLTVDKSRWQQGNVFSCSVMHE ALHNHYTQKSLSLSPGK |
| 6 | LCDR1 | RASQSISSWLA |
| 7 | LCDR2 | KASSLES |
| 8 | LCDR3 | QQYNSYSYT |
| 9 | Light chain variable region (VL) | DIQMTQSPSTLSASVGDRVTITCRASQSISSWLAWYQQKPGKAP KLLIYKASSLESGVPSRFSGSGSGTEFTLTISSLQPDDFATYYCQQ YNSYSYTFGQGTKLEIK |
| 10 | Light chain | RTVAAPSVFIFPPSDEQLKSGTASVVCLLNNFYPREAKVQWKVD |
| | constant region (LC) | NALQSGNSQESVTEQDSKDSTYSLSSTLTLSKADYEKHKVYACE VTHQGLSSPVTKSFNRGEC |
| 12 | Light Chain | DIQMTQSPSTLSASVGDRVTITCRASQSISSWLAWYQQKPGKAP KLLIYKASSLESGVPSRFSGSGSGTEFTLTISSLQPDDFATYYCQQ YNSYSYTFGQGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC |
| SEQ ID NO | Name of antibody | Positive control antibody Zmab |
| 13 | HCDR1 | GYTFTSYWIN |
| 14 | HCDR2 | NIYPSDSYTNYNQKFK |
| 15 | HCDR3 | SWRGNSFDY |
| 16 | VH | QVQLQQPGAELVRPGASVKLSCKASGYTFTSYWINWVKQRPGQ GLEWIGNIYPSDSYTNYNQKFKDKATLTVDKSSSTAYMQLSSPT SEDSAVYYCTRSWRGNSFDYWGQGTTLTVSS |

(continued)

| SEQ ID NO | Name of antibody | Positive control antibody Zmab |
|---|---|---|
| 5 | HC | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKAEPKSCDKTHTCPPCPAPELLGGPSVFLFPPKPKDTL MISRTPEVTCVVVDVSHEDPEVKFNWYVDGVEVHNAKTKPREE QYNSTYRVVSVLTVLHQDWLNGKEYKCKVSNKALPAPIEKTIS KAKGQPREPQVYTLPPSRDELTKNQVSLTCLVKGFYPSDIAVEW ESNGQPENNYKTTPPVLDSDGSFFLYSKLTVDKSRWQQGNVFS CSVMHEALHNHYTQKSLSLSPGK |
| 17 | LCDR1 | KSSQSLLNSGNQKNYLT |
| 18 | LCDR2 | WASTRES |
| 19 | LCDR3 | QNDYSYPFT |
| 20 | VL | DIVMTQSPSSLTVTAGEKVTMSCKSSQSLLNSGNQKNYLTWYQ QKPGQPPKLLIYWASTRESGVPDRFTGSGSGTDFTLTISSVQAED LAVYYCQNDYSYPFTFGSGTKLEIK |
| 10 | LC | RTVAAPSVFIFPPSDEQLKSGTASVVCLLNNFYPREAKVQWKVD NALQSGNSQESVTEQDSKDSTYSLSSTLTLSKADYEKHKVYACE VTHQGLSSPVTKSFNRGEC |
| SEQ ID NO | Name of antibody | Negative control IgG |
| 21 | Heavy chain | QVQLVQSGAEVKKPGASVKVSCQASGYRFSNFVIHWVRQAPG QRFEWMGWINPYNGNKEFSAKFQDRVTFTADTSANTAYMELR SLRSADTAVYYCARVGPYSWDDSPQDNYYMDVWGKGTTVIVS SASTKGPSVFPLAPSSKSTSGGTAALGCLVKDYFPEPVTVSWNS GALTSGVHTFPAVLQSSGLYSLSSVVTVPSSSLGTQTYICNVNHK PSNTKVDKKVEPKSCDKTHTCPPCPAPELLGGPSVFLFPPKPKDT LMISRTPEVTCVVVDVSHEDPEVKFNWYVDGVEVHNAKTKPRE EQYNSTYRVVSVLTVLHQDWLNGKEYKCKVSNKALPAPIEKTI SKAKGQPREPQVYTLPPSRDELTKNQVSLTCLVKGFYPSDIAVE WESNGQPENNYKTTPPVLDSDGSFFLYSKLTVDKSRWQQGNVF SCSVMHEALHNHYTQKSLSLSPGK |
| 22 | Light chain | EIVLTQSPGTLSLSPGERATFSCRSSHSIRSRRVAWYQHKPGQAP RLVIHGVSNRASGISDRFSGSGSGTDFTLTITRVEPEDFALYYCQ VYGASSYTFGQGTKLERKRTVAAPSVFIFPPSDEQLKSGTASVV CLLNNFYPREAKVQWKVDNALQSGNSQESVTEQDSKDSTYSLS STLTLSKADYEKHKVYACEVTHQGLSSPVTKSFNRGEC |

## Claims

1. An antibody-drug conjugate having formula I:

$$Ab\text{-}(L\text{-}(D)_r)_p \qquad (I)$$

or a pharmaceutically acceptable salt or solvate thereof,
wherein:

Ab is an antibody or a fragment thereof that binds to CLDN18.2 (e.g., human CLDN18.2);
L is a linker;
D is a drug, such as an anti-tumor compound; and
p is 1-10, e.g., 1-9, 2-8, 3-7, 4-6, or 2-6, such as 1, 2, 3, 4, 5, 6, 7, 8, 9, or 10;
r is 1-5, preferably 1 or 2;
wherein
Ab in the formula (I) comprises HCDR1, HCDR2, and HCDR3 having amino acid sequences set forth in SEQ ID NOs: 1, 2, and 3, respectively, and LCDR1, LCDR2, and LCDR3 having amino acid sequences set forth in SEQ ID NOs: 6, 7, and 8, respectively.

2. The antibody-drug conjugate or the pharmaceutically acceptable salt or solvate thereof according to claim 1, wherein Ab in the formula (I) comprises a heavy chain variable region and/or a light chain variable region, wherein the heavy chain variable region

(i) comprises or consists of an amino acid sequence having at least 90%, 91%, 92%, 93%, 94%, 95%, 96%, 97%, 98%, or 99% identity to the amino acid sequence set forth in SEQ ID NO: 4; or
(ii) comprises or consists of the amino acid sequence set forth in SEQ ID NO: 4; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence set forth in SEQ ID NO: 4, wherein preferably, the amino acid changes do not occur in the CDRs; and/or

the light chain variable region

(i) comprises or consists of an amino acid sequence having at least 90%, 91%, 92%, 93%, 94%, 95%, 96%, 97%, 98%, or 99% identity to the amino acid sequence set forth in SEQ ID NO: 9; or
(ii) comprises or consists of the amino acid sequence set forth in SEQ ID NO: 9; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence set forth in SEQ ID NO: 9, wherein preferably, the amino acid changes do not occur in the CDRs.

3. The antibody-drug conjugate or the pharmaceutically acceptable salt or solvate thereof according to any one of claims 1-2, wherein Ab in the formula (I) further comprises a heavy chain constant region and/or a light chain constant region.

4. The antibody-drug conjugate or the pharmaceutically acceptable salt or solvate thereof according to any one of claims 1-3, wherein Ab in the formula (I) is an IgG antibody.

5. The antibody-drug conjugate or the pharmaceutically acceptable salt or solvate thereof according to any one of claims 1-4, wherein the heavy chain constant region of Ab in the formula (I) is from IgG1, IgG2, IgG3, or IgG4, preferably IgG1.

6. The antibody-drug conjugate or the pharmaceutically acceptable salt or solvate thereof according to any one of claims 1-5, wherein Ab in the formula (I) comprises a heavy chain and a light chain, wherein the heavy chain

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 11;
(ii) comprises or consists of the amino acid sequence set forth in SEQ ID NO: 11; or

(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 11; and

the light chain

(i) comprises or consists of an amino acid sequence having at least 85%, 90%, 91%, 92%, 93%, 94%, 95%, 96%, 97%, 98%, or 99% identity to the amino acid sequence set forth in SEQ ID NO: 12;
(ii) comprises or consists of the amino acid sequence set forth in SEQ ID NO: 12; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence set forth in SEQ ID NO: 12.

**7.** The antibody-drug conjugate or the pharmaceutically acceptable salt or solvate thereof according to any one of claims 1-6, wherein Ab in the formula (I) is a human antibody.

**8.** The antibody-drug conjugate or the pharmaceutically acceptable salt or solvate thereof according to any one of claims 1-7, wherein Ab in the formula (I) is an antigen-binding fragment, such as an Fv; a Fab; a Fab'; a Fab'-SH; a $F(ab')_2$; a dAb (domain antibody); a linear antibody; a single-chain antibody (e.g., scFv); a single-domain antibody, e.g., VHH; a bi-valent antibody or a fragment thereof; or a camelid antibody.

**9.** The antibody-drug conjugate or the pharmaceutically acceptable salt or solvate thereof according to any one of claims 1-8, wherein the linker is MC-VC-PAB, vc-PAB, SMCC, or MC-GGFG.

**10.** The antibody-drug conjugate or the pharmaceutically acceptable salt or solvate thereof according to any one of claims 1-8, wherein the antibody is an antibody with an engineered glycosylation, e.g., an antibody obtained after enzymatic modification of a glycan chain *in vitro* (e.g., modification of a glycan chain by a glycosidase (e.g., an endoglycosidase or a glycosyltransferase)); preferably, the antibody with an engineered glycosylation is an antibody in which a glycan chain at a glycosylation site of the antibody is engineered from an N-glycan chain with a non-homogeneous structure to an N-glycan chain with a single structure having a reactive group (e.g., any reactive group capable of reacting with a linker moiety, such as azido, keto, and alkynyl); more preferably, the N-glycosylation site is a conservative N-glycosylation site, e.g., Asn297, on an Fc domain of the antibody.

**11.** The antibody-drug conjugate or the pharmaceutically acceptable salt or solvate thereof according to any one of claims 1-8, wherein the linker is a linker enabling site-specific conjugation and preferably a linker linked to an antibody oligosaccharide; preferably, the oligosaccharide linker is a linker comprising alkynyl; most preferably, the oligosaccharide linker is a linker enabling site-specific conjugation to a reactive group (e.g., azido) on an N-glycan chain at a conservative N-glycosylation site (e.g., Asn297) on an Fc domain of the antibody.

**12.** The antibody-drug conjugate or the pharmaceutically acceptable salt or solvate thereof according to any one of claims 1-11, wherein the anti-tumor compound is a cytotoxic agent or a chemotherapeutic agent, such as exatecan, Dxd, MMAE, or DM1.

**13.** An antibody-drug conjugate having formula II or a pharmaceutically acceptable salt or solvate thereof

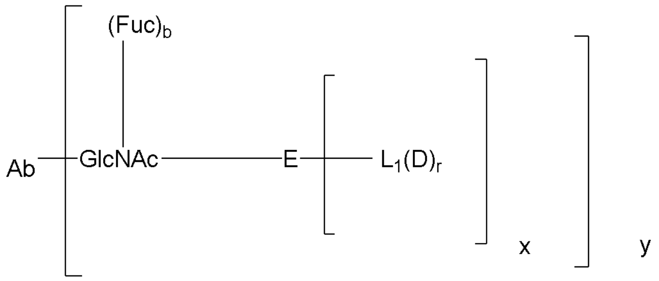

(formula II)

wherein

Ab is as defined in one of claims 1-8,
$L_1$ is a linker;
E is a saccharide or a saccharide derivative and is preferably selected from galactose (Gal), mannose (Man), N-acetylglucosamine (GlcNAc), glucose (Glc), N-acetylgalactosamine (GalNAc), glucuronic acid (Gcu), fucose (Fuc), and N-acetylneuraminic acid (sialic acid);
GlcNAc is N-acetylglucosamine, and Fuc is fucose;
D and r are as defined in the formula I of one of claims 1-8;
b is 0 or 1;
x is 1, 2, 3, or 4;
y is 1-20.

**14.** The antibody-drug conjugate or the pharmaceutically acceptable salt or solvate thereof according to claim 13, wherein

x is 1 or 2; and
y is 1-10;
preferably, x is 1 and y is 2.

**15.** The antibody-drug conjugate or the pharmaceutically acceptable salt or solvate thereof according to claim 13, wherein E is GalNAc.

**16.** The antibody-drug conjugate or the pharmaceutically acceptable salt or solvate thereof according to claim 15, wherein E is 6-deoxy-2-acetylgalactosamine and is linked to $L_1$ through the C atom at position 6.

**17.** The antibody-drug conjugate or the pharmaceutically acceptable salt or solvate thereof according to any one of claims 13-15, wherein GlcNAc linked to Ab is present at Asn297-glycosylation sites of the two heavy chains of the antibody.

**18.** The antibody-drug conjugate or the pharmaceutically acceptable salt or solvate thereof according to any one of claims 11-17, wherein $L_1$ has the following structure:

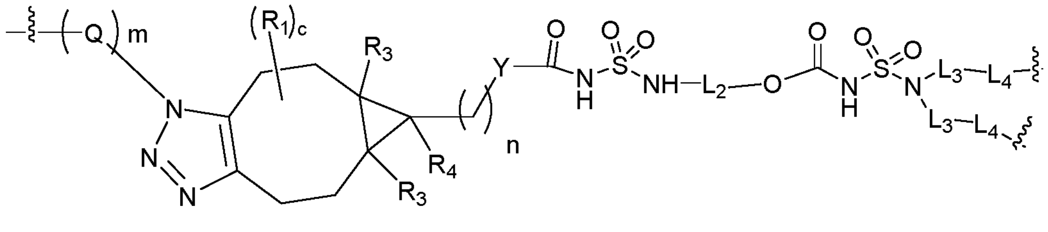

wherein, Q is $-N(H)C(O)CH_2-$ or $-CH_2-$;

$R_1$ is independently selected from hydrogen, halogen, $-OR_2$, -NOz, -CN, $-S(O)_2R_2$, $C_1$-$C_{12}$ alkyl, aryl, heteroaryl, $C_1$-$C_{12}$ alkylaryl, $C_1$-$C_{12}$ alkylheteroaryl, aryl$C_1$-$C_{12}$ alkyl, and heteroaryl$C_1$-$C_{12}$ alkyl, wherein the alkyl, aryl, heteroaryl, alkylaryl, alkylheteroaryl, arylalkyl, and heteroarylalkyl are optionally substituted, wherein two sub-

stituents $R_1$ may be linked together to form a fused, bridged, or spiro cycloalkyl or a fused or spiro arene or heteroarene substituent, and wherein $R_2$ is independently selected from hydrogen, halogen, $C_1$-$C_{24}$ alkyl, aryl, heteroaryl, $C_1$-$C_{12}$ alkylaryl, $C_1$-$C_{12}$ alkylheteroaryl, aryl$C_1$-$C_{12}$ alkyl, and heteroaryl$C_1$-$C_{12}$ alkyl;
$R_3$ and $R_4$ are each independently selected from hydrogen, halogen, $C_1$-$C_{24}$ alkyl, aryl, heteroaryl, $C_1$-$C_{12}$ alkylaryl, $C_1$-$C_{12}$ alkylheteroaryl, aryl$C_1$-$C_{12}$ alkyl, and heteroaryl$C_1$-$C_{12}$ alkyl;
c is 0, 1, 2, 3, or 4;
m is 0 or 1;
n is 0, 1, 2, 3, 4, 5, 6, 7, 8, 9, or 10;
Yis O, S, or $NR_1$;
$L_2$ and $L_3$ are each independently $C_1$-$C_{12}$ alkyl, wherein one or more carbon atoms in the alkyl are optionally replaced by a heteroatom selected from O, N, and S, provided that the O, N, and/or S are not directly linked to each other;
$L_4$ are each independently a cleavable linker.

**19.** The antibody-drug conjugate or the pharmaceutically acceptable salt or solvate thereof according to claim 18, wherein $L_4$ are each independently

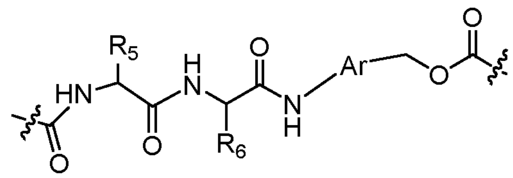

,

wherein, $R_5$ and $R_6$ are each independently selected from hydrogen and $C_1$-$C_{12}$ alkyl, and
Ar is selected from aryl.

**20.** The antibody-drug conjugate or the pharmaceutically acceptable salt or solvate thereof according to claim 19, wherein $L_4$ are each independently

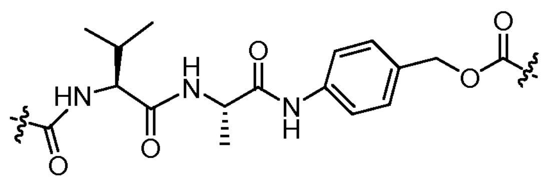

.

**21.** The antibody-drug conjugate or the pharmaceutically acceptable salt or solvate thereof according to any one of claims 13-17, wherein -$L_1$- has the following structure:

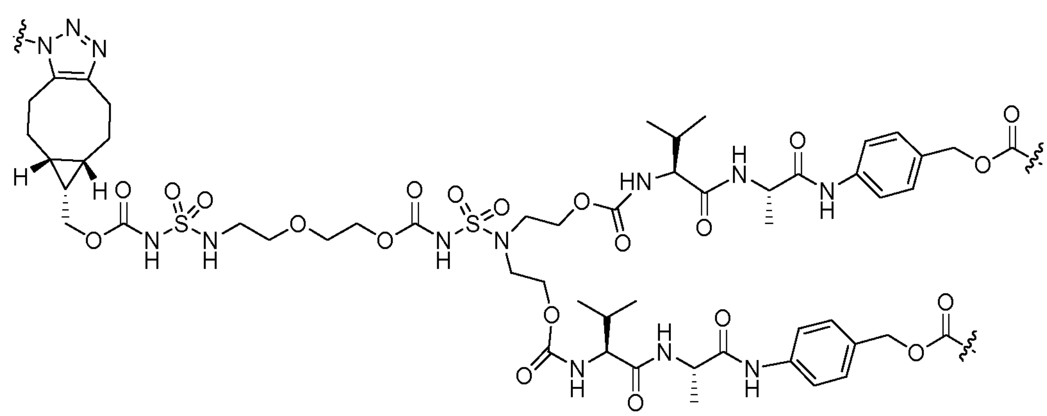

.

**22.** The antibody-drug conjugate or the pharmaceutically acceptable salt or solvate thereof according to any one of claims 1-21, wherein the antibody-drug conjugate has an average DAR of 1-15, such as 2-10, 2-8, or 3-5.

**23.** The antibody-drug conjugate or the pharmaceutically acceptable salt or solvate thereof according to any one of

claims 1-13, wherein the antibody-drug conjugate is selected from:

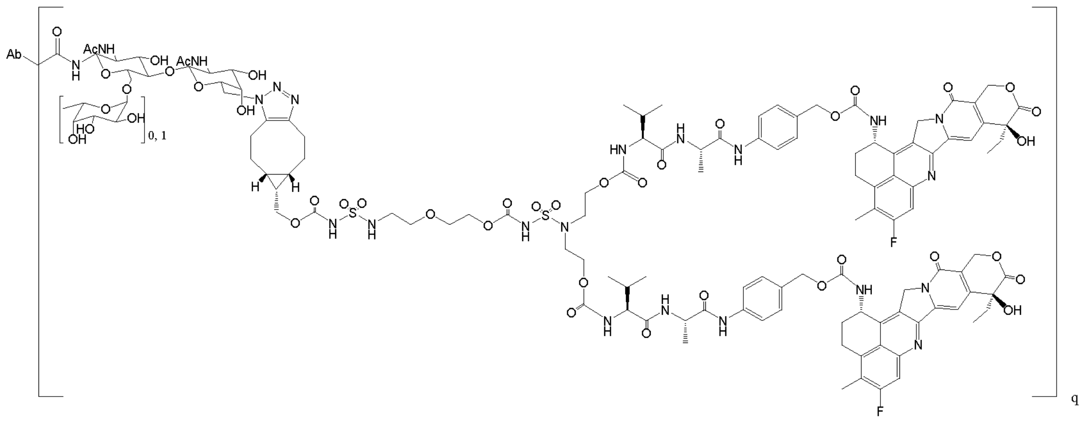

IEX019-02

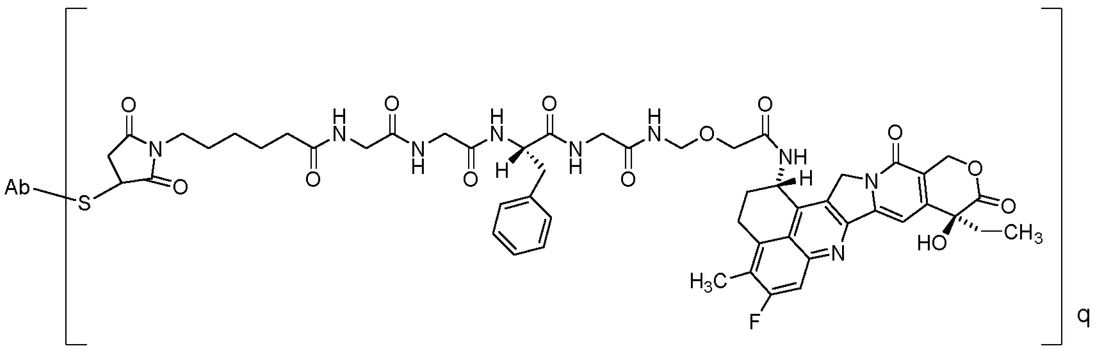

IEX019-03

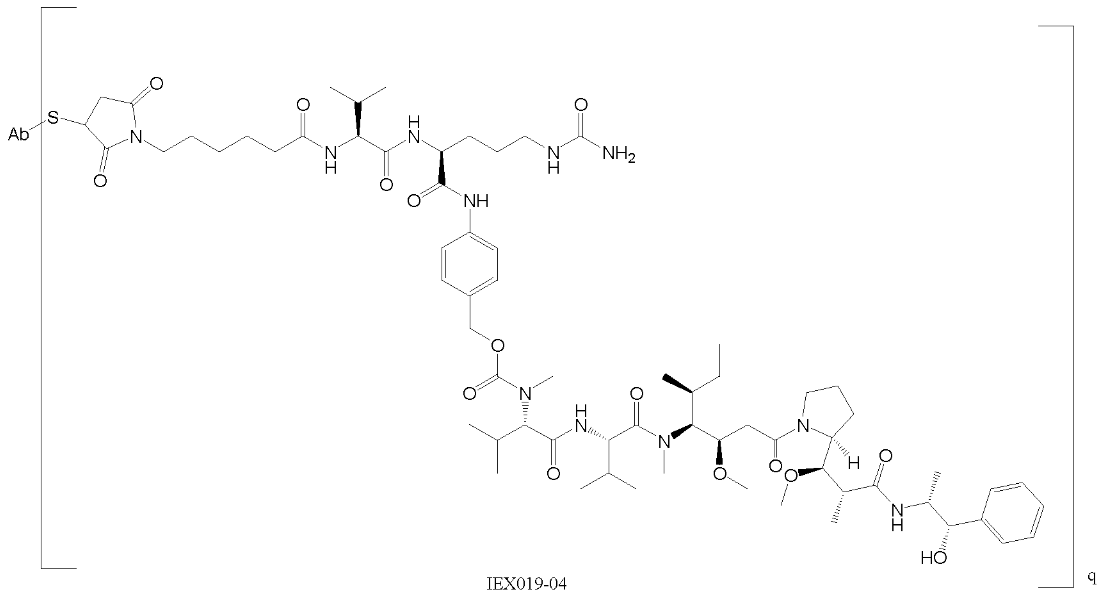

IEX019-04

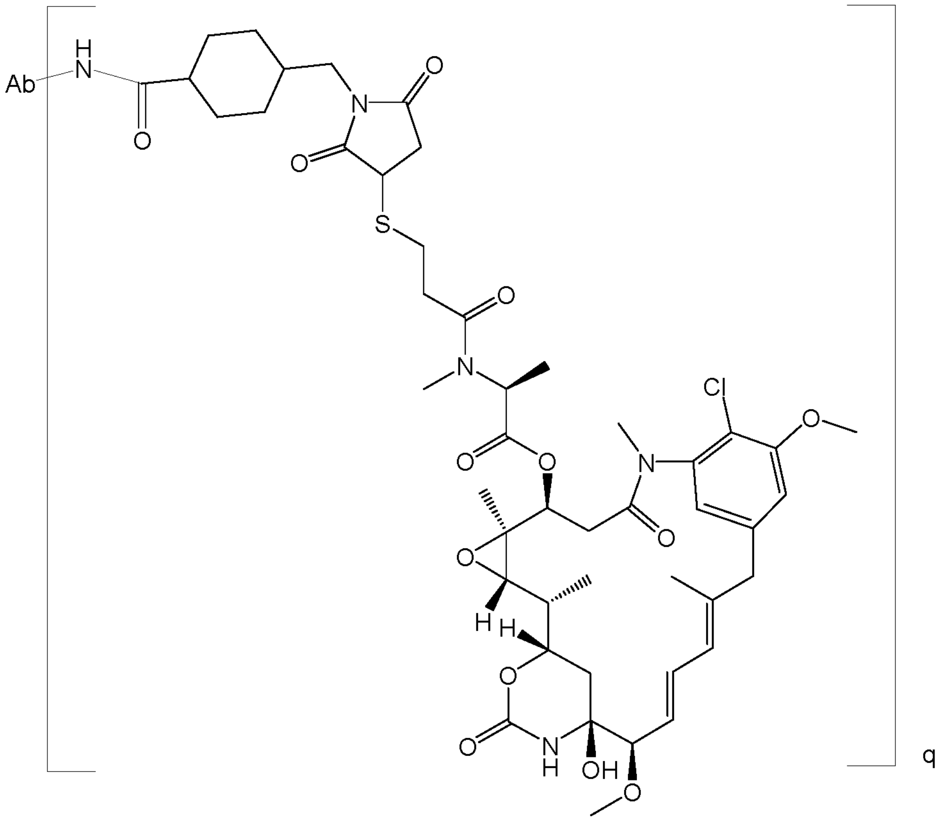

IEX019-05

wherein, Ab is HB37A6;

q represents an average DAR;
in IEX019-02, IEX019-04, and IEX019-05, q is 2-5, such as 3-5 or 3.5-4.5;
in IEX019-03, q is 5-11, such as 7-9 or 7.5-8.5.

**24.** A pharmaceutical composition, comprising the antibody-drug conjugate or the pharmaceutically acceptable salt or solvate thereof according to any one of claims 1-23, and optionally one or more other therapeutic agents, such as a chemotherapeutic agent, an angiogenesis inhibitor, a cytokine, a cytotoxic agent, other antibodies, a small molecule drug, or an immunomodulatory agent, and optionally a pharmaceutical supplementary material.

**25.** A pharmaceutical combination, comprising the antibody-drug conjugate or the pharmaceutically acceptable salt or solvate thereof according to any one of claims 1-23, and optionally one or more other therapeutic agents, such as a chemotherapeutic agent, an angiogenesis inhibitor, a cytokine, a cytotoxic agent, other antibodies, a small molecule drug, or an immunomodulatory agent.

**26.** A method for preventing or treating a tumor in a subject, comprising administering to the subject an effective amount of the antibody-drug conjugate or the pharmaceutically acceptable salt or solvate thereof according to any one of claims 1-23, the pharmaceutical composition according to claim 24, or the pharmaceutical combination according to claim 25.

**27.** The method according to claim 26, wherein the tumor is a cancer; preferably, the cancer has an elevated level (e.g., at the nucleic acid or protein level) of CLDN18.2; preferably, tumor cells in the subject moderately or highly express CLDN18.2; the cancer is, e.g., epithelial cancer or gastrointestinal cancer, such as gastric cancer, gastroesophageal junction cancer, pancreatic cancer, colorectal cancer, or colon cancer.

**28.** The method according to any one of claims 26-27, further comprising administering to a patient one or more therapies, such as therapeutic modalities and/or other therapeutic agents, wherein preferably, the therapeutic modalities comprise radiotherapy or surgery, or the therapeutic agents comprise a chemotherapeutic agent, an angiogenesis

inhibitor, a cytokine, a cytotoxic agent, other antibodies, a small molecule drug, or an immunomodulatory agent.

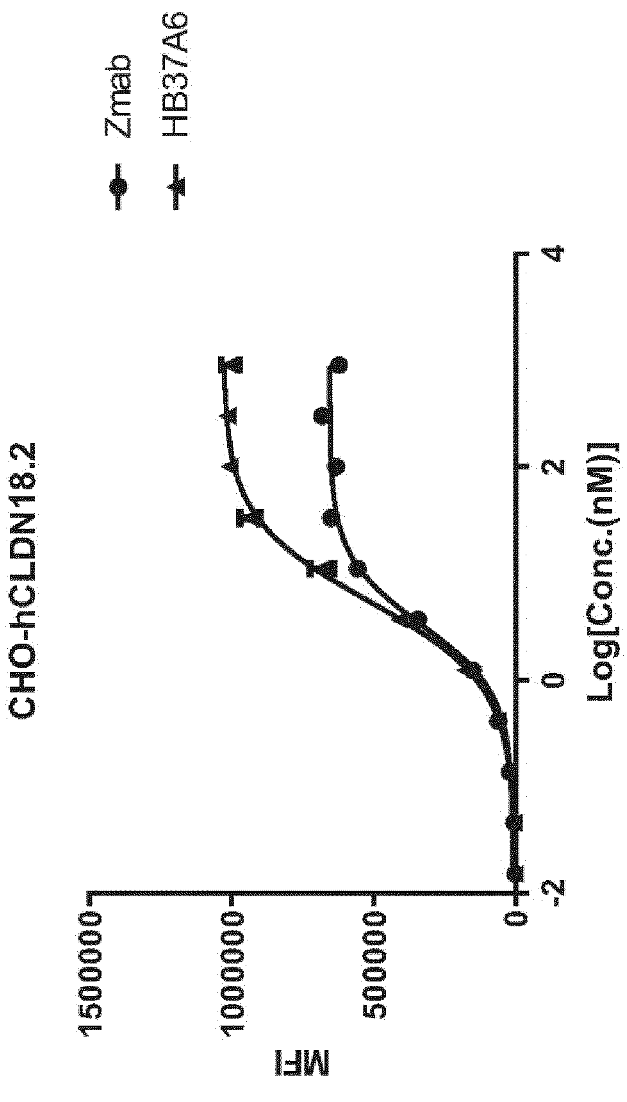


| | Zmab | HB37A6 |
|---|---|---|
| EC50 | 3.313 | 5.513 |

FIG. 1

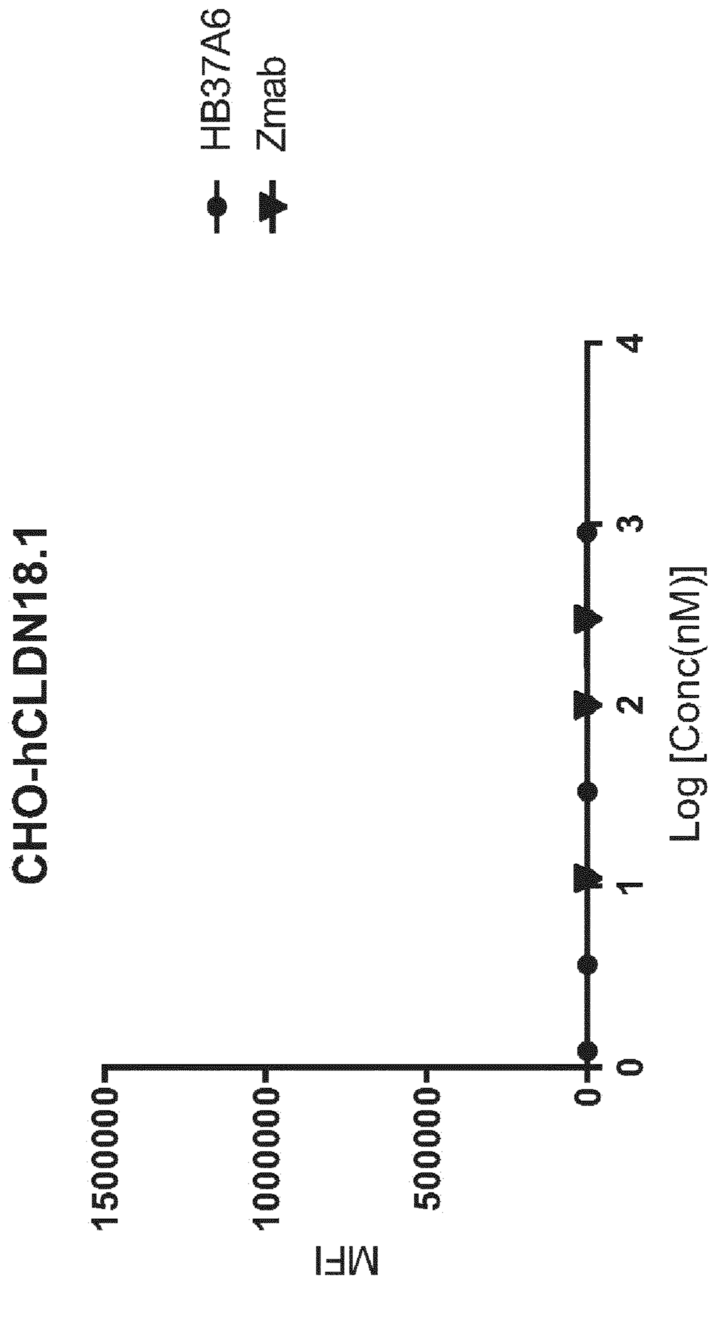
CHO-hCLDN18.1
HB37A6
Zmab
MFI
1500000
1000000
500000
0
0
1
2
3
4
Log [Conc(nM)]

FIG. 2

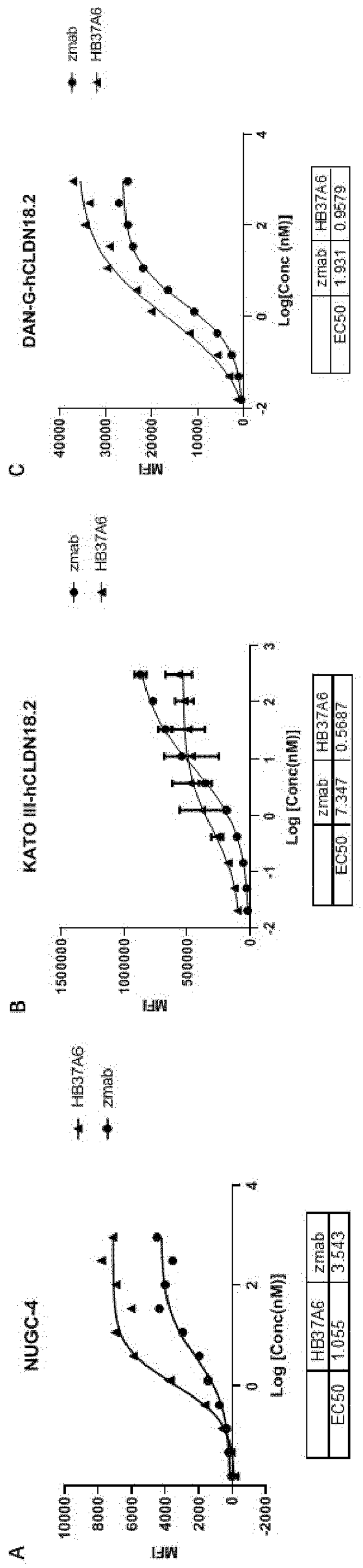
A
NUGC-4
MFI
10000
8000
6000
4000
2000
0
-2000
0
2
4
Log [Conc(nM)]
HB37A6
zmab
EC50
1.055
3.543
B
KATO III-hCLDN18.2
MFI
1500000
1000000
500000
0
-2
-1
0
1
2
3
Log [Conc(nM)]
zmab
HB37A6
EC50
7.347
0.5687
C
DAN-G-hCLDN18.2
MFI
40000
30000
20000
10000
0
-2
0
2
4
Log[Conc (nM)]
zmab
HB37A6
EC50
1.931
0.9579

FIG. 3

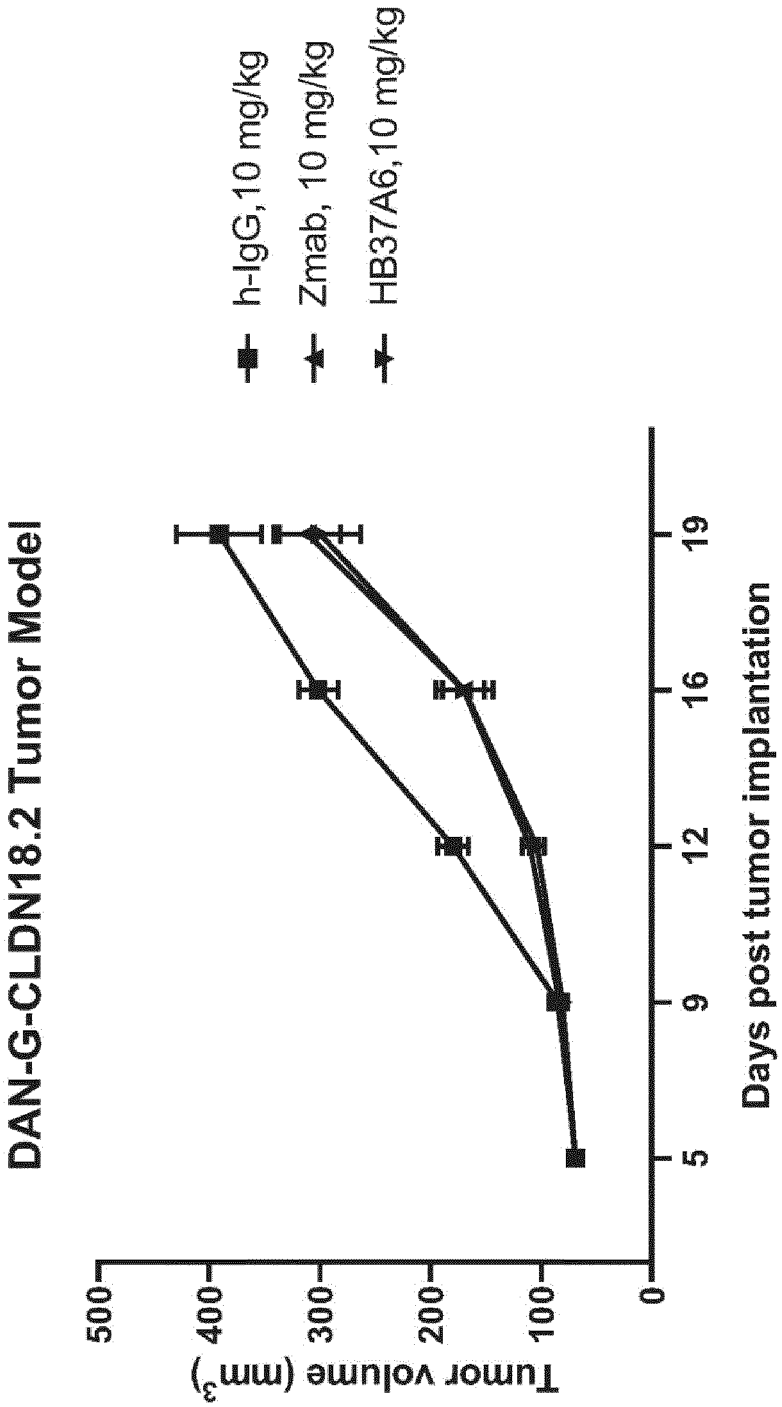
DAN-G-CLDN18.2 Tumor Model
Tumor volume (mm³)
0
100
200
300
400
500
5
9
12
16
19
Days post tumor implantation
h-IgG,10 mg/kg
Zmab, 10 mg/kg
HB37A6,10 mg/kg

FIG. 4

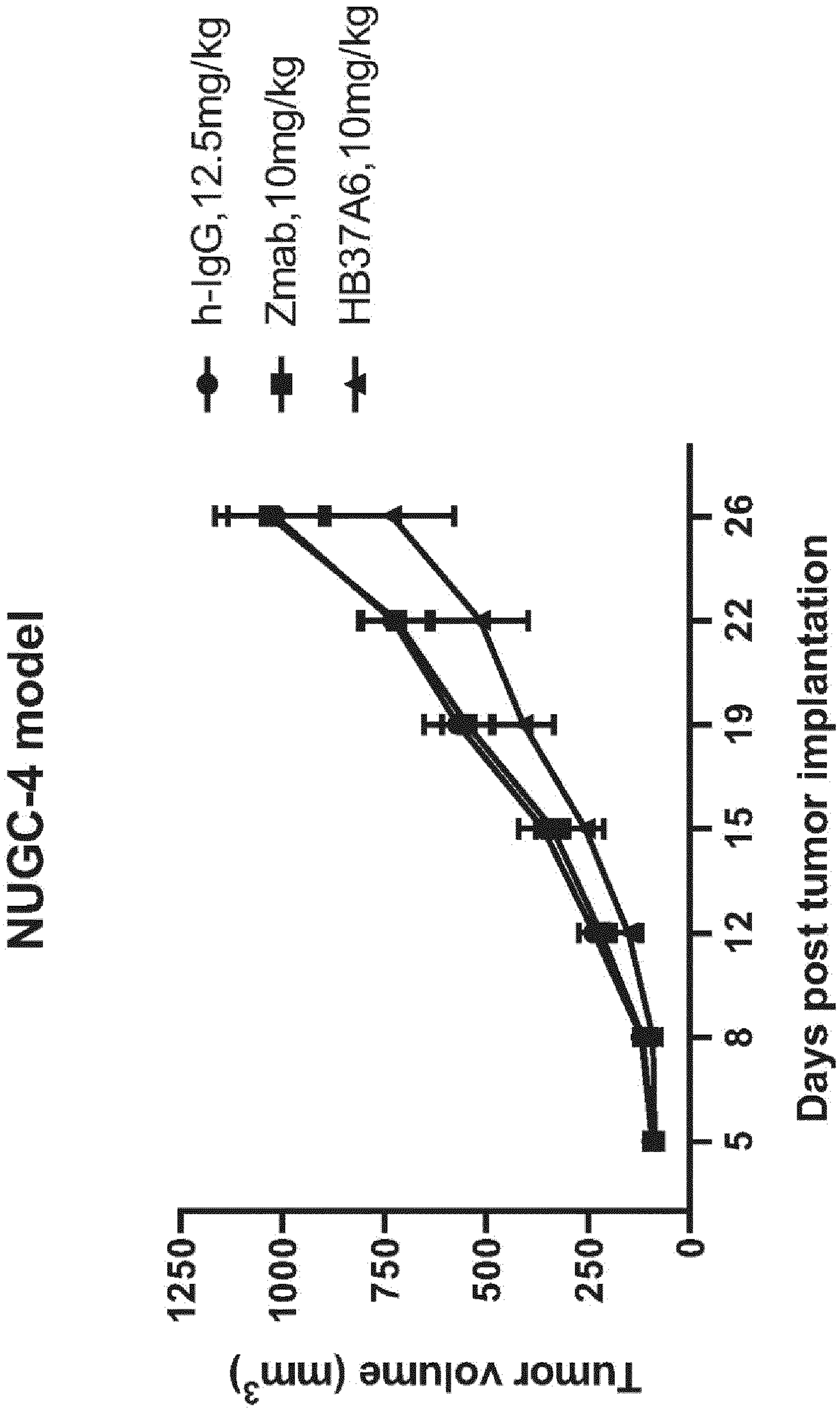
NUGC-4 model
Tumor volume (mm3)
1250
1000
750
500
250
0
5
8
12
15
19
22
26
Days post tumor implantation
h-IgG,12.5mg/kg
Zmab,10mg/kg
HB37A6,10mg/kg

FIG. 5

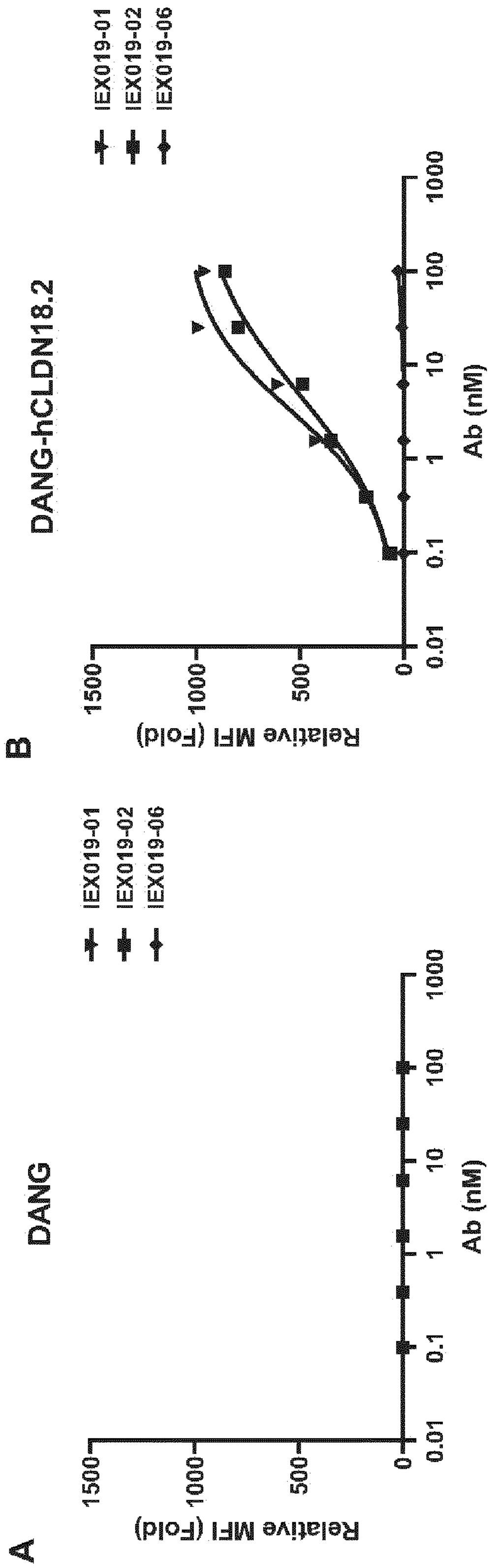
A
DANG
Relative MFI (Fold)
1500
1000
500
0
0.01
0.1
1
10
100
1000
Ab (nM)
IEX019-01
IEX019-02
IEX019-06
B
DANG-hCLDN18.2
Relative MFI (Fold)
1500
1000
500
0
0.01
0.1
1
10
100
1000
Ab (nM)
IEX019-01
IEX019-02
IEX019-06

FIG. 6

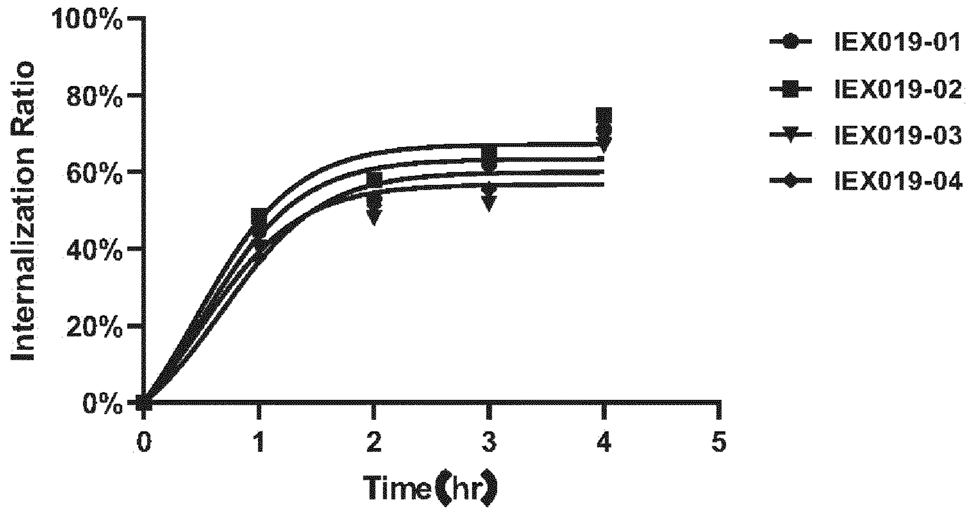
100%
80%
60%
40%
20%
0%
Internalization Ratio
0
1
2
3
4
5
Time(hr)
IEX019-01
IEX019-02
IEX019-03
IEX019-04

Fig. 7.

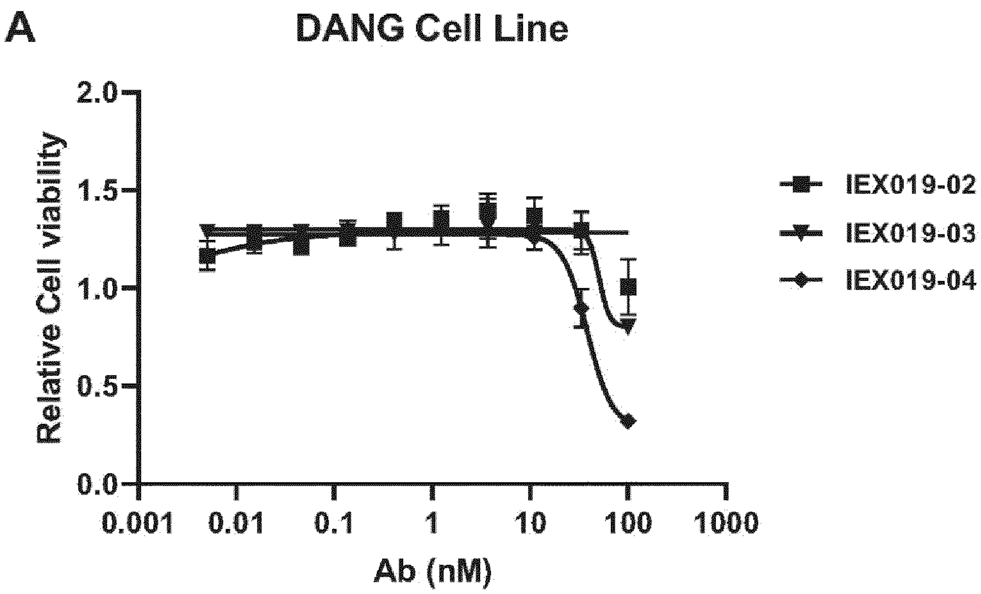
A
DANG Cell Line
2.0
1.5
1.0
0.5
0.0
Relative Cell viability
0.001
0.01
0.1
1
10
100
1000
Ab (nM)
IEX019-02
IEX019-03
IEX019-04

FIG. 8A

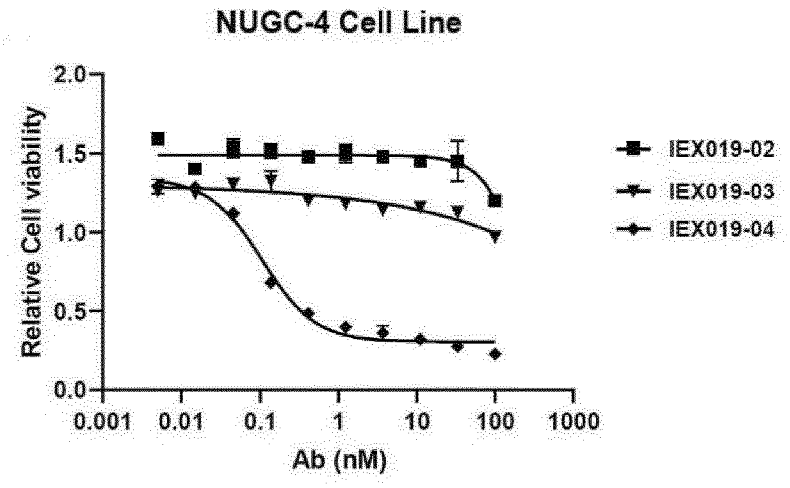
NUGC-4 Cell Line
Relative Cell viability
2.0
1.5
1.0
0.5
0.0
0.001
0.01
0.1
1
10
100
1000
Ab (nM)
IEX019-02
IEX019-03
IEX019-04

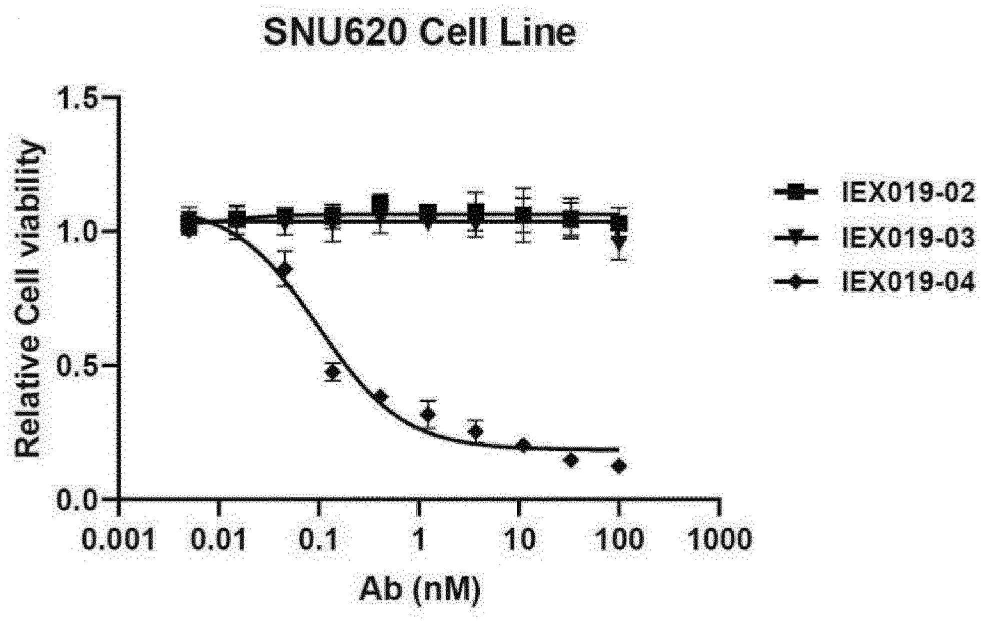
SNU620 Cell Line
Relative Cell viability
1.5
1.0
0.5
0.0
0.001
0.01
0.1
1
10
100
1000
Ab (nM)
IEX019-02
IEX019-03
IEX019-04

FIG. 8B

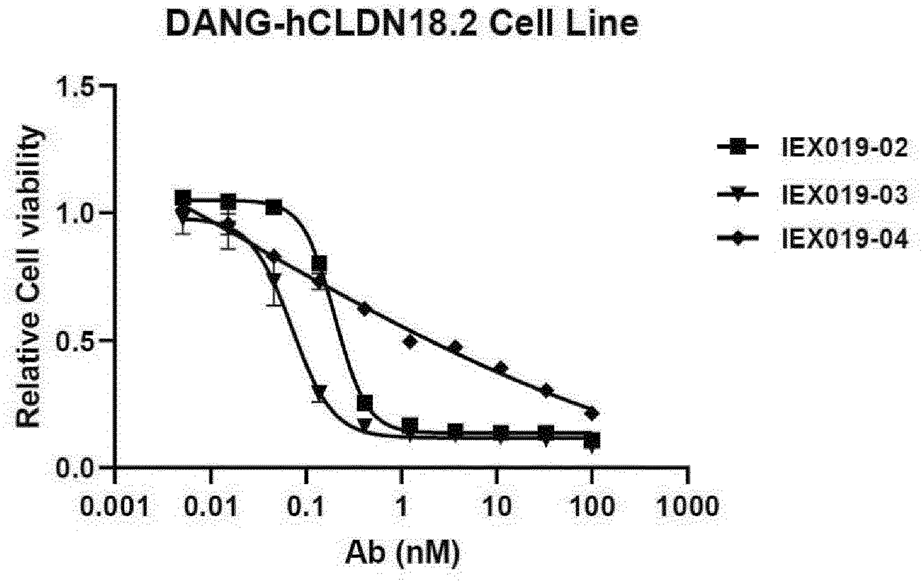
DANG-hCLDN18.2 Cell Line
Relative Cell viability
1.5
1.0
0.5
0.0
0.001
0.01
0.1
1
10
100
1000
Ab (nM)
IEX019-02
IEX019-03
IEX019-04

FIG. 8C

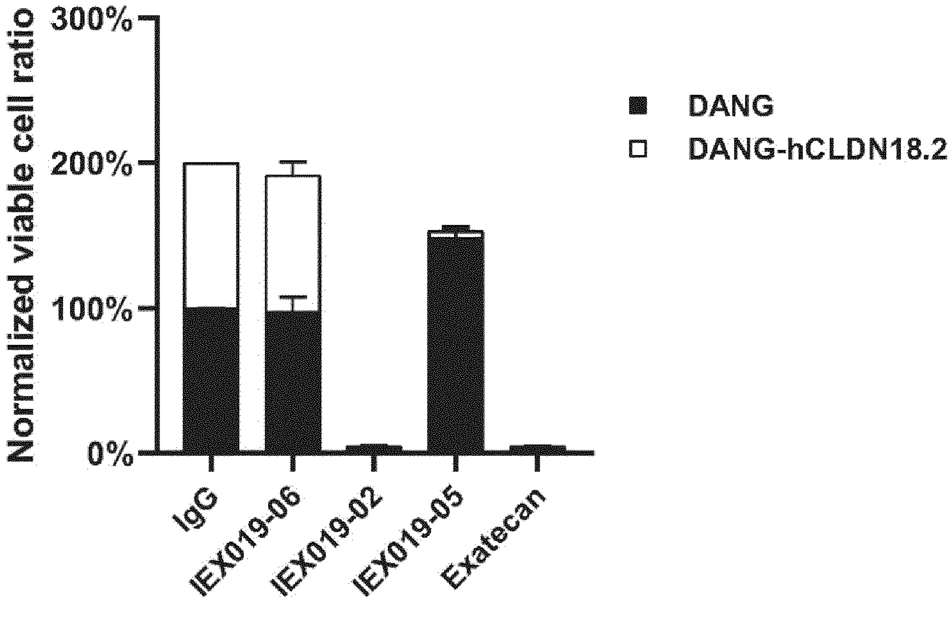
Normalized viable cell ratio
300%
200%
100%
0%
IgG
IEX019-06
IEX019-02
IEX019-05
Exatecan
DANG
DANG-hCLDN18.2

FIG. 9

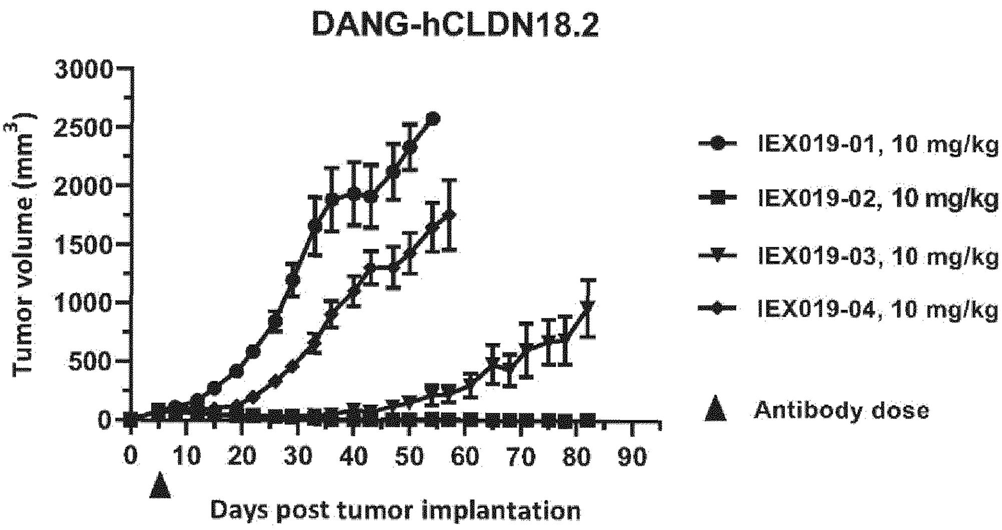


FIG. 10A

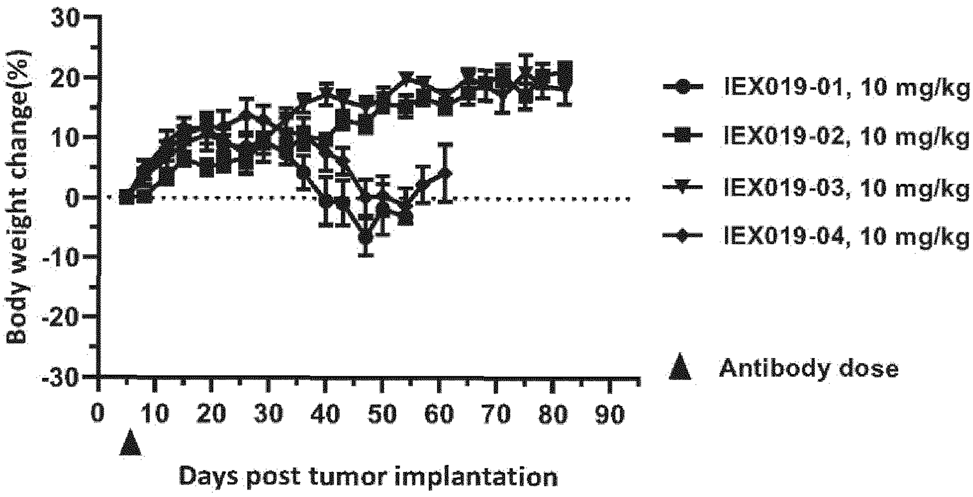


FIG. 10B

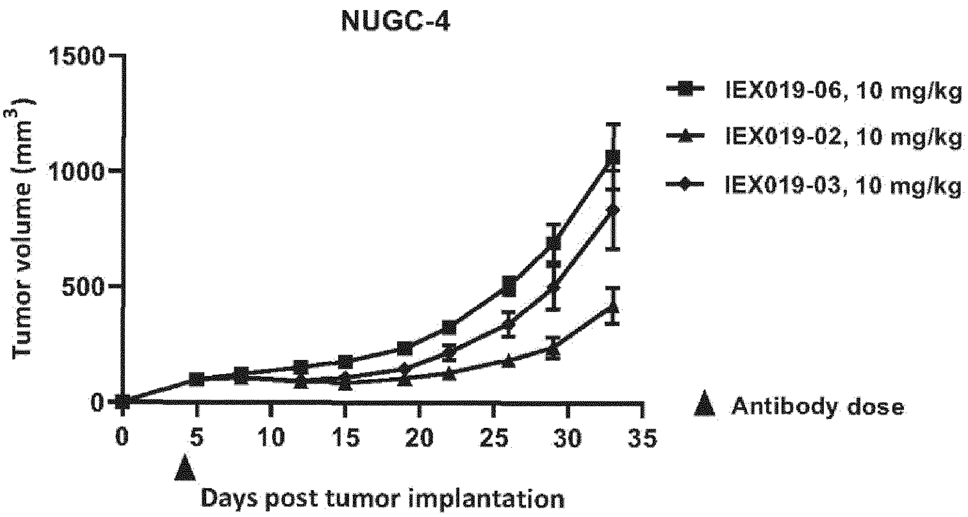


FIG. 11A

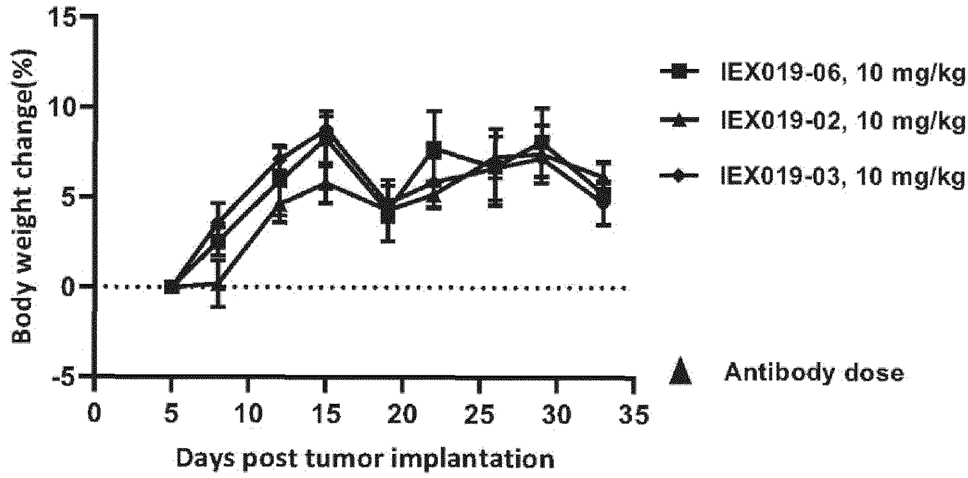


FIG. 11B

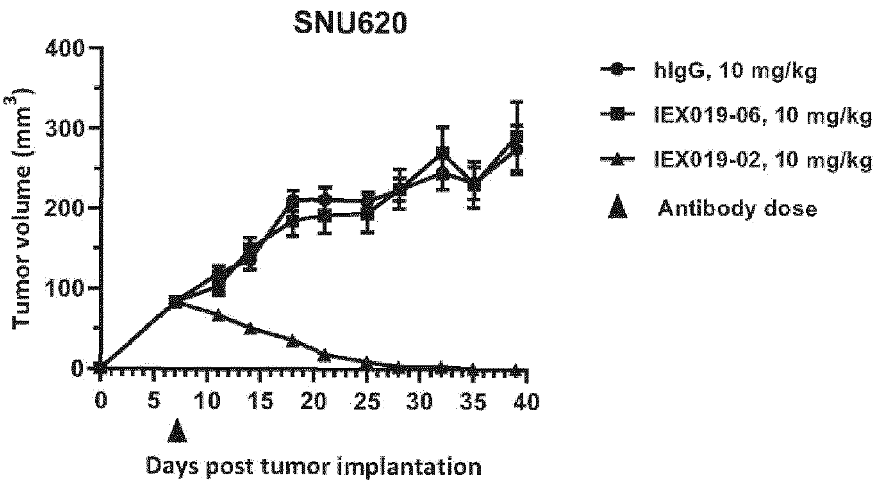


FIG. 12A

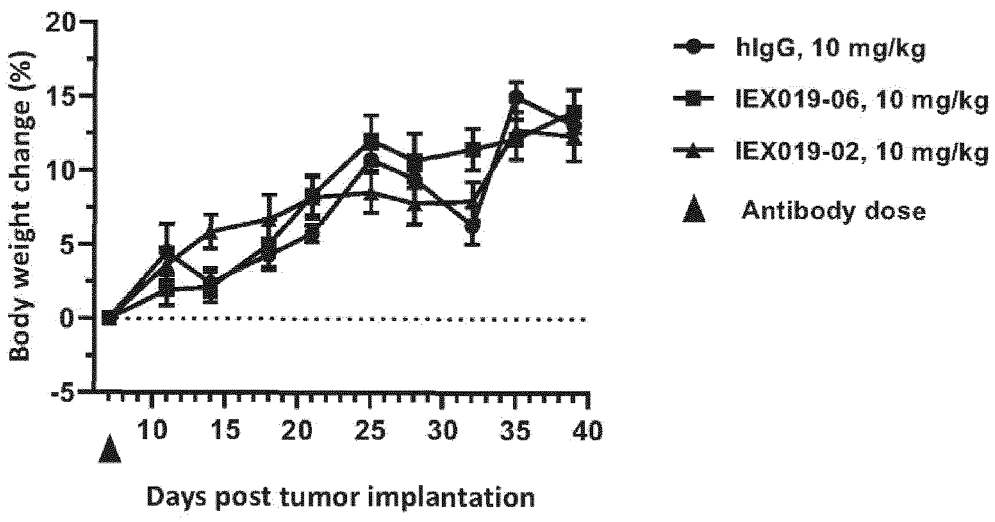


FIG. 12B

## INTERNATIONAL SEARCH REPORT

International application No. **PCT/CN2022/139637**

**A. CLASSIFICATION OF SUBJECT MATTER**

C07K16/28(2006.01)i;A61K47/68(2017.01)i;A61K31/551(2006.01)i;A61K39/395(2006.01)i;A61P35/00(2006.01)i

According to International Patent Classification (IPC) or to both national classification and IPC

**B. FIELDS SEARCHED**

Minimum documentation searched (classification system followed by classification symbols)

C07K,A61K,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pudmed, STN on web, 信达生物制药(苏州)有限公司, 何 开杰, CLDN18.2, 偶联物, 缀合物, conjugate, 糖基化, glycan, Asn297, N297, SEQ ID NOs: 1-12

**C. DOCUMENTS CONSIDERED TO BE RELEVANT**

| Category* | Citation of document, with indication, where appropriate, of the relevant passages | Relevant to claim No. |
|---|---|---|
| PX | WO 2021254481 A1 (INNOVENT BIOLOGICS (SUZHOU) CO., LTD.) 23 December 2021 (2021-12-23)<br>claims 1-23 | 1-9, 22-28 |
| A | CN 112707965 A (HARBOUR BIOMED SUZHOU CO., LTD.) 27 April 2021 (2021-04-27)<br>entire document | 1-28 |
| A | WO 2021008463 A1 (FUTUREGEN BIOPHARMACEUTICAL BEIJING CO., LTD. et al.) 21 January 2021 (2021-01-21)<br>entire document | 1-28 |
| A | WO 2020135201 A1 (Sichuan Kelun-Biotech Biopharmaceutica Co., LTD. et al.) 02 July 2020 (2020-07-02)<br>entire document | 1-28 |
| A | WO 2014164503 A1 (GENZYME CORPORATION) 09 October 2014 (2014-10-09)<br>entire document | 1-28 |
| A | WO 2019065964 A1 (DAIICHI SANKYO COMPANY, LIMITED) 04 April 2019 (2019-04-04)<br>entire document | 1-28 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 March 2023** | **07 March 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/139637**

**C. DOCUMENTS CONSIDERED TO BE RELEVANT**

| Category* | Citation of document, with indication, where appropriate, of the relevant passages | Relevant to claim No. |
|---|---|---|
| A | WO 2020196474 A1 (DAIICHI SANKYO COMPANY, LIMITED) 01 October 2020 (2020-10-01)<br>entire document | 1-28 |

Form PCT/ISA/210 (second sheet) (July 2022)

**INTERNATIONAL SEARCH REPORT**

International application No.
**PCT/CN2022/139637**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2022/139637**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **26-28**
because they relate to subject matter not required to be searched by this Authority, namely:

Claims 26-28 relate to a method for preventing or treating a tumor in a subject, which belongs to a method for treating a disease (PCT Rule 39.1(iv)); however, the search opinion is still provided on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/139637**

| Patent document cited in search report | Publication date (day/month/year) | Patent family member(s) | Publication date (day/month/year) |
|---|---|---|---|
| WO 2021254481 A1 | 23 December 2021 | CA 3178855 A1 | 23 December 2021 |
| | | TW 202204416 A | 01 February 2022 |
| | | WO 2021254481 A9 | 17 February 2022 |
| | | AU 2021291259 A1 | 19 January 2023 |
| CN 112707965 A | 27 April 2021 | None | |
| WO 2021008463 A1 | 21 January 2021 | CA 3147122 A1 | 21 January 2021 |
| | | EP 3992209 A1 | 04 May 2022 |
| | | EP 3992209 A4 | 18 January 2023 |
| | | JP 2022540524 A | 15 September 2022 |
| | | US 2022235129 A1 | 28 July 2022 |
| | | KR 20220032077 A | 15 March 2022 |
| | | AU 2020314119 A1 | 17 February 2022 |
| WO 2020135201 A1 | 02 July 2020 | EP 3904386 A1 | 03 November 2021 |
| | | EP 3904386 A4 | 07 September 2022 |
| | | KR 20210109520 A | 06 September 2021 |
| | | JP 2022515318 A | 18 February 2022 |
| WO 2014164503 A1 | 09 October 2014 | JP 2021106600 A | 29 July 2021 |
| | | US 2015079070 A1 | 19 March 2015 |
| | | US 9701753 B2 | 11 July 2017 |
| | | BR 112015020885 A2 | 10 October 2017 |
| | | IL 289918 A | 01 March 2022 |
| | | KR 20150126024 A | 10 November 2015 |
| | | KR 102330681 B1 | 24 November 2021 |
| | | US 2017267774 A1 | 21 September 2017 |
| | | US 10214589 B2 | 26 February 2019 |
| | | US 2017369584 A1 | 28 December 2017 |
| | | US 10494439 B2 | 03 December 2019 |
| | | AU 2018282451 A1 | 24 January 2019 |
| | | AU 2018282451 B2 | 04 March 2021 |
| | | JP 2016512425 A | 28 April 2016 |
| | | JP 6739330 B2 | 12 August 2020 |
| | | RU 2015143057 A | 17 April 2017 |
| | | RU 2708314 C2 | 05 December 2019 |
| | | JP 2019103514 A | 27 June 2019 |
| | | JP 7121675 B2 | 18 August 2022 |
| | | IL 241084 A0 | 30 November 2015 |
| | | IL 241084 B | 01 March 2022 |
| | | RU 2015142992 A | 13 April 2017 |
| | | RU 2711935 C2 | 23 January 2020 |
| | | US 2019233531 A1 | 01 August 2019 |
| | | US 11130816 B2 | 28 September 2021 |
| | | MX 2015012527 A | 26 May 2016 |
| | | MX 370679 B | 19 December 2019 |
| | | MX 2019014833 A | 17 February 2020 |
| | | AU 2014249224 A1 | 24 September 2015 |
| | | AU 2014249224 B2 | 17 January 2019 |
| | | JP 2020000252 A | 09 January 2020 |
| | | JP 7021159 B2 | 16 February 2022 |
| | | EP 2970469 A1 | 20 January 2016 |
| | | EP 2970469 B1 | 03 October 2018 |

Form PCT/ISA/210 (patent family annex) (July 2022)

**INTERNATIONAL SEARCH REPORT**
**Information on patent family members**

International application No.
**PCT/CN2022/139637**

| Patent document cited in search report | Publication date (day/month/year) | Patent family member(s) | Publication date (day/month/year) |
|---|---|---|---|
| | | EP 2983701 A2 | 17 February 2016 |
| | | JP 2022062157 A | 19 April 2022 |
| | | BR 112015020315 A2 | 10 October 2017 |
| | | HK 1214501 A1 | 29 July 2016 |
| | | CA 2902530 A1 | 09 October 2014 |
| | | WO 2014164534 A2 | 09 October 2014 |
| | | WO 2014164534 A3 | 27 November 2014 |
| | | US 2014294867 A1 | 02 October 2014 |
| | | US 9580511 B2 | 28 February 2017 |
| | | EP 4098663 A1 | 07 December 2022 |
| | | KR 20150126023 A | 10 November 2015 |
| | | KR 102420934 B1 | 15 July 2022 |
| | | KR 20210145294 A | 01 December 2021 |
| | | EP 4063389 A2 | 28 September 2022 |
| | | EP 4063389 A3 | 28 December 2022 |
| | | EP 3424956 A1 | 09 January 2019 |
| | | HK 1213271 A1 | 30 June 2016 |
| | | US 2020140564 A1 | 07 May 2020 |
| | | AU 2014249290 A1 | 24 September 2015 |
| | | AU 2014249290 B2 | 22 November 2018 |
| | | MX 2015012570 A | 12 January 2016 |
| | | MX 370356 B | 10 December 2019 |
| | | AU 2021203458 A1 | 24 June 2021 |
| | | SG 11201506088 RA | 29 September 2015 |
| | | RU 2020100459 A | 28 April 2020 |
| | | CA 2902525 A1 | 09 October 2014 |
| | | IL 275376 A | 30 July 2020 |
| | | US 2022089763 A1 | 24 March 2022 |
| | | IL 241085 A0 | 30 November 2015 |
| | | IL 241085 B | 30 June 2020 |
| | | SG 11201506086 SA | 29 September 2015 |
| | | SG 10201809779 RA | 28 December 2018 |
| | | JP 2016512426 A | 28 April 2016 |
| | | JP 6588422 B2 | 09 October 2019 |
| | | KR 20220103204 A | 21 July 2022 |
| WO 2019065964 A1 | 04 April 2019 | IL 273471 A | 31 May 2020 |
| | | US 2020261594 A1 | 20 August 2020 |
| | | BR 112020004126 A2 | 08 September 2020 |
| | | MX 2020003125 A | 01 October 2020 |
| | | CA 3177158 A1 | 04 April 2019 |
| | | TW 201922788 A | 16 June 2019 |
| | | CO 2020004921 A2 | 05 May 2020 |
| | | SG 11202001430 SA | 29 April 2020 |
| | | CA 3078218 A1 | 04 April 2019 |
| | | CA 3126646 A1 | 04 April 2019 |
| | | RU 2020109412 A | 29 October 2021 |
| | | EP 3690038 A1 | 05 August 2020 |
| | | EP 3690038 A4 | 19 May 2021 |
| | | PH 12020500398 A1 | 04 January 2021 |
| | | AU 2018342527 A1 | 02 April 2020 |

Form PCT/ISA/210 (patent family annex) (July 2022)

**INTERNATIONAL SEARCH REPORT**
**Information on patent family members**

International application No.
**PCT/CN2022/139637**

| Patent document cited in search report | Publication date (day/month/year) | Patent family member(s) | Publication date (day/month/year) |
|---|---|---|---|
| | | JPWO 2019065964 A1 | 10 September 2020 |
| | | US 2022125943 A1 | 28 April 2022 |
| | | CA 3125713 A1 | 04 April 2019 |
| | | US 2022395579 A1 | 15 December 2022 |
| | | JP 2022036943 A | 08 March 2022 |
| | | JP 7133079 B2 | 07 September 2022 |
| | | KR 20200061376 A | 02 June 2020 |
| WO 2020196474 A1 | 01 October 2020 | TW 202102228 A | 16 January 2021 |
| | | KR 20210143237 A | 26 November 2021 |
| | | JPWO 2020196474 A1 | 01 October 2020 |
| | | EP 3950061 A1 | 09 February 2022 |
| | | EP 3950061 A4 | 16 November 2022 |
| | | US 2022168440 A1 | 02 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- NL 2013050744 W
- EP 2016059194 W
- EP 2017052792 W
- EP 2021075401 W
- CN 202010570517X


### Non-patent literature cited in the description


- **SINGH, P. ; TOOM, S. ; HUANG, Y.** Anti-CLDN18.2 antibody as new targeted therapy for advanced gastric cancer. *J Hematol Oncol,* 2017, vol. 10, 105, https://doi.org/10.1186/s13045-017-0473-4
- **O. TURECI. et al.** *Gene,* 2011, vol. 481, 83-92
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. U.S. Department of Health and Human Services, National Institutes of Health, 1987
- **NORTH et al.** A New Clustering of Antibody CDR Loop Conformations. *Journal of Molecular Biology,* 2011, vol. 406, 228-256
- **KABAT et al.** Sequences of Proteins of Immunological Interest. Public Health Service, National Institutes of Health, 1991
- **KABAT et al.** Sequences of Proteins of Immunological Interes. Public Health Service, National Institutes of Health, 1991
- **SU, Z. ; XIAO, D. ; XIE, F. ; LIU, L. ; WANG, Y ; FAN, S. ; LI, S.** Antibody-drug conjugates: Recent advances in linker chemistry. *Acta Pharmaceutica Sinicα B,* 2021
- **R. C. ROWE ; P. J. SESKEY ; S. C. OWEN.** Handbook of Pharmaceutical Excipients. Pharmaceutical Press